# EUROPEAN PATENT APPLICATION

(11) **EP 4 807 007 A1**
(43) Date of publication of application: **16.09.2026**
(21) Application number: 24887937.1
(22) Date of filing: 05.11.2024
(51) Int. Cl.: C12N 15/74, C12N 15/70, C12N 9/12, A61K 38/16, A61P 35/00

(54) **CONSTITUTIVE EXPRESSION VECTOR CONTAINING DUAL BACTERIOPHAGE-DERIVED PROMOTERS, BACTERIUM CONTAINING SAME, AND USES THEREOF**

(30) Priority: 08.11.2023 CN 202311480978
(71) Applicant: Shaoxing Salvectors Biotechnology Ltd., Shaoxing, Zhejiang 312080 (CN)
(72) Inventor: YU, Bin, Hong Kong (HK)
(74) Representative: Gille Hrabal Partnerschaftsgesellschaft mbB Patentanwälte
(86) International application number: PCT/CN2024/129925
(87) International publication number: WO 2025/098325

(57) **Abstract**

A constitutive expression vector containing dual bacteriophage-derived promoters, a gram-negative bacterium containing the expression vector, and uses thereof in preparation of tumor drugs and treatment of tumors. By selecting bacteriophage-derived promoters, a specific bacteriophage RNA polymerase gene, a membrane-disrupting protein gene, and a therapeutic protein gene, the expression vector is divided into a protein expression control module, a drug delivery module, and a drug-mediated killing module. By organically combining the three modules, a three-in-one system is formed, and a system and method for stable and efficient drug protein expression, drug delivery, and drug-mediated killing are realized.

## Description

This application claims the benefit of priority to Chinese Patent Application No. 202311480978.8, filed with the China National Intellectual Property Administration (CNIPA) on November 8, 2023, entitled "Constitutive expression vector containing dual bacteriophage-derived promoters, bacterium containing same, and uses thereof", the entire contents of which are incorporated herein by reference.

### Technical Field

The present invention relates to constitutive expression vector containing dual bacteriophage-derived promoters, bacterium containing same, and uses thereof, and belongs to the fields of genetic engineering and biological therapy.

### Background Art

Cancer is one of the leading causes of death worldwide, and the prevalence of cancer continues to increase annually. Among all malignant tumors, solid tumors account for approximately 90%, such as sarcomas, melanomas, breast cancer, lung cancer, colon cancer, prostate cancer, and the like.

The tumor microenvironment of solid tumors shares common characteristics, including abnormal tumor vasculature, excessive connective tissue, immunosuppression, an acidic environment, and hypoxic regions. Furthermore, the abnormal microenvironment of solid tumors serves as a natural barrier that is difficult for traditional therapeutic drugs to penetrate, making it challenging for chemotherapeutic drugs and antibody-based drugs to diffuse within the solid tumor microenvironment. Moreover, the lack of oxygen free radicals renders tumors resistant to chemotherapy and radiotherapy.

Due to the hypoxic environment within solid tumors, facultative anaerobic bacteria and obligate anaerobic bacteria are capable of invading tumors and inhibiting their growth, and thus can be used as therapeutic agents or vectors with tremendous potential.

As early as 1868, the German physician W. Busch first reported that in some tumor patients concurrently infected with bacteria (*Streptococcus pneumoniae*), the growth of tumors in their bodies was inhibited or even completely eradicated. Within the following thirty years, the American physician Coley and another German physician Fehleisen separately reported the phenomenon that bacterial infection could inhibit tumors. These early research efforts remained controversial because their results were difficult to reproduce, and the virulence of the bacteria was difficult to control. However, later rigorous animal experiments proved that bacterial infection could indeed reduce tumor size, and the host's immune system would also be activated during the treatment process. In 1975, Carswell first reported that endotoxin (lipopolysaccharide) from Gram-negative bacteria could stimulate the immune system to release tumor necrosis factor alpha (TNF-α), which could lead to the death of tumor cells. Furthermore, some bacterial vaccines have also been proven to stimulate the immune system, thereby treating tumors. Among them, Bacillus Calmette-Guérin (BCG) is the earliest biological agent applied in clinical tumor therapy. BCG is an attenuated live bacterial suspension prepared from a highly pathogenic *Mycobacterium bovis* strain through continuous serial subculturing for 230 passages, originally intended for the prevention of tuberculosis. Extensive experiments and clinical practice have confirmed that BCG is one of the most effective means for treating bladder cancer.

In recent years, with the rapid development of molecular biology and genetic engineering technologies, studies have found that certain facultative or obligate anaerobic bacteria can target, colonize, and proliferate within solid tumors, and induce tumor regression, such as the obligate anaerobic bacterium *Clostridium* and the probiotic *Bifidobacterium,* and the like. Among them, the Gram-negative facultative anaerobic bacterium *Salmonella enterica* has the most extensive application prospects.

Studies have demonstrated that *Salmonella typhimurium*, upon attenuation via various methods, exhibits a colonization capacity within tumor tissues that is 1,000 to 10,000 times greater than that in normal tissues, thereby serving as a potential targeted therapeutic agent for tumor treatment.

Regarding improvements in recombinant bacteria targeting tumor regions, existing literature has reported that such recombinant bacteria can be engineered to carry various types of therapeutic agents, thereby effectuating therapeutic protein delivery to tumor sites. However, the therapeutic efficacy remains suboptimal. This limitation is likely attributable to a failure to comprehensively account for the efficiency of in situ drug synthesis and subsequent delivery by the bacteria within the tumor microenvironment. Consequently, it is imperative to address the following three critical aspects:
First, the expression intensity and stability of the target therapeutic protein.

Conventional in vitro bacterial expression technologies primarily focus on achieving high-level expression of target proteins under stable culture conditions, such as high-density cultivation in nutrient-rich media. However, when bacteria are introduced into an in vivo tumor environment, they are subjected to the severe stresses of the complex tumor microenvironment (TME)-including the extreme environments of hypoxia, low pH, high lactate levels, and bacteria-targeting immune cells. Consequently, both bacterial growth and the synthesis of the target protein are significantly compromised. Furthermore, endogenous bacterial promoters are highly susceptible to external factors, such as chemical agents, environmental fluctuations, temperature changes, or other regulatory elements. Therefore, the direct utilization of native bacterial promoters or conventional high-expression strategies may severely impair the optimal expression of the target therapeutic protein. Additionally, due to the limited carrying capacity of the bacterial chromosome, most genes exist as single copy. Coupled with the inherently weak activity of native bacterial promoters, the overall expression level of the therapeutic protein is inevitably restricted, thereby diminishing its tumor-suppressive efficacy.

Second, the selection of the therapeutic protein is of paramount importance.

The intratumoral environment inside the tumor is not completely isolated. Consequently, the direct release of anti-tumor agents within the tumor can result in the diffusion of a portion of the drug into surrounding blood vessels and subsequent systemic circulation to other organs and tissues, thereby inducing undesirable off-target side effects. Therefore, it is particularly crucial to select a therapeutic protein that exerts its tumoricidal effects exclusively within the intracellular environment of tumor cells.

Third, the mode of therapeutic protein delivery is of critical importance.

To successfully deliver therapeutic protein into the intracellular compartment of tumor cells, the bacteria must first invade the tumor cells and subsequently release the drug internally. Because bacterial invasion typically occurs via induced endocytosis by the tumor cells-resulting in the formation of phagocytic vesicles (phagosomes or endosomes)-the therapeutic protein synthesized by the bacteria must traverse this vesicular membrane to reach the cytoplasm and exert their efficacy. Furthermore, the natural drug release efficiency of wild-type bacteria is profoundly low. Therefore, it is essential to incorporate a highly efficient mechanism for vesicular penetration and drug release, thereby maximizing the therapeutic efficacy of the delivered proteins.

In summary, there is an urgent and unmet need for a therapeutic protein system and method capable of enabling bacteria to achieve stable, high-level expression and highly efficient release of therapeutic proteins specifically within the *in vivo* tumor microenvironment.

### Summary of the Invention

Unless otherwise defined herein, all scientific and technical terms used in connection with the present invention have the same meaning as commonly understood by one of ordinary skill in the art to which this invention pertains. Furthermore, the terminology and laboratory procedures relating to protein and nucleic acid chemistry, molecular biology, cell and tissue culture, microbiology, and immunology as used herein are standard terms and conventional techniques widely utilized in their respective fields.

In view of the aforementioned deficiencies in the prior art, the present invention establishes an integrated three-in-one bacterial system capable of stable and highly efficient therapeutic protein expression, delivery, and potent tumoricidal activity.

To this end, in one aspect, the present invention provides a constitutive plasmid expression vector comprising dual bacteriophage-derived promoters that respectively control the expression of a therapeutic protein gene and a membrane-disrupting protein gene; wherein a specific bacteriophage RNA polymerase gene, driven by a constitutive promoter, is integrated into the chromosome of the host bacterium.

The constitutive plasmid expression vector of the present invention comprises three functional modules, specifically: (1) a protein expression control module; (2) a therapeutic protein delivery module; and (3) a therapeutic protein-mediated killing module.

Through the synergistic integration of these three modules, in conjunction with the intrinsic characteristics of the host bacteria, the constitutive plasmid expression vector of the present invention realizes an integrated three-in-one system capable of stable and highly efficient therapeutic protein expression, delivery, and potent tumoricidal activity within the bacterial vector.

### (1) Protein Expression Control Module

To achieve stable and efficient gene expression within the complex tumor microenvironment, it is imperative to employ a robust promoter expression system that is refractory to environmental fluctuations.

The present invention preferably utilizes the constitutive artificial promoter lacUV5 to drive the expression of the bacteriophage-derived T7 RNA polymerase gene. This expression cassette is integrated into the bacterial chromosome to ensure stable inheritance. Because T7 RNA polymerase specifically recognizes the T7 promoter, it enables stable and robust expression of genes under the control of the T7 promoter. Accordingly, the present invention configures the therapeutic protein delivery module gene and the therapeutic protein-mediated killing module gene downstream of respective T7 promoters, placing them within the plasmid expression vector for precise transcriptional regulation.

### (2) Therapeutic Protein Delivery Module

Upon bacterial invasion into tumor cells, phagocytic vesicles (phagosomes) are formed. The presence of these phagocytic vesicles restricts the further intracellular release of the bacteria and their synthesized therapeutic proteins. To overcome this barrier, the present invention preferably employs listeriolysin O (LLO) as a membrane-disrupting protein to achieve the objective of efficient delivery.

### (3) Therapeutic Protein-Mediated Killing Module

To achieve precision therapeutic protein delivery wherein the tumoricidal effect is exerted exclusively within tumor cells, the present invention employs the A subunit (A fragment) of a bacterial AB-type exotoxin to mediate precise intracellular cytotoxicity. A defining characteristic of bacterial AB-type exotoxins is their structural organization into two distinct functional domains. The B subunit (B fragment) is characterized by its ability to specifically bind to extracellular receptors on target cells, inducing conformational changes in the cell membrane and mediating the translocation of the A subunit into the cytoplasm. Conversely, the A subunit exerts its cytotoxic effects exclusively within the cytoplasm, with its specific mechanism of action depending on the origin of the bacterial exotoxin gene. Therefore, by utilizing only the A subunit in the expression vector, any excess A subunit released into the human systemic circulation will not induce toxic side effects.

During the course of tumor therapy utilizing the constitutive plasmid expression vector of the present invention, the engineered bacteria enter the tumor tissue via the systemic circulation and subsequently colonize therein. Within the bacterial cell, the stable expression of T7 RNA polymerase drives the robust and stable co-expression of the therapeutic protein-mediated killing module (the exotoxin A fragment) and the therapeutic protein delivery module (the membrane-disrupting protein LLO) located on the plasmid. Upon encountering cancer cells within the tumor microenvironment, the bacteria exploit their intrinsic invasion systems to induce cellular endocytosis, resulting in the formation of bacteria-containing phagocytic vesicles (phagosomes). Subsequently, these phagosomes fuse with intracellular vacuoles such as lysosomes, leading to a decrease in the intra-vesicular pH. This acidic environment activates the bacterially expressed LLO, which disrupts the vesicular membrane and mediates the release of the exotoxin A fragment into the host cell cytoplasm, ultimately culminating in cancer cell death. As the bacteria continuously proliferate and replicate within the tumor, this cycle is iteratively repeated, driving the rapid eradication of the tumor cells.

Accordingly, it is evident that the present invention, predicated on the seamless integration of the aforementioned three-in-one system, realizes stable and highly efficient therapeutic protein expression, delivery, and potent tumoricidal activity within the bacterial host.

Therefore, in the present invention, the dual bacteriophage-derived promoters are employed to independently drive the expression of the therapeutic exotoxin A fragment and the membrane-disrupting protein gene, respectively, wherein said bacteriophage-derived promoters specifically bind to an RNA polymerase encoded by a specific bacteriophage RNA polymerase gene integrated into the host bacterial chromosome.

In a preferred embodiment of the present invention, the dual bacteriophage-derived promoters are selected from the group consisting of the T7 promoter (SEQ ID NO: 1), the T3 promoter (SEQ ID NO: 2), and the SP6 promoter (SEQ ID NO: 3).

In a more preferred embodiment of the present invention, the dual bacteriophage-derived promoters are T7 promoters (SEQ ID NO: 1).

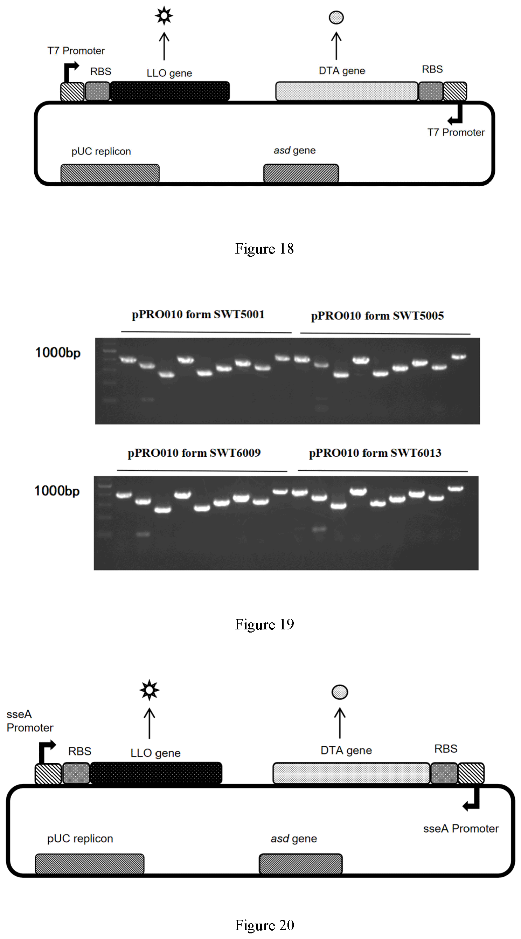

Furthermore, depending on the orientation of the dual bacteriophage-derived promoters, in a preferred expression vector of the present invention, the dual bacteriophage-derived promoters are oriented convergently (facing each other), constituting a convergent promoter expression vector, as specifically illustrated in FIG. 18.

In addition, to prevent the loss of the plasmid expression vector, an essential gene of *Salmonella* is inserted during the construction of the vector, while said gene is simultaneously deleted from the chromosome of the host bacterium, thereby establishing a balanced-lethal control mechanism. Namely, if the *Salmonella* loses the harbored plasmid vector, the bacterium will rapidly die due to the lack of the essential gene. Consequently, all surviving bacteria harbor the plasmid expression vector, thereby ensuring the stable and efficient expression of the protein therapeutics by the entire system.

The *asd* gene of the genus *Salmonella* (SEQ ID NO: 11) and the Asd protein sequence (SEQ ID NO: 12) encode aspartate β-semialdehyde dehydrogenase, which is an enzyme required during the synthesis of diaminopimelic acid (DAP), an essential component of the cell wall in Gram-negative bacteria. Knocking out the *asd* gene in *Salmonella* leads to the lysis and death of the bacteria; however, the normal growth of the *Salmonella* can be maintained either by exogenously supplementing the culture medium with DAP or by enabling the Salmonella to harbor a vector comprising the *asd* gene.

Therefore, in a preferred embodiment of the present invention, the expression vector comprises the essential *asd* gene of *Salmonella*, and the *asd* gene on the chromosome of the host bacterium is deleted.

Furthermore, to achieve highly efficient expression from the expression vector, the expression vector of the present invention preferably comprises an origin of replication essential for plasmid replication.

Simultaneously, the expression vector of the present invention does not comprise an antibiotic resistance gene.

In a preferred embodiment of the present invention, the origin of replication is selected from the group consisting of pUC, p15A, ColE1, and R6K.

In a more preferred embodiment of the present invention, the origin of replication is pUC (SEQ ID NO: 36).

To achieve stable and highly efficient therapeutic killing, the present invention selects therapeutic proteins with high efficiency and specific lethality, which can kill tumor cells while preventing collateral damage to other organs upon leakage of the therapeutic proteins.

Therefore, in a preferred embodiment of the present invention, the therapeutic protein gene is the A fragment gene of a bacteria-derived AB-type exotoxin.

In a preferred embodiment of the present invention, the therapeutic protein gene used in the present invention is the diphtheria toxin A fragment (DTA) gene (SEQ ID NO: 15) and its protein sequence (SEQ ID NO: 16), which have been codon-optimized for *Salmonella*.

Diphtheria toxin (DT) is a protein toxin composed of two relatively independent functional subunits (A and B) linked by a disulfide bond.

The A subunit (DTA) possesses enzymatic activity and is capable of inactivating elongation factor 2 (EF-2) by catalyzing its ADP-ribosylation, thereby blocking protein synthesis in eukaryotic cells. DTA is the primary source of the cytotoxicity of diphtheria toxin.

The B subunit (DTB) lacks cytotoxicity; it binds to receptors on the surface of eukaryotic cells, thereby mediating the entry of the A subunit into the cells. Based on the independent functions of diphtheria toxin DTA and DTB, when DTA is expressed independently in bacteria but has not entered eukaryotic cells, it causes no harm to either the bacteria or the eukaryotic cells. Only when the DTA protein is released into eukaryotic cells does it exert toxicity against them.

Furthermore, in other preferred embodiments of the present invention, the therapeutic protein gene can also be selected from the genes of other AB-type bacterial exotoxins having similar A subunit and B subunit functions. For example, the cholera toxin A subunit fragment gene (SEQ ID NO: 17) and its protein sequence (SEQ ID NO: 18), the A fragment of the Shiga toxin gene Stx1 (SEQ ID NO: 19) and its protein sequence (SEQ ID NO: 20), the A fragment of the Shiga toxin gene Stx2 (SEQ ID NO: 21) and its protein sequence (SEQ ID NO: 22), the pertussis toxin A subunit fragment gene (SEQ ID NO: 23) and its protein sequence (SEQ ID NO: 24), and the Pseudomonas exotoxin A subunit fragment gene (SEQ ID NO: 25) and its protein sequence (SEQ ID NO: 26).

Cholera toxin (CT), derived from *Vibrio cholerae*, is composed of two subunits, A and B. The A subunit is an ADP-ribosyltransferase that disrupts G protein signal transduction, leading to cell dehydration and death. In contrast, the cholera toxin B subunit (CTB) attaches to the cell surface by binding to the pentasaccharide chain of ganglioside GM1; it can be taken up by axon terminals and transported retrogradely to the cell body.

The Shiga toxin gene family encompasses related toxins, primarily classified into two major types: Stx1 and Stx2. These toxins consist of two subunits, A and B, classifying them as AB toxins. The B subunit is a pentamer that binds to specific glycolipids on host cells, particularly globotriaosylceramide (Gb3). The binding of the B subunit to Gb3 induces narrow tubular membrane invaginations, thereby driving the formation of endocytic tubules for cellular uptake of the toxin. Once inside the cell, the A subunit cleaves a specific adenine nucleobase from the 28S RNA of the 60S ribosomal subunit, thereby halting protein synthesis.

Pertussis toxin (PT) is a protein-based AB-type exotoxin produced by *Bordetella pertussis*, the causative agent of whooping cough. The A subunit of PT possesses enzymatic activity and is translocated into the host cell following conformational changes in the membrane-bound transport B subunit. Pertussis toxin is a hexameric exotoxin comprising six subunits (designated S1 through S5, with each complex containing two copies of S4). The subunits are arranged in an AB architecture: the enzymatically active A component is formed by the S1 subunit, while the receptor-binding B component consists of subunits S2 through S5. PT is released from *Bordetella pertussis* in an inactive form. Following binding to cell membrane receptors, PT is internalized into endosomes and subsequently undergoes retrograde transport to the trans-Golgi network and the endoplasmic reticulum (ER). During this transport, the A subunit is activated by the action of glutathione and ATP to catalyze the ADP-ribosylation of heterotrimeric G proteins. This prevents the inhibition of adenylate cyclase activity, leading to an increase in intracellular cAMP concentrations and ultimately inducing cell death.

Pseudomonas exotoxin is an AB-type exotoxin produced by *Pseudomonas aeruginosa*. The A subunit transfers the adenosine diphosphate ribose (ADP-ribose) moiety from NAD+ to a target protein, a process known as ADP-ribosylation. Its pathogenic mechanism involves the inhibition of eukaryotic elongation factor 2 (eEF2) by ADP-ribosylating the diphthamide residue of eEF2, thereby arresting polypeptide chain elongation. (The mechanism of this toxin is analogous to that of diphtheria toxin).

To achieve stable and efficient therapeutic protein delivery, the present invention designs a precise therapeutic protein delivery system. Its objective is to precisely release the bacterially synthesized therapeutic protein into tumor cells. This process is further divided into two steps: the bacterial invasion of host cancer cells, and the release of the synthesized therapeutic protein.

In a preferred embodiment of the present invention, the natural ability of Salmonella to invade host animal cells is utilized to achieve the bacterial invasion of host cancer cells.

*Salmonella enterica* is capable of invading and replicating within host cells, including host epithelial cells and macrophages. This capability is encoded within *Salmonella* Pathogenicity Island 1 (SPI-1). Through the Type III Secretion System 1 (T3SS1), *Salmonella* secretes T3SS effector proteins that induce massive actin rearrangement within the host cell, leading to membrane ruffling and the subsequent formation of *Salmonella-*containing vacuoles (SCVs). Subsequently, the Type III Secretion System 2 (T3SS2), encoded by genes within *Salmonella* Pathogenicity Island 2 (SPI-2), promotes the survival and proliferation of *Salmonella* within these vacuoles.

In a preferred embodiment of the present invention, the release of the synthesized therapeutic protein is achieved by utilizing a *Salmonella* codon-optimized listeriolysin O (LLO) gene (SEQ ID NO: 13) and its corresponding protein sequence (SEQ ID NO: 14), which are endogenously synthesized by the Salmonella.

LLO, naturally encoded by the *hlyA* gene of *Listeria monocytogenes* (LM), binds to cholesterol on the host cell membrane to form pore structures with a diameter of 35 nm (membrane-disrupting activity).

LLO possesses a pH-sensitive acidic domain. The optimal pH for the maturation of the LLO precursor protein and the initiation of its membrane membrane-disrupting activity is approximately 5.0-5.5, whereas it becomes inactive at a neutral pH.

Following the invasion of *Salmonella* and the subsequent formation of phagocytic vacuoles, these vacuoles fuse with intracellular vesicles such as lysosomes, thereby significantly lowering the pH. Under these acidic conditions, the activity of LLO is triggered to exert its membrane-disrupting function, enabling Salmonella to achieve the release of the synthesized therapeutic proteins.

Therefore, in a preferred embodiment of the present invention, the membrane-disrupting protein gene is the listeriolysin *hlyA* gene.

In a preferred embodiment of the present invention, among the dual bacteriophage-derived promoters, one is sequentially linked to a Ribosomal Binding Site (RBS) (SEQ ID NO: 35) and the membrane-disrupting protein gene, while the other is sequentially linked to a Ribosomal Binding Site (RBS) (SEQ ID NO: 35) and the therapeutic protein gene.

Furthermore, in a preferred embodiment of the present invention, the host bacterium of the expression vector is a Gram-negative bacterium.

In another aspect, the present invention provides a method for constructing the plasmid expression vector described herein, comprising the following steps:
1. Sequentially ligating the bacteriophage-derived promoter, the ribosomal binding site, and the membrane-disrupting protein gene of the present invention to construct a membrane-disrupting protein expression unit;
2. Sequentially ligating the bacteriophage-derived promoter, the ribosomal binding site, and the therapeutic protein gene of the present invention to construct a therapeutic protein expression unit;
3. Ligating a plasmid replicon, an *asd* gene, and the two aforementioned gene expression units together to form a complete plasmid expression vector.

In another aspect, the present invention provides a modified Gram-negative bacterium comprising the expression vector of the present invention.

In a preferred embodiment of the present invention, the specific bacteriophage RNA polymerase gene is a gene corresponding to the dual bacteriophage-derived promoters.

In a more preferred embodiment of the present invention, the specific bacteriophage RNA polymerase gene is selected from the group consisting of a *Salmonella* codon-optimized T7 RNA polymerase gene (SEQ ID NO: 4) and T7 RNA polymerase protein sequence (SEQ ID NO: 5), a T3 RNA polymerase gene (SEQ ID NO: 6) and T3 RNA polymerase protein sequence (SEQ ID NO: 7), and an SP6 RNA polymerase gene (SEQ ID NO: 8) and SP6 RNA polymerase protein sequence (SEQ ID NO: 9).

In a further preferred embodiment of the present invention, the specific bacteriophage RNA polymerase gene is the T7 RNA polymerase gene.

In order to express the therapeutic protein stably and efficiently, the present invention places the therapeutic protein gene on a multi-copy plasmid expression vector and controls it via a T7 promoter, thereby achieving maximum-intensity expression of the therapeutic protein gene.

To achieve higher-intensity expression, in a preferred embodiment of the present invention, a T7 bacteriophage RNA polymerase expression system is employed, which comprises a T7 RNA polymerase and a T7 promoter to control the expression of the target therapeutic protein.

The T7 RNA polymerase system is derived from the expression system of the *Escherichia coli* T7 bacteriophage, and is widely used for gene expression in *E. coli* due to its robust protein expression capability. A key characteristic of T7 RNA polymerase is its ability to specifically recognize the T7 promoter without being affected by environmental factors. Furthermore, the RNA synthesis rate of T7 RNA polymerase is five times that of the E. coli RNA polymerase, thereby enabling high-intensity expression of the target protein.

In a preferred embodiment of the present invention, the T7 polymerase and T7 promoter can alternatively be replaced with a T3 polymerase and a T3 promoter. Similar to the T7 RNA polymerase system, the T3 RNA polymerase system is an RNA polymerase system derived from the T3 bacteriophage that highly specifically recognizes the T3 promoter sequence. Alternatively, the T7 polymerase and T7 promoter can be replaced with an SP6 polymerase and an SP6 promoter. Similar to the T7 RNA polymerase system, the SP6 polymerase is also an RNA polymerase system derived from the SP6 bacteriophage that highly specifically recognizes the SP6 promoter sequence. The T3 RNA polymerase or SP6 RNA polymerase and their corresponding promoters can functionally replace the T7 RNA polymerase to specifically control the expression of downstream target genes.

To achieve stable expression without being affected by the internal metabolism of the bacteria or the tumor microenvironment, in a preferred embodiment of the present invention, the expression of the specific bacteriophage RNA polymerase gene is regulated by a constitutive expression promoter.

In a more preferred embodiment of the present invention, a constitutively expressed *lacUV5* promoter (SEQ ID NO: 10) is further employed to control the expression of the specific bacteriophage RNA polymerase gene.

The *lacUV5* promoter is highly similar to the classical *lac* promoter. Compared to the *lac* promoter, it contains only a 2-base pair mutation in the -10 region. The *lacUV5* promoter does not require an additional activator and can drive high-level gene expression. Although it does not require an activator, the expression of the *lacUV5* promoter can be regulated by the LacI repressor in *Escherichia coli* and can be induced by IPTG. IPTG is an effective inducer when used in a concentration range of 100 µM to 1.5 mM.

Because *Salmonella* has lost the *lacI* gene and the entire *lac* operon during its evolution, the use of the *lacUV5* promoter in *Salmonella* constitutes an excellent constitutive expression system.

Therefore, in a more preferred embodiment of the present invention, the T7 RNA polymerase expression cassette controlled by the *lacUV5* promoter is further integrated into the chromosome of *Salmonella*, thereby achieving stable constitutive expression.

In a preferred embodiment of the present invention, the Gram-negative bacterium further comprises a hypoxia-specific gene expression cassette, which comprises:
a) a forward hypoxia promoter, which is a promoter comprising FNR binding site, wherein the forward hypoxia promoter is capable of being induced for expression under hypoxia;
b) a survival-essential gene; and
c) a reverse hyperoxia promoter, which is a promoter comprising FNR and ArcA binding site, wherein the reverse hyperoxia promoter is capable of functioning under oxygen concentrations of normal organs;
wherein the survival-essential gene is a gene encoding alanine racemase.

The terms "polypeptide", "peptide", and "protein" are used interchangeably herein and refer to a polymer of amino acid residues. The term applies to amino acid polymers in which one or more amino acid residues are artificial chemical analogs of a corresponding natural amino acid, as well as to polymers of natural amino acids. The terms "polypeptide", "peptide", "amino acid sequence", and "protein" may also include modified forms, including but not limited to glycosylation, lipidation, sulfation, γ-carboxylation of glutamic acid residues, hydroxylation, and ADP-ribosylation.

In the present invention, "polynucleotide" refers to a macromolecule formed by multiple nucleotides linked via phosphodiester bonds, wherein the nucleotides include ribonucleotides and deoxyribonucleotides. The sequence of the polynucleotide of the present invention can be codon-optimized for different host cells (such as *E. coli*) to improve polypeptide expression. Methods for codon optimization are known in the art.

"Sequence identity" between two polypeptide sequences or two polynucleotide sequences refers to the percentage of identical amino acids or nucleotides between said sequences. Methods for assessing the level of sequence identity between polypeptide or polynucleotide sequences are known in the art. Sequence identity can be assessed using various known sequence analysis software. For example, sequence identity can be evaluated using the online alignment tools provided by EMBL-EBI (https://www.ebi.ac.uk/Tools/psa/). Sequence identity between two sequences can be assessed using the Needleman-Wunsch algorithm with default parameters.

To enable bacteria to survive only under low oxygen conditions, three requirements need to be met:
1. Control over the strength of the upstream promoter and basal leaky expression.
   If the oxygen sensor exhibits partial leaky expression even without binding to the upstream promoter, it would disrupt oxygen-dependent regulation, resulting in bacterial survival under all oxygen concentrations; or if the strength of the upstream promoter is too weak to initiate the expression of the downstream gene, the bacteria will fail to survive under any oxygen concentration;
2. Selection of a suitable survival-essential gene.
   Through the selection of the survival-essential gene, it can be ensured that the bacteria die when the survival-essential gene is not expressed, while bacterial survival can be rapidly ensured when expression is induced under hypoxia;
3. Upon transition between hypoxia and hyperoxia, the upstream promoter can rapidly initiate transcription to rapidly achieve the synthesis and expression of the survival-essential gene, ensuring that the modified bacteria can survive, whereas under high oxygen conditions, the expression of the survival-essential gene will not be initiated, thereby resulting in bacterial death.

Therefore, in the present invention, a "hypoxia-specific gene expression cassette" refers to a segment of DNA capable of initiating the expression of an essential gene under hypoxic conditions, comprising an essential gene under the control of a hypoxia-inducible promoter, and, if necessary, further comprising other regulatory elements required for the expression of the essential gene.

In the present invention, "essential gene" refers to a gene that plays a decisive role in the growth and/or survival of bacteria; once a bacterium lacks this gene or its functional expression product, it cannot survive, divide, and/or grow normally. A typical example of lacking an essential gene or its functional expression product is an auxotrophic strain, which cannot survive, divide, and/or grow normally under *in vitro* culture conditions or in an *in vivo* environment in the absence of a specific exogenous supplement. Essential genes usually exist as a single copy on the bacterial chromosome.

Accordingly, the requirements for the survival-essential gene are as follows:
(1) it is an essential gene for bacterial replication, and its absence results in rapid death of the bacteria;
(2) the product of this gene does not exist in the ordinary environment or the human body, which ensures that the bacteria will not escape control in the human body environment, and facilitates the cultivation and preparation of the bacteria in an ordinary culture environment by adding the corresponding expression product of the gene;
(3) this gene needs to be rapidly initiated under the control of a hypoxia promoter, and can rapidly synthesize the product to achieve the regulatory function of the host bacteria.

In the present invention, the survival-essential gene is a gene encoding alanine racemase.

For Gram-negative bacteria such as *Escherichia coli* and *Salmonella*, the cell wall is an indispensable component. The core component of the cell wall is peptidoglycan. During the synthesis of peptidoglycan, bacteria require D-alanine as an important constituent part. If D-alanine is absent, the bacteria will be unable to synthesize the cell wall, thereby resulting in lysis.

Amino acids existing in nature are typically L-form amino acids; therefore, two genes exist in Gram-negative bacteria: the *alr* gene and *dadX* gene, which synthesize alanine racemase and are responsible for converting L-alanine into D-alanine to meet the requirements of cell wall synthesis.

Studies have found that if both the *alr* gene and the *dadX* gene are mutated simultaneously, a lethal mutation of *Salmonella* can be achieved, and this mutation can be compensated by additionally supplementing D-alanine in the culture medium.

In our previous studies, the *asd* gene was utilized as an essential gene for regulation in YB1 *Salmonella*. Compared to YB1 *Salmonella*, the present invention selects the *alr* gene and the *dadX* gene as survival-essential genes. The *alr* gene and the *dadX* gene are functionally homologous genes. Therefore, in the present invention, the alanine racemase gene can be the *Salmonella alr* gene (SEQ ID No. 27) and the *Salmonella alr* protein sequence (SEQ ID No. 28) or the *Salmonella dadX* gene (SEQ ID No. 29) and the *Salmonella dadX* protein sequence (SEQ ID No. 30); or the *alr* or *dadX* gene from other Gram-negative bacteria or genes with equivalent function.

In the present invention, the alanine racemase *alr* gene and *dadX* gene comprise nucleotide sequences having greater than or equal to 81%, preferably greater than or equal to 82%, more preferably greater than or equal to 83%, greater than or equal to 84%, greater than or equal to 85%, greater than or equal to 86%, greater than or equal to 87%, greater than or equal to 88%, greater than or equal to 89%, greater than or equal to 90%, greater than or equal to 91%, greater than or equal to 92%, greater than or equal to 93%, greater than or equal to 94%, greater than or equal to 95%, greater than or equal to 96%, greater than or equal to 97%, greater than or equal to 98%, or most preferably greater than or equal to 99% sequence identity to the specific sequences described above.

In the present invention, first, bacteria with an *alr* gene knockout are constructed, and then the *dadX* gene is modified so that the bacteria become *alr* and *dadX* deficient strains after editing. Simultaneously, a forward hypoxia promoter and a reverse hyperoxia promoter are used to regulate an additional *alr* or *dadX* gene.

The forward hypoxia promoter described in the present invention is a hypoxia-condition promoter regulated by FNR; the reverse hyperoxia promoter described in the present invention is an antisense promoter negatively regulated by FNR and ArcA. The fumarate and nitrate reduction gene *fnr* is an important gene regulating the aerobic and anaerobic growth of *Salmonella.* This complex regulatory system has been extensively studied in *Escherichia coli* and *Salmonella.* The DNA-binding protein FNR encoded by the *fnr* gene senses changes in oxygen and controls the expression of different genes, achieving a switch at the overall metabolic level. Therefore, DNA binding sequences such as FNR and ArcA become important modes for controlling downstream gene expression.

FNR possesses an oxygen-sensitive [4Fe-4S]²⁺ domain, which can directly sense oxygen and regulate site-specific DNA binding. In contrast, ArcA senses signals from the aerobic respiratory chain. Thus, two distinct mechanisms exist in Gram-negative facultative anaerobic bacteria such as *Salmonella* and *Escherichia coli* for sensing changes in oxygen concentration. When regulating gene expression under hypoxia, FNR can act through two modes: activation and repression.

To this end, the present invention employs the forward hypoxia promoters *yAbU* and *ynfK,* which activate downstream gene expression via FNR under hypoxic conditions, and the reverse hyperoxia promoters *ydcI* and *cyoA,* which inhibit downstream gene expression via FNR and ArcA under hypoxic conditions.

Wherein, the forward hypoxia promoters applied in the present invention are as follows:
(1) *Salmonella yhbU* promoter (yhbU-S) (SEQ ID NO: 31), wherein the FNR binding site is "CTGCCTTAAATCAA";
(2) *Escherichia coli ynfK* promoter (ynfK-E) (SEQ ID NO: 32), wherein the FNR binding site is "TTGCGCTATCTCAA";

Wherein, the reverse hyperoxia promoters applied in the present invention are as follows:
(1) *Salmonella cyoA* promoter (cyoA-S) (SEQ ID NO: 33), wherein the FNR binding site is "TTTATTGATAATAA", and the ArcA binding site is "GTTAAGTA";
(2) *Salmonella ydcI* promoter (ydcI-S) (SEQ ID NO: 34), wherein the FNR binding site is "GTTATCAAAAACAA", and the ArcA binding site is "GTTAATAA";

Through analysis of the aforementioned forward hypoxia promoters and reverse hyperoxia promoters, we found that the FNR binding sites conform to the pattern of "TTGATNNNNATCAA", and wherein any base in the TTGAT and ATCAA sequences of the conserved binding site can be substituted, provided that the total number of substitutions does not exceed 3, and 3 consecutive adjacent bases cannot be substituted; and the ArcA conforms to the pattern of its core region "GTTAATTA", and wherein any base in the GTTAATTA sequence of the conserved binding site can be substituted, provided that the total number of substitutions does not exceed 2.

Thus, in a preferred embodiment of the present invention, the FNR binding site of the forward hypoxia promoter or reverse hyperoxia promoter conforms to the pattern of TTGATNNNNATCAA, wherein N is any base selected from A, T, C, and G, and wherein any base in the TTGAT and ATCAA sequences of the conserved binding site can be substituted, provided that the total number of substitutions does not exceed 3, and 3 consecutive adjacent bases cannot be substituted; the ArcA binding site of the reverse hyperoxia promoter conforms to the pattern of GTTAATTA, wherein any base can be substituted, provided that the total number of substitutions does not exceed 2.

In a preferred embodiment of the present invention, the forward hypoxia promoter and/or the reverse hyperoxia promoter of the hypoxia-specific gene expression cassette is derived from the promoters of the Gram-negative bacterium.

In a more preferred embodiment of the present invention, the forward hypoxia promoter is selected from the promoter region sequences of *yhbU* or *ynfK;* the survival-essential gene is selected from *alr* or *dadX;* the reverse hyperoxia promoter is selected from the promoter region sequences of *cyoA* or *ydcI.*

In a further preferred embodiment of the present invention, the forward hypoxia promoter is *yhbU,* which is selected from *Salmonella, Escherichia coli, Shigella, Yersinia, Enterobacter cloacae, Cronobacter, Klebsiella, Pantoea, Serratia,* and *Shimwellia.*

In a further preferred embodiment of the present invention, the forward hypoxia promoter is *ynfK,* which is selected from *Salmonella, Escherichia coli, Serratia, Shigella,* and *Enterobacter ludwigii.*

In a further preferred embodiment of the present invention, the *alr* gene and the *dadX* gene are selected from *Salmonella, Escherichia coli, Shigella, Klebsiella, Yersinia, Haemophilus,* and *Pseudomonas.*

In a further preferred embodiment of the present invention, the expression cassette consists of the forward hypoxia promoter *yhbU,* the survival-essential gene *alr,* and the reverse hyperoxia promoter *cyoA.*

In a further preferred embodiment of the present invention, the expression cassette consists of the forward hypoxia promoter *yhbU,* the survival-essential gene *alr,* and the reverse hyperoxia promoter *ydcI.*

In a further preferred embodiment of the present invention, the expression cassette consists of the forward hypoxia promoter *ynfK,* the survival-essential gene *dadX,* and the reverse hyperoxia promoter *cyoA.*

In a further preferred embodiment of the present invention, the expression cassette consists of the forward hypoxia promoter *ynfK,* the survival-essential gene *dadX,* and the reverse hyperoxia promoter *ydcI.*

The forward hypoxia promoter disclosed in patent CN104471057B is *pepT.* The *pepT* promoter is not completely regulated by FNR, but is a promoter dually regulated by CRP-cAMP and FNR. Therefore, the FNR binding site in the *pepT* promoter is half a CRP binding site and half an FNR binding site. The CRP-FNR binding region sequence of the *pepT* promoter is *GTGACCTGACGCAA,* wherein the first half, *GTGA,* conforms to the first half of the CRP conserved binding site *GTGANNNNNNTCAC,* and the latter half, *CGCAA,* conforms to a part of the FNR conserved region *ATCAA,* wherein the tenth position A is substituted by C, and the eleventh position T is substituted by G, and thus it does not conform to the rules for the forward hypoxia promoter of the present invention.

The FNR binding region of the reverse hyperoxia promoter *sodA* disclosed in patent CN104471057B, *TTGATAATCATTTT*, only contains the first half-region of FNR, *TTGAT*, and the latter half-region contains three consecutive substitutions, with ATCAA being substituted by *ATTTT*; therefore, it does not comprise a complete FNR binding site. The ArcA binding site comprised therein is *TTTAATTA*, which has the 'G' at the first position substituted by 'T' when compared to the conserved core ArcA binding site 5'*-GTTAATTA*-3'. Therefore, the *sodA* promoter only comprises a single ArcA binding site, and its FNR binding site is incomplete, and thus it does not conform to the rules for the reverse hyperoxia promoter in the present invention.

In a preferred embodiment of the present invention, the hypoxia-specific gene expression cassette is regulated by oxygen concentration.

To enable Gram-negative bacteria such as *Salmonella* to survive in hypoxic regions, a 1% oxygen concentration is a very important key point, as it represents pathological hypoxia. This is because an oxygen concentration below 1% is a clear hallmark of a tumor hypoxic region, and regions with an oxygen concentration below 1% do not exist in normal organ tissues. For example, the oxygen concentration in the hypoxic regions of various tumors, such as pancreatic cancer, cervical cancer, and prostate cancer, is lower than 0.7%.

The hypoxia-specific gene expression cassette designed by the present invention enables Gram-negative bacteria such as *Salmonella* to recognize tumor hypoxic regions and proliferate therein through precise oxygen regulation. The objective achieved by the present invention is that when the oxygen concentration is lower than 0.8%, the modified Gram-negative bacteria such as *Salmonella* can survive and proliferate, and achieve suicide lysis in a normal oxygen concentration environment.

Thus, in a more preferred embodiment of the present invention, the forward hypoxia promoter functions when the oxygen content is lower than 1%, and does not function when the oxygen content is higher than 1%; and/or
the reverse hyperoxia promoter functions when the oxygen content is higher than 1%, and does not function when the oxygen content is lower than 1%.

In a further preferred embodiment of the present invention, the forward hypoxia promoter functions when the oxygen content is lower than 0.8%, and does not function when the oxygen content is higher than 0.8%; and/or
the reverse hyperoxia promoter functions when the oxygen content is higher than 0.8%, and does not function when the oxygen content is lower than 0.8%.

In another aspect, the present invention provides a method for controlling therapeutic protein expression in a prokaryotic cell using the expression vector described herein, comprising the following steps:
1. Preparing the expression vector of the present invention;
2. Integrating a constitutively expressed bacteriophage RNA polymerase gene expression cassette into a host bacterium chromosome;
3. Knocking out the *asd* gene from the host bacterium chromosome;
4. Transforming the expression vector of the present invention into the host bacterium.

In a preferred embodiment of the present invention, the method further comprises integrating the hypoxia-specific gene expression cassette described herein into the host bacterium chromosome.

In a preferred embodiment of the present invention, the host bacterium is a Gram-negative bacterium.

In the present invention, the term "Gram-negative bacteria" refers to bacteria that do not retain the initial basic dye stain (e.g., crystal violet) after the known partial procedure of Gram staining. In an exemplary Gram staining, cells are first fixed on a slide by heating and stained with a basic dye (e.g., crystal violet), the basic dye being absorbed by both Gram-negative and Gram-positive bacteria. Then, the slide is treated with a mordant (e.g., Gram's iodine), which binds to the basic dye (e.g., crystal violet) and traps it within the cells. The cells are then washed with acetone or alcohol and counterstained with a second dye of different color (e.g., safranin). Gram-positive organisms retain the initial purple stain, whereas Gram-negative organisms are decolorized by the organic solvent wash and thus show the counterstain. Exemplary Gram-negative bacteria include, but are not limited to, *Escherichia* spp., *Shigella* spp., *Salmonella* spp., *Campylobacter* spp., *Neisseria* spp., *Haemophilus* spp., *Aeromonas* spp., *Francisella* spp., *Yersinia* spp., *Klebsiella* spp., *Bordetella* spp., *Legionella* spp., *Corynebacterium* spp., *Citrobacter* spp., *Chlamydia* spp., *Brucella* spp., *Pseudomonas* spp., *Helicobacter* spp., and *Vibrio* spp.

Thus, in a more preferred embodim*e*nt of the present invention, the Gram-negative bacterium is selected from the group consisting of *Salmonella*, *Escherichia coli*, *Shigella*, *Yersinia*, *Enterobacter cloacae*, *Cronobacter*, *Klebsiella*, *Pantoea*, *Serratia*, *Shimwellia*, *Enterobacter ludwigii*, *Haemophilus*, *Vibrio*, *Pseudomonas*, *Pasteurella*, *Bordetella*, *Bordetella pertussis*, *Acinetobacter baumannii*, *Burkholderia*, *Vibrio vulnificus*, *Bacteroides fragilis*, *Pseudomonas syringae*, *Pseudomonas putida*, *Legionella*, *Klebsiella pneumoniae*, *Vibrio parahaemolyticus*, *Vibrio cholerae*, *Yersinia pestis*, *Catarrhalis coccus*, *Moraxella catarrhalis*, *Campylobacter jejuni*, *Shigella dysenteriae*, *Neisseria gonorrhoeae*, *Haemophilus influenzae*, *Moraxella*, *Neisseria meningitidis*, *Proteus vulgaris*, *Proteus mirabilis*, *Pasteurella haemolytica*, *Legionella pneumophila*, *Yersinia pestis*, *Shigella sonnei*, *Pseudomonas aeruginosa*, *Yersinia enterocolitica*, *Cryptococcus neoformans*, *Burkholderia cenocepacia*, and *Helicobacter pylori*.

In the present invention, the term "live bacteria" refers to a bacterial strain that is viable, possesses active nutritional metabolic characteristics, and is capable of exercising its own biological functions. Live bacteria may include bacterial biomass generated during the metabolic processes of the strain.

In a more preferred embodiment of the present invention, the Gram-negative bacterium is *Salmonella*.

When the expression vector of the present invention is transformed into a Gram-negative bacterium (e.g., *Salmonella typhimurium*) to serve as a therapeutic bacterium, the high expression of the therapeutic protein gene and membrane-disrupting protein gene controlled by the specific bacteriophage RNA polymerase integrated into the bacterial chromosome and the dual bacteriophage-derived promoters can lead to increased bacterial toxicity. Therefore, to ensure safety for use as a therapeutic bacterium, the aforementioned bacteria integrated with the expression vector of the present invention need to be modified by one or more attenuation methods. Attenuation methods include, but are not limited to, hypoxia-specific gene expression cassette regulatory systems, auxotrophy, stress response defects, and virulence island regulation defects.

Therefore, in a preferred embodiment of the present invention, the Gram-negative bacterium is an attenuated Gram-negative bacterium.

In a more preferred embodiment of the present invention, the attenuated Gram-negative bacterium is *Salmonella.*

In a more preferred embodiment of the present invention, the attenuation method is selected from *Salmonella aroA* gene deficiency, and hypoxia-specific gene expression cassette regulatory system.

Another aspect of the present invention provides the use of the expression vector of the present invention or the Gram-negative bacterium of the present invention in the preparation of tumor drugs.

In a preferred embodiment of the present invention, the tumor is a solid tumor.

The term "solid tumor" as used in the present invention refers to an abnormal mass of tissue, which usually does not contain cysts or liquid areas. Solid tumors may be benign (non-cancerous) or malignant (cancerous). Different types of malignant solid tumors are named according to the type of cells from which they form. Examples of malignant solid tumors are sarcomas, carcinomas, and lymphomas. Leukemias (blood cancers) generally do not form malignant solid tumors. Malignant solid tumors include, but are not limited to, abnormal cell masses that may originate from different tissue types, such as liver, colon, colorectal, skin, breast, pancreas, cervix, uterine body, bladder, gallbladder, kidney, larynx, lip, oral cavity, esophagus, ovary, prostate, stomach, testis, thyroid, or lung, etc.; therefore, malignant solid tumors include malignant solid liver tumors, colon tumors, colorectal tumors, skin tumors, breast tumors, pancreatic tumors, cervical tumors, uterine body tumors, bladder tumors, gallbladder tumors, kidney tumors, laryngeal tumors, lip tumors, oral cavity tumors, esophageal tumors, ovarian tumors, prostate tumors, stomach tumors, testicular tumors, thyroid tumors, or lung tumors, etc.

Thus, in a more preferred embodiment of the present invention, the solid tumor is selected from the group consisting of tumors/cancers of the breast, bone, liver, lung, skin, kidney, stomach, pancreas, prostate, lymph (non-Hodgkin's lymphoma, Hodgkin's lymphoma), intestine (colon cancer, rectal cancer), pelvis (cervical cancer, ovarian malignancy, endometrial cancer, ovarian cancer), nervous system, head and neck, and bladder.

In a further preferred embodiment of the present invention, the solid tumor is breast cancer, osteosarcoma, liver cancer, lung cancer, melanoma, kidney cancer, stomach cancer, pancreatic cancer, prostate cancer, colon cancer, ovarian cancer, neuroblastoma, squamous cell carcinoma, and bladder cancer.

In the present invention, for therapeutic purposes, the term "subject" preferably refers to a subject in need of treatment for a target pathological condition such as a tumor. For prevention purposes, the subject is preferably a subject at risk of developing a target pathological condition or predisposed to developing a target pathological condition. The term "subject" includes living organisms, such as prokaryotes and eukaryotes. Examples of subjects include mammals, such as humans, dogs, cows, horses, pigs, sheep, goats, cats, mice, rabbits, hedgehogs, rats, and transgenic non-human animals. In a specific embodiment of the present invention, the subject is a human.

As used in the present invention, "treatment" is a process used to obtain beneficial or desired clinical results. For the purposes of the present invention, beneficial or desired clinical results include, but are not limited to, one or more of the following: reducing the proliferation of neoplastic or cancerous cells (or destroying neoplastic or cancerous cells), inhibiting metastasis of neoplastic cells, shrinking or reducing tumor size, alleviating malignancy, alleviating symptoms caused by malignancy, improving the quality of life of subjects suffering from malignancy, reducing the dose of other drugs required to treat malignancy, delaying the progression of malignancy, curing malignancy, and/or prolonging the survival of patients suffering from malignancy.

As used in the present invention, an "effective amount" or "effective dose" of a bacterium, drug, or pharmaceutical composition is an amount sufficient to achieve any one or more beneficial or desired results. For prophylactic use, beneficial or desired results include eliminating or reducing the risk of disease, reducing the severity of disease, or delaying the onset of disease, including biochemical, histological, and/or behavioral symptoms of the disease, its complications, and intermediate pathological phenotypes presenting during the development of the disease. For therapeutic use, beneficial or desired results include such as alleviating one or more symptoms of a disease (such as tumor), reducing the dose of other drugs required to treat the disease, enhancing the effect of another drug, prolonging the survival of the treated subject, and/or delaying the progression of cancer in patients. For example, compared to untreated subject, an "effective amount" preferably inhibits cell growth or tumor growth by at least about 10%, preferably at least about 20%, more preferably at least about 30%, more preferably at least about 40%, more preferably at least about 50%, more preferably at least about 60%, more preferably at least about 70%, more preferably at least about 80%. The ability to inhibit tumor growth can be evaluated in animal model systems predictive of efficacy against human tumors. Alternatively, it can also be evaluated by examining the ability to inhibit cell growth, which can be determined *in vitro* by assays well known to those skilled in the art. A therapeutically effective amount of a therapeutic compound can reduce tumor size or otherwise alleviating symptoms in a subject. Those skilled in the art can determine such amounts based on factors such as the size of the subject, the severity of the subject's symptoms, and the specific composition or route of administration selected.

In some embodiments of the present invention, the effective amount of bacteria in the bacterial agent, drug, or pharmaceutical composition comprises at least about 10⁴ colony-forming units (cfu), for example, at least about 10⁴, 10⁵, 10⁶, 10⁷, 10⁸, 10⁹, 10¹⁰, 10¹¹, 10¹², or 10¹³ cfu, including any ranges between the specified values, such as 10⁴-10⁸cfu, 10⁴-10⁹cfu, 10⁴-10¹⁰cfu, 10⁴-10¹¹cfu, 10⁴-10¹²cfu, 10⁴-10¹³cfu, 10⁵-10⁸cfu, 10⁵-10⁹cfu, 10⁵-10¹⁰cfu, 10⁵-10¹¹cfu, 10⁵-10¹²cfu, 10⁵-10¹³cfu, 10⁶-10⁸cfu, 10⁶-10⁹cfu, 10⁶-10¹⁰cfu, 10⁶-10¹¹cfu, 10⁶-10¹²cfu, 10⁶-10¹³cfu, 10⁷-10⁸cfu, 10⁷-10⁹cfu, 10⁷-10¹⁰cfu, 10⁷-10¹¹cfu, 10⁷-10¹²cfu, 10⁷-10¹³cfu, 10⁸-10⁹cfu, 10⁸-10¹⁰cfu, 10⁸-10¹¹cfu, 10⁸-10¹²cfu or 10⁸-10¹³cfu.

In some embodiments of the present invention, the volume of the effective dose of the bacteria, drug, or pharmaceutical composition of the present invention is less than or equal to about 3 mL, about 2.5 mL, about 2 mL, about 1.5 mL, about 1 mL, about 0.75 mL, about 0.5 mL, about 0.25 mL, or about 0.1 mL. In some embodiments, the volume of the effective dose of the bacteria, drug, or pharmaceutical composition is about 20 mL, about 19 mL, about 18 mL, about 17 mL, about 16 mL, about 15 mL, about 14 mL, about 13 mL, about 12 mL, about 11 mL, about 10 mL, about 9 mL, about 8 mL, about 7 mL, about 6 mL, about 5 mL, about 4 mL, about 3 mL, about 2 mL, or about 1 mL. Alternatively, the volume of the effective dose of the bacteria, drug, or pharmaceutical composition is about 20.5 mL, about 19.5 mL, about 18.5 mL, about 17.5 mL, about 16.5 mL, about 15.5 mL, about 14.5 mL, about 13.5 mL, about 12.5 mL, about 11.5 mL, about 10.5 mL, about 9.5 mL, about 8.5 mL, about 7.5 mL, about 6.5 mL, about 5.5 mL, about 4.5 mL, about 3.5 mL, about 2.5 mL, about 1.5 mL, or about 0.5 mL. In some embodiments, the volume of the effective dose of the bacteria, drug, or pharmaceutical composition is about 200 mL, about 190 mL, about 180 mL, about 170 mL, about 160 mL, about 150 mL, about 140 mL, about 130 mL, about 120 mL, about 110 mL, about 100 mL, about 90 mL, about 80 mL, about 70 mL, about 60 mL, about 50 mL, about 40 mL, or about 30 mL, as well as ranges between any of the listed values, such as 100 mL-110 mL, 100 mL-120 mL, 90 mL-120 mL, 90 mL-130 mL, etc. Alternatively, the volume of the effective dose of the bacteria, drug, or pharmaceutical composition is about 205 mL, about 195 mL, about 185 mL, about 175 mL, about 165 mL, about 155 mL, about 145 mL, about 135 mL, about 125 mL, about 115 mL, about 105 mL, about 95 mL, about 85 mL, about 75 mL, about 65 mL, about 55 mL, about 45 mL, about 35 mL, or about 25 mL, as well as ranges between any of the listed values, such as 105 mL-115 mL, 105 mL-125 mL, 95 mL-125 mL, 95 mL-135 mL, etc. Alternatively, the volume of the effective dose of the bacteria, drug, or pharmaceutical composition is about 900 microliters, about 800 microliters, about 700 microliters, about 600 microliters, about 500 microliters, about 400 microliters, about 300 microliters, about 200 microliters, or about 100 microliters; alternatively about 950 microliters, about 850 microliters, about 750 microliters, about 650 microliters, about 550 microliters, about 450 microliters, about 350 microliters, about 250 microliters, about 150 microliters, or about 50 microliters.

In some embodiments, the volume of the effective dose of the bacteria, drug, or pharmaceutical composition is less than or equal to about 2.0 mL.

The dosage ranges discussed herein are merely exemplary and do not limit the dosage ranges that may be selected by a physician. The amount of the active ingredient (e.g., the bacteria of the present invention) in the pharmaceutical composition of the present invention may vary depending on factors such as the disease state, age, gender, and weight of the individual, including the presence or absence of a tumor, the type of tumor being treated, the severity of the tumor, the activity or viability of the bacteria, drug, or pharmaceutical composition, the route of administration, the duration of treatment, the drugs used in combination with the bacteria, drug, or pharmaceutical composition (if any), the diet and general health of the subject, and similar factors well known in the art. The dosage regimen may be adjusted to provide the optimum therapeutic response. For example, it may be administered as a single dose, several divided doses may be administered over time, or the dose may be proportionally reduced or increased as indicated by the exigencies of the therapeutic situation.

As used herein, "pharmaceutically acceptable carrier" includes any material which, when combined with the active ingredient, allows the ingredient to retain its biological activity and does not react with the subject's immune system, including but not limited to disintegrants, binders, fillers, buffers, tonicity agents, stabilizers, antioxidants, surfactants, and lubricants. Preferably, the carrier is suitable for intravenous, intramuscular, subcutaneous, parenteral, spinal, or epidermal administration (e.g., by injection or infusion). For example, depending on the route of administration, the bacteria of the present invention may be coated in a material to protect the bacteria from acidic and other natural conditions that could inactivate them. Pharmaceutically acceptable carriers include physiological saline, PBS buffer, sterile aqueous solutions or dispersions, and powders for the preparation of injectable solutions or dispersions. The use of such media and agents for pharmaceutically active substances is well known in the art. Conventional media or agents except those incompatible with the active compound may be present in the pharmaceutical compositions of the present invention.

Accordingly, another aspect of the present invention provides a pharmaceutical composition comprising an effective amount of the expression vector of the present invention or the modified bacteria of the present invention. In one embodiment, the modified bacterium is a live bacterium.

In a preferred embodiment of the present invention, the pharmaceutical composition further comprises a pharmaceutically acceptable carrier.

In a more preferred embodiment of the present invention, the pharmaceutically acceptable carrier is selected from disintegrants, binders, fillers, buffers, tonicity agents, stabilizers, antioxidants, surfactants, and lubricants.

In a preferred embodiment of the present invention, the pharmaceutical composition is used for treating solid tumors. The bacteria, drug, or pharmaceutical composition of the present invention is administered via the following routes: intravenous injection, intratumoral injection, intramuscular injection, subcutaneous injection, intraperitoneal injection, intracerebral administration, gastrointestinal administration, topical administration, buccal administration, nasal administration, rectal administration, or vaginal administration.

In a preferred embodiment of the present invention, the bacteria, drug, or pharmaceutical composition of the present invention can be formulated into a form suitable for administration via the following routes: intravenous injection, intratumoral injection, intramuscular injection, subcutaneous injection, intraperitoneal injection, intracerebral administration, gastrointestinal administration, topical administration, buccal administration, nasal administration, rectal administration, or vaginal administration.

The dosage forms of the drug or pharmaceutical composition of the present invention may be in the form of solutions, emulsions, lyophilized formulation, or suspensions; for oral administration, the dosage form may be in the form of tablets or capsules; for intranasal administration, the dosage form may be in the form of powders, nasal drops, or aerosols; for topical administration, the dosage form may be aqueous solutions, suspensions, ointments, creams, or gels; for rectal or vaginal administration, the dosage form may be suppositories, enemas, or delivered as part of an endoscopic or colonoscopic procedure.

The bacteria, drug, or pharmaceutical composition of the present invention can be manufactured by methods well known in the art, such as microbial growth in a fermenter, followed by centrifugal concentration and washing, filtration or dialysis, conventional granulation, mixing, dissolving, encapsulation, lyophilization, or emulsification processes, and other methods. The bacteria, drug, or pharmaceutical composition of the present invention can be produced in various forms, including granules, precipitates or particulates, powders (including freeze-dried, rotary-dried, or spray-dried powders), amorphous powders, injectables, emulsions, elixirs, suspensions, or solutions. The formulation may optionally contain stabilizers, pH adjusters, surfactants, bioavailability modifiers, and combinations thereof.

The bacteria, drug, or pharmaceutical composition of the present invention can be administered alone or in combination with other compounds or compositions in the presence of a carrier. In a preferred embodiment of the present invention, the bacteria, drug, or pharmaceutical composition can be administered in combination with other malignant tumor therapies (including but not limited to radiotherapy, chemotherapy, and surgery). The bacteria, drug, or pharmaceutical composition can be used as an adjuvant in therapy in such cases.

In another aspect, the present invention provides a method for treating a tumor, comprising administering to a subject the expression vector described in the present invention, the Gram-negative bacteria described in the present invention, or the pharmaceutical composition described in the present invention.

In one embodiment, the expression vector, Gram-negative bacteria, or pharmaceutical composition is used for treating a tumor. In one embodiment, the expression vector, Gram-negative bacteria, or pharmaceutical composition is used for inducing an anti-tumor specific immune response in a subject having a tumor. In one embodiment, the expression vector, Gram-negative bacteria, or pharmaceutical composition is used for inducing anti-tumor immune memory in a subject having a tumor. In one embodiment, the expression vector, Gram-negative bacteria, or pharmaceutical composition is used for preventing or treating metastasis or recurrence of a tumor. In one embodiment, the expression vector, Gram-negative bacterium, or pharmaceutical composition is used for treating a tumor that has developed resistance to prior anti-tumor treatments or where treatment has failed.

In one embodiment of the present invention, the tumor is a tumor of the nervous system, respiratory system, digestive system, urinary system, reproductive system, hematopoietic system, lymphatic system, endocrine system, or skin and mucous membranes. In one embodiment, the tumor is a sarcoma or carcinoma. In one embodiment, the tumor is a solid tumor.

In a further preferred embodiment of the present invention, the solid tumor is selected from tumors/cancers of the breast, bone, liver, lung, skin, kidney, stomach, pancreas, prostate, lymph (non-Hodgkin's lymphoma, Hodgkin's lymphoma), intestine (colon cancer, rectal cancer), pelvis (cervical cancer, ovarian malignancy, endometrial cancer, ovarian cancer), nervous system, head and neck cancer, and bladder, etc.

In a more preferred embodiment of the present invention, the solid tumor is breast cancer, osteosarcoma, liver cancer, lung cancer, melanoma, kidney cancer, gastric cancer, pancreatic cancer, prostate cancer, colon cancer, ovarian cancer, neuroblastoma, squamous cell carcinoma, and bladder cancer.

The method for treating a tumor provided by the present invention comprises administering an effective amount of the expression vector, Gram-negative bacteria, or pharmaceutical composition of the present invention to a subject having a tumor.

In an embodiment of the present invention, the expression vector, Gram-negative bacteria, or pharmaceutical composition of the present invention may be administered via the following routes: intravenous injection, intratumoral injection, intramuscular injection, subcutaneous injection, intraperitoneal injection, intracerebral administration, gastrointestinal administration, topical administration, buccal administration, nasal administration, rectal administration, or vaginal administration.

In some embodiments of the present invention, the expression vector, Gram-negative bacteria, or pharmaceutical composition of the present invention may be administered at intervals of, for example, about 1 minute, 2, 3, 4, 5, 6, 7, 8, 9, 10, 15, 20, 25, 30, 35, 40, 45, 50, 55 minutes, 1 hour, 2, 3, 4, 5, 6, 7, 8, 9, 10, 11, 12, 13, 14, 15, 16, 17, 18, 19, 20, 21, 22, 23 hours, 1 day, 2, 3, 4, 5, 6, 7, 8, 9, or 10 days, including ranges between any two of the listed values, for example, 1 minute-10 minutes, 1 minute-30 minutes, 1 minute-1 hour, 1 minute-2 hours, 1 minute-4 hours, 1 minute-12 hours, 1 minute-18 hours, 1 minute-1 day, 10 minutes-30 minutes, 10 minutes-1 hour, 10 minutes-2 hours, 10 minutes-4 hours, 10 minutes-12 hours, 10 minutes-18 hours, 10 minutes-1 day, 30 minutes-1 hour, 30 minutes-2 hours, 30 minutes-4 hours, 30 minutes-12 hours, 30 minutes-18 hours, 30 minutes-1 day, 30 minutes-2 days, 1 hour-2 hours, 1 hour-4 hours, 1 hour-12 hours, 1 hour-18 hours, 1 hour-1 day, 4 hours-12 hours, 4 hours-18 hours, 4 hours-1 day, 1 day-2 days, 1 day-3 days, 1 day-4 days, 1 day-5 days, 1 day-7 days, 1 day-10 days, 2 days-3 days, 2 days-4 days, 2 days-5 days, 2 days-7 days, 2 days-10 days, or 5 days-10 days. In some embodiments, the expression vector, Gram-negative bacteria, or pharmaceutical composition is administered once a week, once every two weeks, once every three weeks, once every four weeks, once every five weeks, once every six weeks, once every seven weeks, once every eight weeks, once every nine weeks, once every ten weeks, once every fifteen weeks, once every twenty weeks, once every twenty-five weeks, or once every twenty-six weeks. In some embodiments, the expression vector, Gram-negative bacteria, or pharmaceutical composition is formulated in a form that, for example, can be administered at intervals of about 1 minute, 2, 3, 4, 5, 6, 7, 8, 9, 10, 15, 20, 25, 30, 35, 40, 45, 50, 55 minutes, 1 hour, 2, 3, 4, 5, 6, 7, 8, 9, 10, 11, 12, 13, 14, 15, 16, 17, 18, 19, 20, 21, 22, 23 hours, 1 day, 2, 3, 4, 5, 6, 7, 8, 9, or 10 days, including ranges between any two of the listed values, for example, 1 minute-10 minutes, 1 minute-30 minutes, 1 minute-1 hour, 1 minute-2 hours, 1 minute-4 hours, 1 minute-12 hours, 1 minute-18 hours, 1 minute-1 day, 10 minutes-30 minutes, 10 minutes-1 hour, 10 minutes-2 hours, 10 minutes-4 hours, 10 minutes-12 hours, 10 minutes-18 hours, 10 minutes-1 day, 30 minutes-1 hour, 30 minutes-2 hours, 30 minutes-4 hours, 30 minutes-12 hours, 30 minutes-18 hours, 30 minutes-1 day, 30 minutes-2 days, 1 hour-2 hours, 1 hour-4 hours, 1 hour-12 hours, 1 hour-18 hours, 1 hour-1 day, 4 hours-12 hours, 4 hours-18 hours, 4 hours-1 day, 1 day-2 days, 1 day-3 days, 1 day-4 days, 1 day-5 days, 1 day-7 days, 1 day-10 days, 2 days-3 days, 2 days-4 days, 2 days-5 days, 2 days-7 days, 2 days-10 days, or 5 days-10 days. In some embodiments, the expression vector, bacterium, drug, or pharmaceutical composition is formulated in a form to be administered once a week, once every two weeks, once every three weeks, once every four weeks, once every five weeks, once every six weeks, once every seven weeks, once every eight weeks, once every nine weeks, once every ten weeks, once every fifteen weeks, once every twenty weeks, once every twenty-five weeks, or once every twenty-six weeks. The dosage regimen may depend on the pattern of pharmacokinetic decay expected by the medical practitioner. The progress of the therapy can be monitored by conventional techniques and assays. The administration regimen may change over time.

The present invention also provides a method for inducing an anti-tumor specific immune response in a subject having a tumor, comprising administering to the subject having a tumor an effective amount of the expression vector, Gram-negative bacteria, or pharmaceutical composition of the present invention.

The present invention also provides a method for inducing anti-tumor immune memory in a subject having a tumor, comprising administering to the subject having a tumor an effective amount of the expression vector, Gram-negative bacteria, or pharmaceutical composition of the present invention.

The present invention also provides a method for preventing or treating metastasis or recurrence of a tumor, comprising administering to a subject having a tumor an effective amount of the expression vector, Gram-negative bacteria, or pharmaceutical composition of the present invention.

The present invention also provides a method for preventing or treating metastasis or recurrence of a tumor, comprising administering an effective amount of the expression vector, Gram-negative bacteria, or pharmaceutical composition of the present invention to a subject having tumor metastasis or recurrence or a subject at high risk of tumor metastasis or recurrence.

The present invention also provides a method for treating a tumor that has developed resistance to prior anti-tumor treatments or where treatment has failed, comprising administering an effective amount of the expression vector, Gram-negative bacteria, or pharmaceutical composition of the present invention to a subject having a tumor that has developed resistance to prior anti-tumor treatments or where treatment has failed.

By adopting the above technical solutions, the present invention achieves the following beneficial effects:
1. The present invention, by integrating a bacteriophage-derived promoter with a specific bacteriophage RNA polymerase gene to enable their coordinated action, and utilizing a plasmid-based expression platform, achieves stable and efficient expression of therapeutic proteins specifically within tumor regions.
2. The present invention, by combining a bacteriophage-derived promoter with a membrane-disrupting protein gene and a therapeutic protein gene, enabling their coordinated action, achieves stable and efficient drug delivery, leading to effective killing of tumor cells.
3. The present invention, through the rational design of a hypoxia-specific gene expression cassette, results in modified bacteria with a low overall mutation rate, achieving specific distribution within tumors, and exhibiting faster clearance from normal organs.

### Brief Description of the Drawings

Figure 1 Verification diagram of positive hypoxia promoter *yhbU-S* and *ynfK-S* clone identified by agarose gel electrophoresis.
Figure 2 Amplification diagram of verification of essential gene library identified by agarose gel electrophoresis.
Figure 3 Verification diagram of reverse hyperoxia promoter *cyoA-S* and *ydcI-S* clone library identified by agarose gel electrophoresis.
Figure 4 Schematic diagram of a hypoxia-specific gene expression cassette.
Figure 5 Schematic diagram of lambda RED recombinase and CRE recombinase systems.
Figure 6 Identification diagram of the *aroA* gene knockout in *Salmonella* by experiments.
   (A) Verification diagram of PCR-amplified product of the *aroA* gene knockout fragment identified by agarose gel electrophoresis;
   (B) Verification diagram of PCR-amplification product of target fragment in strain SWT003 identified by agarose gel electrophoresis.
Figure 7 Identification diagram of the *alr* gene knockout in *Salmonella* by experiments.
   (A) Verification diagram of PCR-amplified product of the *alr* gene knockout fragment identified by agarose gel electrophoresis;
   (B) Verification diagram of PCR-amplification product of target fragment in strain SWT004 identified by agarose gel electrophoresis.
Figure 8 Verification diagram of strain SWT1001 and SWT1005 with insertion of hypoxia-specific gene expression cassette (*alr* as an essential gene) identified by agarose gel electrophoresis.
Figure 9 Verification diagram of PCR-amplified products of strain SWT007 identified by agarose gel electrophoresis.
Figure 10 Verification diagram of strain SWT2009 and SWT2013 with insertion of hypoxia-specific gene expression cassette (*dadX* as an essential gene) identified by agarose gel electrophoresis.
Figure 11 Results diagram of oxygen adaptability validation tests for *Salmonella* strain SWT006.
   (A) Anaerobic culture on LB plates without D-alanine;
   (B) Aerobic culture on LB plates without D-alanine.
Figure 12 Results diagram of oxygen adaptability validation tests for *Salmonella* strain SWT1005.
   (A) Anaerobic culture on LB plates without D-alanine;
   (B) Aerobic culture on LB plates without D-alanine.
Figure 13 Results diagram of oxygen adaptability validation tests for *Salmonella* strains SWT2009 and SWT2013.
   (A) SWT2009 cultured on LB plates without D-alanine, the upper part shows culture under anaerobic conditions, the lower part shows culture under aerobic conditions;
   (B) SWT2013 cultured on LB plates without D-alanine, the upper part shows culture under anaerobic conditions, the lower part shows culture under aerobic conditions.

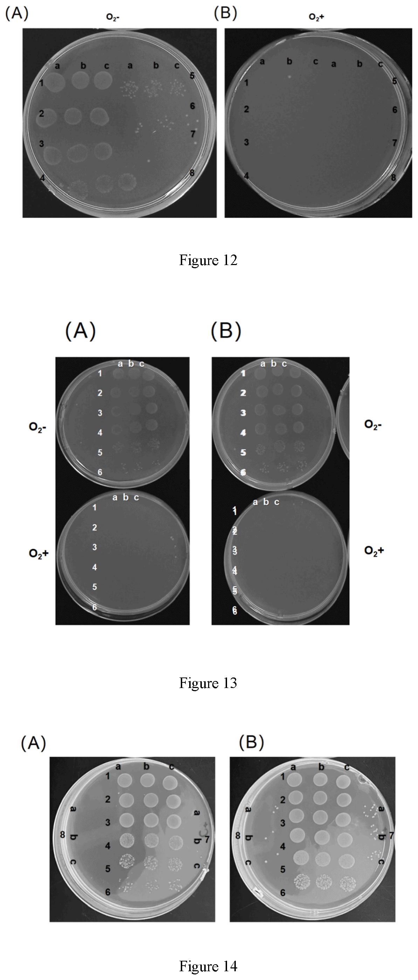

Figure 14 Results diagram of oxygen adaptability validation tests for various *Salmonella* strains under oxygen concentrations below 0.8%.
   (A) *Salmonella* strain SWT1001 cultured on LB plates;
   (B) *Salmonella* strain SWT1005 cultured on LB plates.

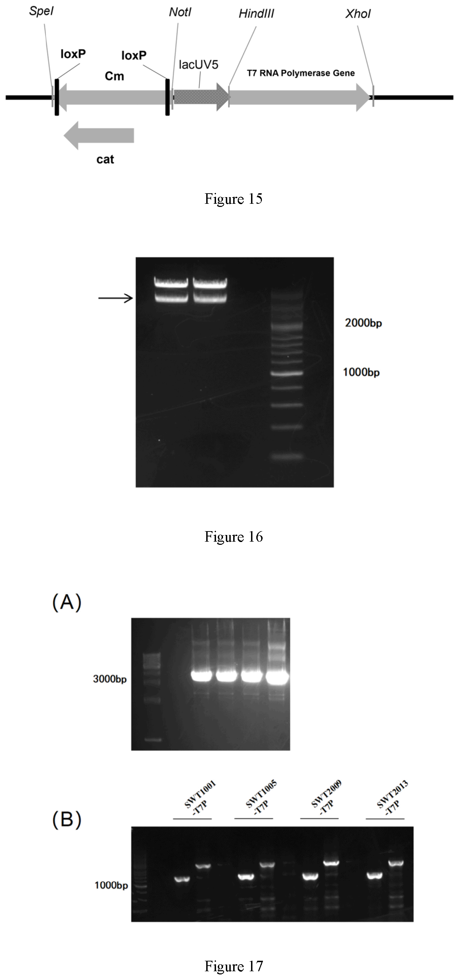

Figure 15 Schematic diagram of plasmid pPRO005, containing the chloramphenicol resistance gene, *lacUV5*, and T7 RNA polymerase gene cassette.
Figure 16 Verification diagram of the enzymatic digestion products of plasmid pPRO005.
   The arrow indicates the excised T7 RNA polymerase gene cassette fragment.
Figure 17 Verification diagram of *lacUV5*-controlled T7 RNA polymerase constitutive expression cassette recombined into the *Salmonella* chromosome.
   (A) PCR amplification fragments used for recombination (primers SWTO102 and SWTO103);
   (B) Verification diagram of PCR-amplification for strains SWT1001-T7P, SWT1005-T7P, SWT2009-T7P, SWT2013-T7P after construction.
Figure 18 Schematic diagram of the construction for plasmid expression vector pPRO010.
Figure 19 Verification diagram of PCR-amplification for plasmid pPRO010 in strains SWT5001, SWT5005, SWT6009, and SWT6013.
Figure 20 Schematic diagram of the construction for plasmid expression vector pPRO012.

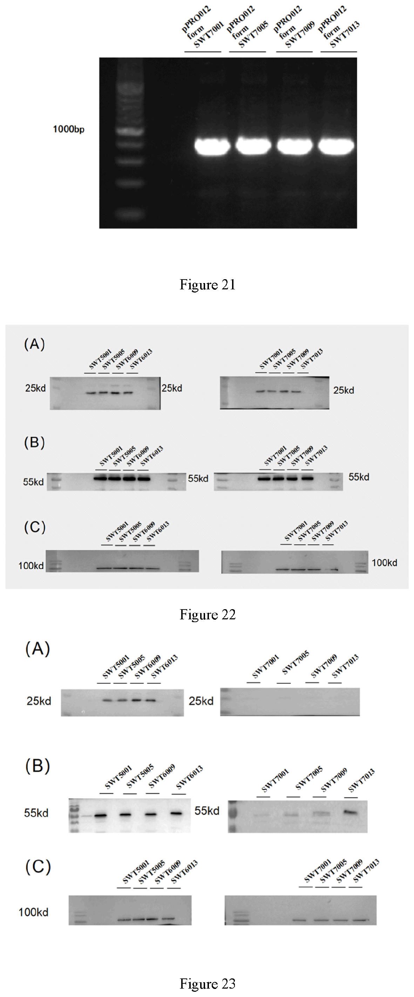

Figure 21 Verification diagram of PCR-amplification for plasmid pPRO012 in strains SWT7001, SWT7005, SWT7009, and SWT7013.
Figure 22 Identification diagram of Western Blot analysis for target gene expression in expression vector strains and control vector strains under culture conditions.
   (A) Expression of DTA in strains SWT5001, SWT5005, SWT6009, SWT6013 and strains SWT7001, SWT7005, SWT7009, SWT7013;
   (B) Expression of LLO in strains SWT5001, SWT5005, SWT6009, SWT6013 and strains SWT7001, SWT7005, SWT7009, SWT7013;
   (C) Expression of T7 RNA polymerase in strains SWT5001, SWT5005, SWT6009, SWT6013 and strains SWT7001, SWT7005, SWT7009, SWT7013.
Figure 23: Identification diagram of Western Blot analysis for target gene expression in expression vector strains and control vector strains in tumors within mice.
   (A) Expression of DTA in strains SWT5001, SWT5005, SWT6009, SWT6013 and strains SWT7001, SWT7005, SWT7009, SWT7013;
   (B) Expression of LLO in strains SWT5001, SWT5005, SWT6009, SWT6013 and strains SWT7001, SWT7005, SWT7009, SWT7013;
   (C) Expression of T7 RNA polymerase in strains SWT5001, SWT5005, SWT6009, SWT6013 and strains SWT7001, SWT7005, SWT7009, SWT7013.

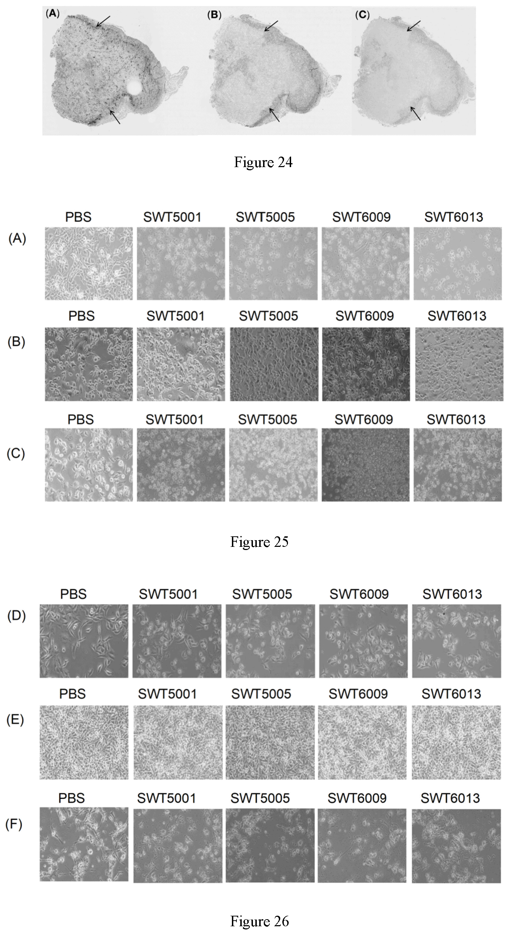

Figure 24: Identification diagram of histological sections for mouse tumors after treatment with strain SWT5005.
   Darkly stained areas indicated by arrows are antibody-positive regions;
   (A) Tumor section stained with anti-*Salmonella* antibody;
   (B) Tumor section stained with anti-HIF1α antibody;
   (C) Tumor section stained with anti-Diphtheria toxin antibody.
Figure 25 Microscopic images diagram showing cell killing after co-culture of strains SWT5001, SWT5005, SWT6009, and SWT6013 with cancer cells under anaerobic conditions.
   (A) EMT6;
   (B) K7M2;
   (C) Hepa1-6.
Figure 26 Microscopic images diagram showing cell killing after co-culture of strains SWT5001, SWT5005, SWT6009, and SWT6013 with cancer cells under anaerobic conditions.
   (D) A549;
   (E) B16F10;
   (F) Renca.

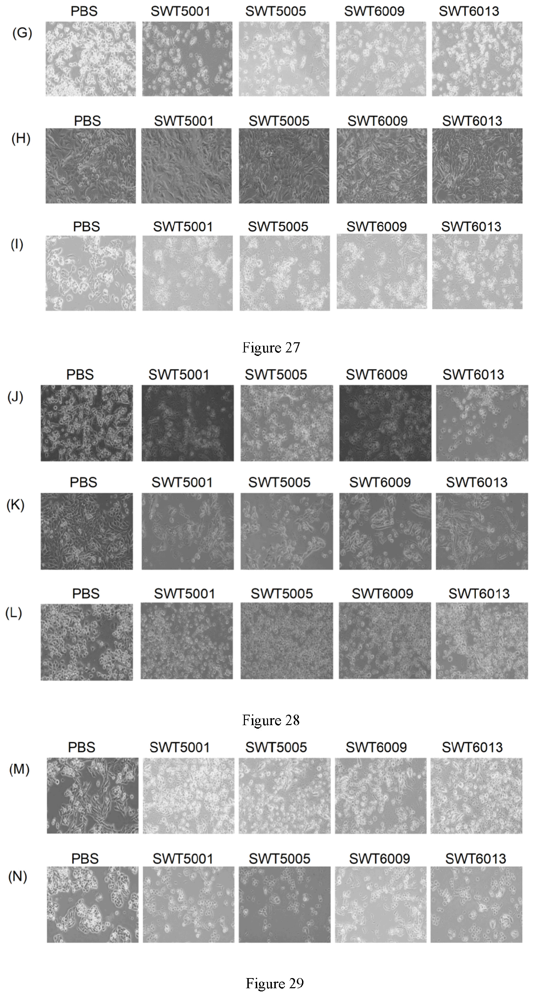

Figure 27 Microscopic images diagram showing cell killing after co-culture of strains SWT5001, SWT5005, SWT6009, and SWT6013 with cancer cells under anaerobic conditions.
   (G) MFC;
   (H) Pan02;
   (I) RM-1.
Figure 28 Microscopic images diagram showing cell killing after co-culture of strains SWT5001, SWT5005, SWT6009, and SWT6013 with cancer cells under anaerobic conditions.
   (J) CT26;
   (K) ID8;
   (L) Neuro-2a.
Figure 29 Microscopic images diagram showing cell killing after co-culture of strains SWT5001, SWT5005, SWT6009, and SWT6013 with cancer cells under anaerobic conditions.
   (M) SCC7;
   (N) MB49.

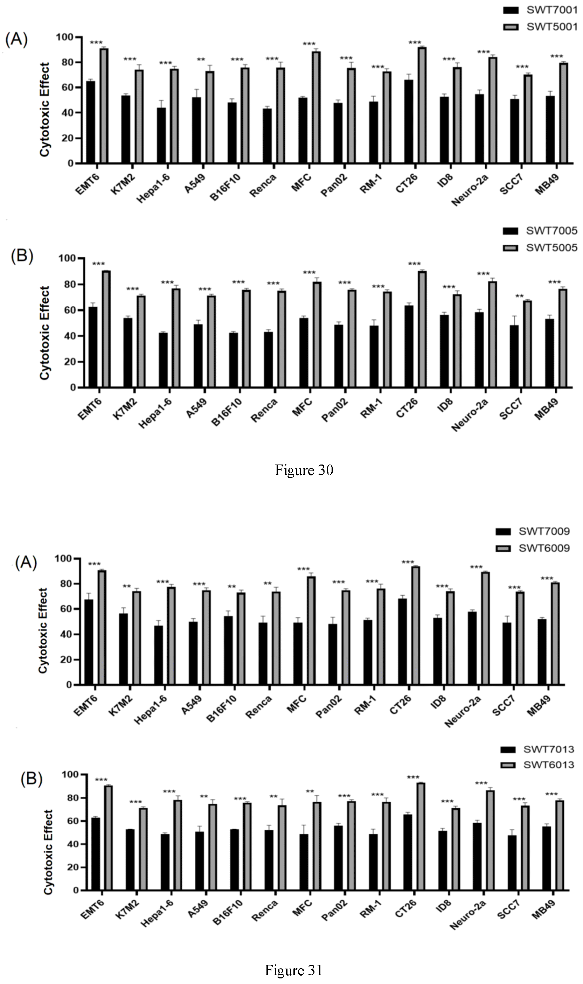

Figure 30 Killing effect diagram of CCK8 assay identifying for expression vector strains and control vector strains on EMT6, K7M2, Hepa1-6, A549, B16F10, Renca, MFC, Pan02, RM-1, CT26, ID8, Neuro-2a, SCC7, and MB49 cancer cells.
   (A) SWT5001 and SWT7001;
   (B) SWT5005 and SWT7005.
Figure 31 Killing effect diagram of CCK8 assay identifying for expression vector strains and control vector strains on EMT6, K7M2, Hepa1-6, A549, B16F10, Renca, MFC, Pan02, RM-1, CT26, ID8, Neuro-2a, SCC7, and MB49 cancer cells.
   (A) SWT6009 and SWT7009;
   (B) SWT6013 and SWT7013.

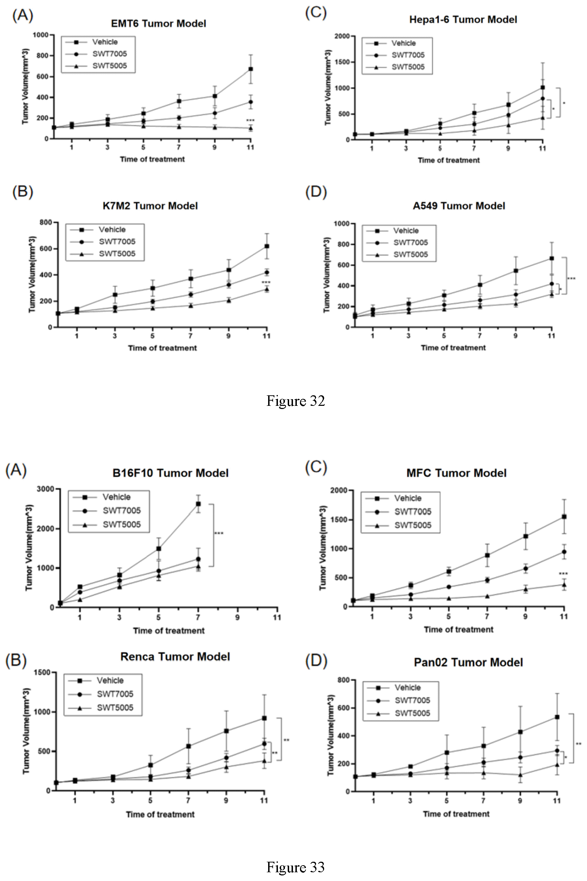

Figure 32 Identification diagram of inhibitory effect for expression vector strain SWT5005 and control vector strain SWT7005 in different mouse tumor models.
   Vehicle: blank control, no drug treatment;
   (A) EMT6;
   (B) K7M2;
   (C) Hepa1-6;
   (D) A549.
Figure 33 Identification diagram of inhibitory effect for expression vector strain SWT5005 and control vector strain SWT7005 in different mouse tumor models.
   Vehicle: blank control, no drug treatment;
   (A) B16F10;
   (B) Renca;
   (C) MFC;
   (D) Pan02.

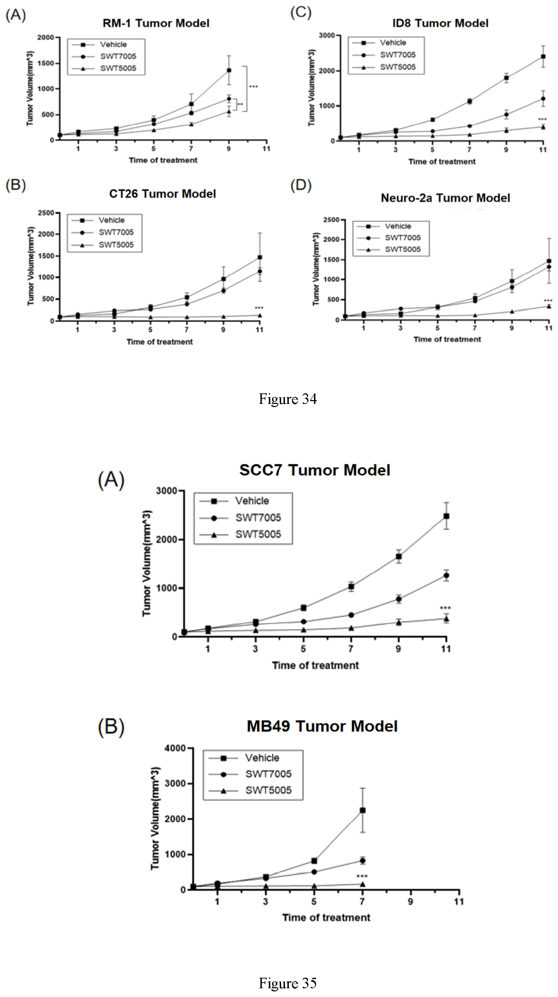

Figure 34 Identification diagram of inhibitory effect for expression vector strain SWT5005 and control vector strain SWT7005 in different mouse tumor models.
   Vehicle: blank control, no drug treatment;
   (A) RM-1;
   (B) CT26;
   (C) ID8;
   (D) Neuro-2a.
Figure 35 Identification diagram of inhibitory effect for expression vector strain SWT5005 and control vector strain SWT7005 in different mouse tumor models.
   Vehicle: blank control, no drug treatment;
   (A) SCC7;
   (B) MB49.

### Detailed Description of the Embodiments

The present application will be further described in detail below with reference to the accompanying drawings and embodiments. Through these descriptions, the features and advantages of the present application will become clearer and more apparent.

### Example 1: Construction of hypoxia-specific gene expression cassette using the alr gene and dadX gene as survival-essential gene

In this embodiment, hypoxia-specific gene expression cassettes were constructed by using the following combinations: The forward hypoxia promoter employed was the *Salmonella yhbU(yhbU-S)* (SEQ ID No. 31), the survival-essential gene was the *Salmonella alr* gene (SEQ ID No. 27), and the reverse hyperoxia promoters were, respectively, the *Salmonella cyoA (cyoA-S)* (SEQ ID No. 33) and the *Salmonella ydcI (ydcI-S)* (SEQ ID No. 34). Additionally, the forward hypoxia promoter employed was the *Escherichia coli ynfK (ynfK-E)* (SEQ ID No. 32), the survival-essential gene was the *Salmonella dadX* gene (SEQ ID No. 29), and the reverse hyperoxia promoters were, respectively, the *Salmonella cyoA (cyoA-S)* (SEQ ID No. 33) and the *Salmonella ydcI(ydcI-S)* (SEQ ID No. 34).

The combinations for the hypoxia-specific gene expression cassette are shown in Table 1.

**Table 1. Combination Table of Forward Hypoxia Promoters and Reverse Hyperoxia Promoters (wherein the survival-essential gene are the alr or dadX gene from Salmonella)**

| | *cyoA-S* | *ydcI-S* |
|---|---|---|
| *yhbU-S* | pOL1001 | pOL1005 |
| *ynfK-E* | pOL2009 | pOL2013 |

### (I) Construction of the Forward Hypoxia Promoter Clone Library

1. A single colony of wild-type *Salmonella Typhimurium* (strain SWT001, purchased from the China Center for Industrial Culture Collection, CICC) or *Escherichia coli* DH10B (purchased from Shanghai Weidi Biotechnology Co., Ltd.) was picked and inoculated into 5 ml of LB broth. The culture was then incubated in a shaking incubator at 37°C and 220 rpm for 16 hours, until the OD₆₀₀ was between 2 and 3.
2. Two microliters (2 µl) of the corresponding bacterial culture were added to a reaction mixture containing the corresponding primers and a high-fidelity PCR enzyme (purchased from Takara), and the mixture was placed in a PCR amplification equipment.
   The PCR program was as follows: 95°C for 2 minutes; followed by 30 cycles of 95°C for 30 seconds, 60°C for 30 seconds, and 72°C for 60 seconds; 72°C for 10 minutes; and holding at 4°C for 5 minutes.
3. The amplified PCR product was purified and recovered using a DNA gel purification kit.
4. The recovered product was mixed with a restriction digestion mixture containing restriction enzymes (purchased from NEB) and incubated at 37°C for 1 hour.
5. The digestion product was then purified and recovered using a DNA gel purification kit.

The primers and restriction enzymes used for the different forward hypoxia promoters were as follows:
1. *yhbU-S* (SEQ ID NO: 31): The promoter was amplified from strain SWT001 using primers SWTO19 and SWTO20. The product was then double-digested with NotI and HindIII and recovered.
2. *ynfK-E* (SEQ ID NO: 32): The promoter was amplified from *Escherichia coli* strain DH10B using primers SWTO21 and SWTO22. The product was then double-digested with NotI and HindIII and recovered.

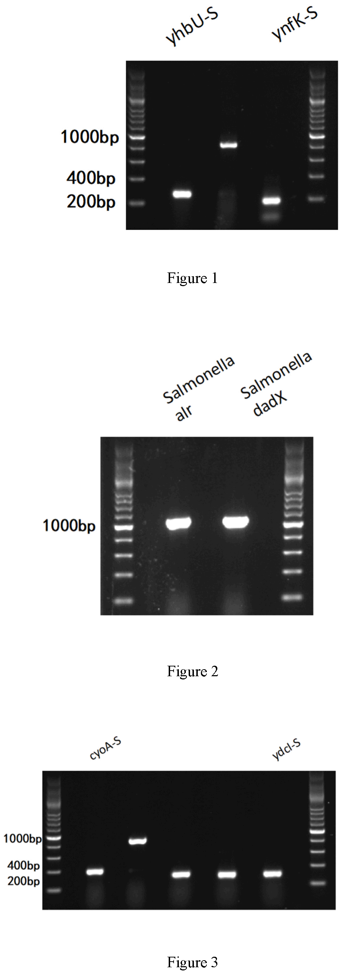

The result of the verification of the forward hypoxia promoter clone library by agarose gel electrophoresis is shown in Figure 1.

### (II) Construction of the Survival-Essential Gene Library

1. A single colony of wild-type *Salmonella Typhimurium* (strain SWT001, purchased from the China Center for Industrial Culture Collection, CICC) was picked and inoculated into 5 ml of LB broth. The culture was then incubated in a shaking incubator at 37°C and 220 rpm for 16 hours.
2. Two microliters (2 µl) of the corresponding bacterial culture were added to a reaction mixture containing the corresponding primers and a high-fidelity PCR enzyme, and the mixture was placed in a PCR amplification equipment.
   The PCR program was as follows: 95°C for 2 minutes; followed by 30 cycles of 95°C for 30 seconds, 60°C for 30 seconds, and 72°C for 60 seconds; 72°C for 10 minutes; and holding at 4°C for 5 minutes.
3. The amplified PCR product was purified and recovered using a DNA gel purification kit.
4. The recovered product was mixed with a restriction digestion mixture containing restriction enzymes and incubated at 37°C for 1 hour.
5. The digestion product was then purified and recovered using a DNA gel purification kit.

The different survival-essential genes were as follows:
1. *Salmonella alr* gene (SEQ ID NO: 27): The gene was amplified from strain SWT001 using primers SWTO35 and SWTO36 The product was then double-digested with HindIII and XhoI and recovered.
2. *Salmonella dadX* gene (SEQ ID NO: 29): The gene was amplified from strain SWT001 using primers SWTO39 and SWTO40. The product was then double-digested with HindIII and XhoI and recovered.

The result of the verification of the survival-essential gene library by agarose gel electrophoresis is shown in Figure 2.

### (III) Construction of the Reverse Hyperoxia Promoter Library

1. A single colony of wild-type *Salmonella Typhimurium* (strain SWT001, purchased from the China Center for Industrial Culture Collection, CICC) was picked and inoculated into 5 ml of LB broth. The culture was then incubated in a shaking incubator at 37°C and 220 rpm for 16 hours.
2. Two microliters (2 µl) of the corresponding bacterial culture were added to a reaction mixture containing the corresponding primers and a high-fidelity PCR enzyme, and the mixture was placed in a PCR amplification equipment.

The PCR program was as follows: 95°C for 2 minutes; followed by 30 cycles of 95°C for 30 seconds, 60°C for 30 seconds, and 72°C for 60 seconds; 72°C for 10 minutes; and holding at 4°C for 5 minutes.
3. The amplified PCR product was purified and recovered using a DNA gel purification kit.
4. The recovered product was mixed with a restriction digestion mixture containing restriction enzymes and incubated at 37°C for 1 hour.
5. The digestion product was then purified and recovered using a DNA gel purification kit.

The primers and restriction enzymes used for the different reverse hyperoxia promoters were as follows:
1. *cyoA-S* (SEQ ID NO: 33): The promoter was amplified from strain SWT001 using primers SWTO43 and SWTO44. The product was then double-digested with XhoI and PstI and recovered.
2. *ydcI-S* (SEQ ID NO: 34): The promoter was amplified from strain SWT001 using primers SWTO51 and SWTO52. The product was then double-digested with XhoI and PstI and recovered.

The result of the verification of the reverse hyperoxia promoter clone library by agarose gel electrophoresis is shown in Figure 3.

### (IV) Construction of a Combinatorial Library of Forward Hypoxia Promoters, Survival-Essential Genes, and Reverse Hyperoxia Promoters

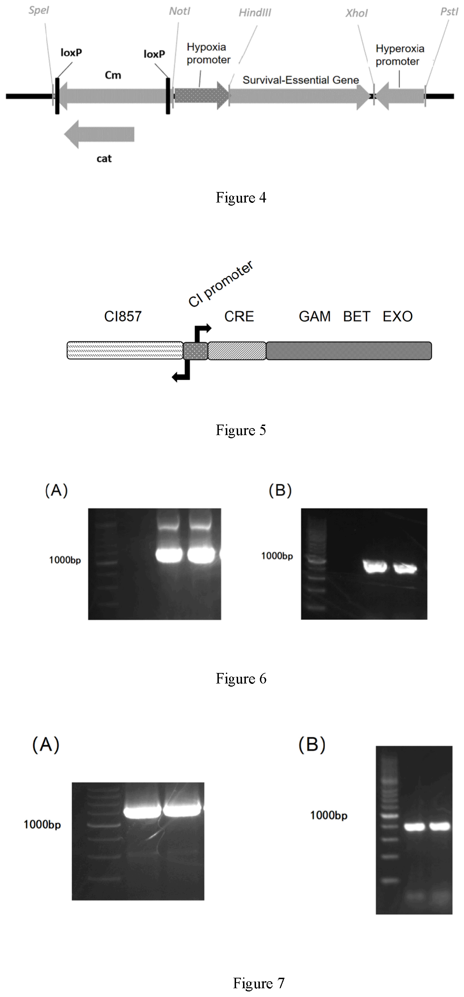

The composition of the hypoxia-specific gene expression cassette is shown in Figure 4. It is composed of a forward hypoxia promoter, an survival-essential gene, and a reverse hyperoxia promoter, arranged in sequence.

As shown in Table 1, the cloned library was formed by combining the following sequences: the forward hypoxia promoter library digested with NotI and HindIII; the survival-essential *Salmonella alr* gene (SEQ ID NO: 27) or *Salmonella dadX* gene (SEQ ID NO: 29) digested with HindIII and XhoI; and the reverse hyperoxia promoter library digested with XhoI and PstI. These were ligated into the plasmid pSWT003 vector (vector backbone: pBlueScript SK(+), purchased from Biofeng), which had been digested with SpeI and PstI, and the plasmid pSWT007 vector (containing bilateral co-directional *loxP* sequences and a DNA fragment of the chloramphenicol resistance gene (SEQ ID NO: 37)), which had been digested with SpeI and NotI.

The products mentioned above obtained by primer amplification from *Salmonella* SWT001 or *Escherichia coli* DH10B followed by enzymatic digestion and recovery, were subjected to combinatorial ligation via an enzymatic ligation reaction. The ligation products were transformed into DH10B and spread onto LB plates containing 25 µg/mL chloramphenicol to obtain the corresponding plasmids shown in Table 1.

### (V) Construction of the strain corresponding to this invention

### 1. Wild-type Salmonella typhimurium (SWT001)

Wild-type *Salmonella typhimurium* (SWT001) was purchased from the China Center of Industrial Culture Collection (CICC).

### 2. Construction of Salmonella (SWT002) containing a temperature-inducible Lambda-RED recombinase and a loxP-CRE enzyme system

The Lambda-RED recombination system is widely used for homologous recombination in Gram-negative bacteria. In the present invention, this system consists of plasmid pSWT001.

As shown in Figure 5, plasmid pSWT001 contains a Lambda-RED recombinase module (SEQ ID NO: 38) (functionally similar to the plasmid vector *p*sim6 from Biofeng) and a *loxP*-Cre recombinase module (SEQ ID NO: 38) (functionally similar to the plasmid vector 705-Cre from Gene Bridges). The Lambda-RED recombinase module is composed of three recombinases: EXO, BET, and GAM, and the recombinases are controlled by the CI857 temperature-sensitive regulator. Therefore, they cannot be expressed under culture conditions at 32°C and are expressed only at temperatures higher than 37°C. Under temperature-induced conditions, the EXO recombinase cleaves the 5' end of double-stranded linear DNA, creating single-stranded DNA with a protruding 3' end. The single-stranded DNA is bound by the BET protein and protected from degradation by other nucleases. The function of GAM is to inhibit bacterial endogenous nuclease activity. The homology arms for homologous recombination are approximately 35-50 bp in length and are added to both sides of the DNA fragment to be recombined via PCR. The advantage of this technology is that it precisely targets the region of interest in the bacterial chromosome without causing extra mutations. The outermost sides of the recombined double-stranded DNA fragment include the homology arm sequences for the recombination site, while the interior contains two *loxP* sequences in the same orientation, totaling 34 bp. Located between the two co-directional *loxP* sites is a chloramphenicol resistance gene used for screening recombinant bacteria. After successful recombination, the CRE enzyme system carried on the pSWT001 plasmid (also controlled by the CI857 temperature-sensitive regulator) specifically recognizes the *loxP* sequences and creates a cut within the sequence between the co-directional *loxP* sites, leaving one *loxP* sequence behind, thereby eliminating the chloramphenicol resistance gene. Repeating the above operations allows for continuous gene knockout and knock-in.

The specific operational steps are as follows:
(1) Strain SWT001 was streaked onto an LB plate and cultured overnight in a constant temperature incubator at 37°C;
(2) A single colony was picked and inoculated into 5 mL of LB liquid medium, then placed in a constant temperature shaker at 37°C and 220 rpm for 16 hours;
(3) The culture was inoculated into fresh LB liquid medium at a ratio of 1:100 and cultured for a further 2-3 hours until the bacterial density reached OD₆₀₀ = 0.3, then placed on ice for 1 hour;
(4) The bacterial cells were washed 3 times with sterilized purified water;
(5) The recovered bacterial cells were mixed with 10 ng of plasmid pSWT001 and electroporated at a voltage of 1.8 kV;
(6) The electroporated bacterial cells were spread onto LB plates containing 100 µg/mL ampicillin sodium and cultured overnight in a constant temperature incubator at 32°C until single colonies grew. The resulting strain was named SWT002.

### 3. Construction of aroA Gene-Knockout Attenuated Salmonella (SWT003)

(1) A single colony of SWT002 was picked and inoculated into 5 mL of LB liquid medium containing 100 µg/mL ampicillin sodium, and cultured in a constant temperature shaker at 32°C and 220 rpm for 16 hours.
(2) The culture was inoculated at a ratio of 1:100 into fresh LB liquid medium containing 100 µg/mL ampicillin sodium and cultured for a further 2-3 hours. When the bacterial density reached an OD₆₀₀ of 0.3, the culture flask was placed in a 42°C water bath and incubated with shaking for 15 minutes, then placed on ice for 1 hour.
(3) The bacterial cells were washed 3 times with sterilized purified water.
(4) Preparation of PCR products using primers SWTO1 and SWTO2:
   Primers SWTO1 and SWTO2 were mixed with plasmid pSWT002 (SEQ ID No.39, containing bilateral co-directional *loxP* sequences with a chloramphenicol resistance gene in between) and a high-fidelity PCR amplification enzyme system, and placed into PCR amplification equipment.

The amplification program was: 95°C for 2 minutes; 30 cycles of 95°C for 30 seconds, 60°C for 30 seconds, and 72°C for 60 seconds; followed by 72°C for 10 minutes; and 4°C for 5 minutes.

The amplified PCR products were verified by agarose gel electrophoresis. The verification results of the PCR amplification products of the *aroA* gene knockout fragment using SWTO1 and SWTO2 are shown in Figure 6A.

The products were purified and recovered using a DNA gel extraction system. The concentration and purity of the recovered PCR products were determined using a nanodrop.

(5) The recovered bacterial cells were mixed with 100 ng of the PCR products generated by SWTO1 and SWTO2, and electroporated at a voltage of 1.8 kV.

(6) The electroporated bacterial cells were spread onto plates containing 25 µg/mL chloramphenicol and cultured overnight in a constant temperature incubator at 32°C until single colonies grew.

(7) Positive clones were identified by colony PCR. Primers SWTO4 and SWTO6, and SWTO3 and SWTO5 were used to verify the insertion of the chloramphenicol resistance gene.

The PCR amplification products of the target fragment of strain SWT003 were verified by agarose gel electrophoresis, and the verification results are shown in Figure 6B.

(8) The positive single colonies were inoculated into 5 mL of LB medium and cultured in a constant temperature shaker at 37°C and 220 rpm for 16 hours to allow the CRE enzyme to function and excise the chloramphenicol resistance gene.

### 4. Construction of alr Gene-Knockout Attenuated Salmonella (SWT004)

(1) A single colony of SWT003 was picked and inoculated into 5 mL of LB liquid medium, and cultured in a constant temperature shaker at 32°C and 220 rpm for 16 hours.
(2) The culture was inoculated at a ratio of 1:100 into fresh LB liquid medium and cultured for a further 2-3 hours. When the bacterial density reached an OD₆₀₀ of 0.3, the culture flask was placed in a 42°C water bath and incubated with shaking for 15 minutes, then placed on ice for 1 hour.
(3) The bacterial cells were washed 3 times with sterilized purified water.
(4) Preparation of PCR products using primers SWTO70 and SWTO71:
   Primers SWTO70 and SWTO71 were mixed with plasmid pSWT002 and a high-fidelity PCR amplification enzyme system, and placed into PCR amplification equipment.

The amplification program was: 95°C for 2 minutes; 30 cycles of 95°C for 30 seconds, 60°C for 30 seconds, and 72°C for 60 seconds; followed by 72°C for 10 minutes; and 4°C for 5 minutes.

The amplified PCR products were purified and recovered using a DNA gel extraction system. The concentration and purity of the recovered PCR products were determined using a nanodrop.

(5) The recovered bacterial cells were mixed with 100 ng of the PCR products generated by SWTO70 and SWTO71, and electroporated at a voltage of 1.8 kV.

The PCR amplification products of the *alr* gene knockout fragment using SWTO70 and SWTO71 were verified by agarose gel electrophoresis, and the verification results are shown in Figure 7A.

(6) The electroporated bacterial cells were spread onto plates containing 25 µg/mL chloramphenicol and cultured overnight in a constant temperature incubator at 32°C until single colonies grew.

(7) Positive clones were identified by colony PCR. Primers SWTO72 and SWTO5, and SWTO6 and SWTO73 were used to verify the insertion of the chloramphenicol resistance gene.

The PCR amplification products of the target fragment of strain SWT004 were verified by agarose gel electrophoresis, and the verification results are shown in Figure 7B (primers used: SWTO72, SWTO5, SWTO6, SWTO73).

(8) The positive single colonies were inoculated into 5 mL of LB medium and cultured in a constant temperature shaker at 37°C and 220 rpm for 16 hours to allow the CRE enzyme to function and excise the chloramphenicol resistance gene.

### Example 2: Integration of the Hypoxia-Specific Gene Expression Cassette into the dadX Gene Locus on the Chromosome of Strain SWT004 (Disrupting its Function)

### Construction of alr-Deficient Attenuated Bacteria Containing the Hypoxia-Specific Gene Expression Cassette

1. A single colony of the *alr* gene-knockout attenuated bacteria SWT004 was picked and inoculated into 5 mL of fresh LB liquid medium, and cultured in a constant temperature shaker at 32°C and 220 rpm for 16 hours.
2. The culture was inoculated at a ratio of 1:100 into fresh LB liquid medium and cultured for a further 2-3 hours until the bacterial density reached an OD₆₀₀ of 0.3. The culture flask was then placed in a 42°C water bath and incubated with shaking for 15 minutes, followed by resting on ice for 1 hour.
3. The bacterial cells were washed 3 times with sterilized purified water.
4. Preparation of PCR products for constructing strains containing the hypoxia-specific expression cassette: Primers SWTO78 and SWTO79 were mixed with the hypoxia-specific gene expression cassette plasmid (pOL1001, pOL1005) and a high-fidelity PCR amplification enzyme system, and placed into PCR amplification equipment.

The amplification program was: 95°C for 2 minutes; 30 cycles of 95°C for 30 seconds, 60°C for 30 seconds, and 72°C for 120 seconds; followed by 72°C for 10 minutes; and 4°C for 5 minutes.

The amplified PCR products were purified and recovered using a DNA gel extraction system. The concentration and purity of the recovered PCR products were determined using a nanodrop.

5. The recovered bacterial cells were mixed with 100 ng of the PCR product and electroporated at a voltage of 1.8 kV.

6. The electroporated bacterial cells were spread onto plates containing 25 µg/mL chloramphenicol and 100 µg/mL D-alanine, and cultured overnight in a constant temperature incubator at 32°C until single colonies grew and the generated strains are SWT1001 and SWT1005, respectively..

7. A single colony was inoculated into 5 mL of fresh LB liquid medium containing 100 µg/mL D-alanine and cultured in a constant temperature shaker at 37°C and 220 rpm for 16 hours to allow the CRE enzyme to function and excise the chloramphenicol resistance gene.

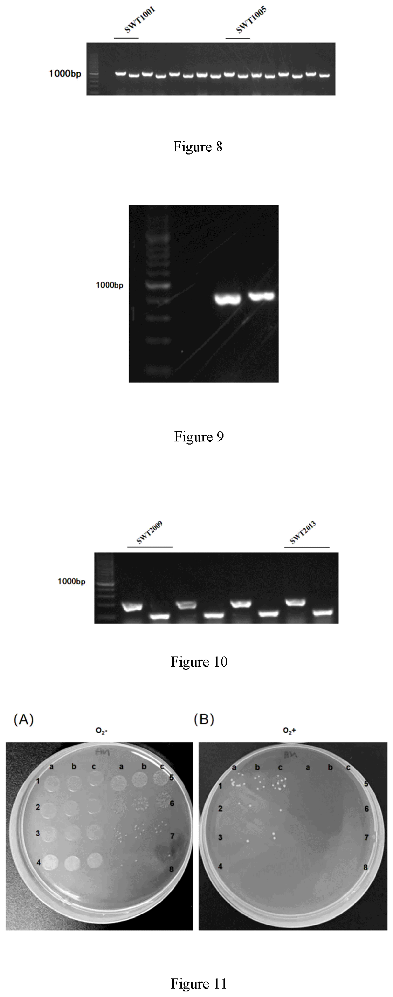

8. Clones were identified by colony PCR using the corresponding primers SWTO61, 20 and SWTO75, 20. The identification results of strain SWT1001 (*yhbU-S + alr + cyoA-S* combination) and strain SWT1005 (*yhbU-S + alr + ydcI-S* combination) are shown in Figure 8.

### Example 3: Construction of the Hypoxia-Specific Gene Expression Cassette with dadX as the Survival-essential Gene

As alanine racemase, the genomes of bacteria such as *Salmonella* and *E. coli* contain two isoenzyme genes, namely the *alr* gene and the *dadX* gene. To verify the effect, the survival-essential gene in the aforementioned hypoxia-specific gene expression cassettes was accordingly replaced with the homologous *dadX* gene in plasmids pOL2009 and pOL2013.

### Integration of the Hypoxia-Specific Gene Expression Cassette into the alr Gene Locus on the Chromosome of Strain SWT007

### 1. Construction of the dadX Gene Knockout Attenuated Bacterium (SWT007)

(1) A single colony of SWT003 was picked and inoculated into 5 ml of LB liquid medium, and placed in a constant temperature shaker at 32°C and 220 rpm for 16 hours of cultivation.
(2) The culture was inoculated into fresh LB liquid medium at a ratio of 1:100 and cultivation were continued for 2-3 hours until the bacterial density grew to an OD₆₀₀=0.3, at which point the culture flask was placed in a 42°C water bath and incubated with shaking for 15 minutes, then placed on ice to stand for 1 hour.
(3) The bacterial cells were washed 3 times with sterilized pure water.
(4) Preparation of the PCR product of SWTO78 and SWTO79.

Primers SWTO78 and SWTO79 were mixed with plasmid pSWT002 (as described above) and a high-fidelity PCR amplification enzyme system, and placed into a PCR amplification instrument.

The amplification program was: 95°C for 2 minutes; 30 cycles of 95°C for 30 seconds, 60°C for 30 seconds, 72°C for 60 seconds; 72°C for 10 minutes; 4°C for 5 minutes.

The amplified PCR product was purified and recovered using a DNA gel recovery system, and the concentration and purity of the recovered PCR product DNA were measured using a nanodrop.
(5) The recovered bacterial cells were mixed with 100 ng of the PCR product of SWTO78 and SWTO79, and electroporated at a voltage of 1.8 kV.
(6) The electroporated bacterial cells were spread onto plates containing 25 µg/mL chloramphenicol and cultured overnight in a constant temperature incubator at 32°C until single colonies grew.
(7) Positive clones were identified through colony PCR using primers SWTO59, SWTO5, SWTO6, and SWTO87 to verify the insertion of the chloramphenicol resistance gene, as shown in figure 9.
(8) The positive single clones were inoculated into 5 ml of LB medium and cultured for 16 hours in a constant temperature shaker at 37°C and 220 rpm, allowing the Cre recombinase to act and excise the chloramphenicol resistance gene.

### 2. Construction of a Deficient and Attenuated Bacterial Strain Containing the Hypoxia-Specific Gene Expression Cassette and with the Salmonella dadX Gene as the Survival-Essential Gene

(1) A single colony of the *dadX* gene knockout attenuated bacterium, SWT007, was picked and inoculated into 5 ml of LB liquid medium, and cultured for 16 hours in a constant temperature shaker at 32°C and 220 rpm.
(2) The culture was inoculated into fresh LB liquid medium at a ratio of 1:100, and cultivation was continued for 2-3 hours until the bacterial density reached an OD₆₀₀ of 0.3, at which point the culture flask was placed in a 42°C water bath and incubated with shaking for 15 minutes, then placed on ice to stand for 1 hour.
(3) The bacterial cells were washed 3 times with sterilized pure water.
(4) To prepare the PCR product for constructing the strain containing the hypoxia-specific gene expression cassette, primers SWTO85 and SWTO86 were mixed with the hypoxia-specific gene expression cassette plasmid (pOL2009,pOL2013) and a high-fidelity PCR amplification enzyme system, and the mixture was placed into a PCR amplification instrument.

The amplification program was: 95°C for 2 minutes; 30 cycles of 95°C for 30 seconds, 60°C for 30 seconds, and 72°C for 120 seconds; followed by 72°C for 10 minutes; and 4°C for 5 minutes.

The amplified PCR product was purified and recovered using a DNA gel recovery system, and the concentration and purity of the recovered PCR product DNA were measured using a Nanodrop spectrophotometer.

(5) The recovered bacterial cells were mixed with 100 ng of the PCR product, and electroporation was performed at a voltage of 1.8 kV.

(6) The electroporated cells were plated on plates containing 25 µg/ml chloramphenicol and 100 µg/ml D-alanine, and incubated overnight in a constant temperature incubator at 32°C until single colonies grew.

(7) The positive single colony was inoculated into 5 ml of LB medium and cultured for 16 hours in a constant temperature shaker at 37°C and 220 rpm, allowing the CRE recombinase to act and excise the chloramphenicol resistance gene.

(8) Clones were identified by colony PCR using the corresponding primers SWTO98,22 and SWTO99, 22. The identification results of strain SWT2013 (*ynfK-E+dadX+ydcI-S*) and strain SWT2009 (*ynfK-E+dadX+cyoA-S*) are shown in Figure 10.

### Example 4: Verification of Oxygen Adaptability of the Salmonella Strain SWT1001, SWT1005, SWT2009, SWT2013 Containing Hypoxia-Specific Gene Expression Cassette

1. Single colonies of strains SWT1001, SWT1005, SWT2009, SWT2013 containing the hypoxia-specific expression cassette were picked and inoculated into 5 mL of fresh LB liquid medium containing 100 µg/mL D-alanine, and cultured in a constant temperature shaker at 37°C and 200 rpm for 16 hours.
2. After culturing was completed, the strains were diluted 10-fold, and the absorbance (OD value) at 600 nm was measured.
3. The volume of bacterial culture corresponding to 1 OD of bacteria was calculated according to the following formula, and deionized water was added to reach 1 mL.
4. The above bacterial suspension was taken
   Volume of bacterial culture for 1 OD = 1/(Absorbance × 10) × 1000 spotted onto plates for culture, labeled as "1". This was repeated three times, labeled as "a", "b", and "c".
5. After performing a 10-fold serial dilution, 10 µL of the diluted solution was taken and spotted onto the above medium, labeled as "2".
6. This 10-fold serial dilution was continued in the same manner until labeled "8".
7. One of the plates was placed in an anaerobic environment at 37°C for culture, and the other plate was placed in an atmospheric environment (21% oxygen concentration) at 37°C for culture.

The verification test results of the oxygen adaptability of the strains containing the hypoxia-specific expression cassette are shown in Figures 11-13. The results demonstrate that *Salmonella* strains SWT1001, SWT1005, SWT2009, and SWT1023-each harboring the described hypoxia-specific gene expression cassette-exhibit hypoxia-regulated capability.

### Example 5: Oxygen Concentration Simulation for the Strain Library Containing Hypoxia-Specific Gene Expression Cassette

Oxygen in the sealed culture jar is consumed by using an anaerobic gas-generating bag, and the oxygen concentration in the sealed culture jar is determined using an oxygen meter. After using the anaerobic gas-generating bag for a certain period, the oxygen concentration in the sealed culture jar can be fixed within a specific concentration range. The specific method is as follows:
An anaerobic gas-generating bag (Brand: Mitsubishi, Japan; Cat. No.: D-119) combination was placed into a 7.0 L sealed culture jar (Brand: Mitsubishi, Japan; Cat. No.: D-112), and an oxygen meter (Brand: Meicheng Electronics; Cat. No.: OX-100A) was placed inside simultaneously.

The above-mentioned anaerobic gas-generating bag combination was used to verify the growth status of the genetically modified strains under an oxygen concentration below 0.8%.

### 1. Adaptability Verification of the Strain Library Containing Hypoxia-Specific Gene Expression Cassettes Under an Oxygen Concentration Below 0.8%

Since the range of the pathological hypoxia zone in tumors corresponds to an oxygen concentration of less than 1%, the present invention intends to demonstrate that *Salmonella* modified with the hypoxia-specific gene expression cassette can still grow normally when the oxygen concentration is less than 1% (or approaches 1%).

The regulation of oxygen concentration was achieved using an anaerobic gas-generating bag. After consumption by the anaerobic gas-generating bag for 1 hour, the reading on the oxygen meter indicated that the oxygen concentration was between 0.8% and 1%. Therefore, this experiment simulated the intratumoral hypoxic environment to verify the growth of *Salmonella* on culture plates after the anaerobic gas-generating bag had consumed oxygen in the sealed culture jar for 1 hour, thereby simulating the growth conditions of *Salmonella* strains containing the hypoxia-specific gene expression cassette within the intratumoral hypoxic environment.

The operational procedure for the spotting assay is as follows:
(1) Single colonies of strains containing the hypoxia-specific expression cassette were picked and inoculated into 5 mL of fresh LB liquid medium containing 100 µg/mL D-alanine, and cultured in a constant temperature shaker at 37°C and 200 rpm for 16 hours.
(2) After culturing was completed, the strains were diluted 10-fold, and the absorbance (OD value) at 600 nm was measured.
(3) The volume of bacterial culture corresponding to 1 OD of bacteria was calculated according to the following formula, and deionized water was added to reach 1 mL.$Volume of bacterial culture for 1 OD = 1 / \left(Absorbance\times 10\right) \times 1000$
(4) 10 µL of the above bacterial suspension was taken and spotted onto an LB plate containing D-alanine for culture, labeled as "1". This was repeated three times, labeled as "a", "b", and "c".
(5) Simultaneously, 10 µL of the above bacterial suspension was taken and spotted onto another LB plate without D-alanine for culture, labeled as "1" with three replicates labeled as "a", "b", and "c".
(6) After performing a 10-fold serial dilution, 10 µL of the diluted solution was taken and spotted onto the above media, labeled as "2".
(7) This 10-fold serial dilution was continued in the same manner until labeled "8".
(8) The two plates were placed in an environment with 0.8% oxygen at 37°C for culture.

As shown in Figure 14, the present invention compared the growth of different strains (Figure 14(A) SWT1001, Figure 14(B) SWT1005) on LB plates without supplemented D-alanine to judge the growth status of each strain under an oxygen concentration below 0.8%. Since the medium was not supplemented with D-alanine, the corresponding *Salmonella* strains relied on their own oxygen regulation systems to grow normally. The results confirmed that strains containing the hypoxia-specific expression cassette, which can grow normally in an anaerobic environment, can all grow normally under an oxygen concentration below 0.8%.

### Example 6: Construction of Salmonella Strains (SWT1001-T7P, SWT1005-T7P, SWT2009-T7P, SWT2013-T7P) with T7 RNA Polymerase Homologously Replaced at the asd Gene Locus

### (I) Construction of Plasmid pPRO005 for Constitutive Expression of T7 RNA Polymerase Controlled by lacUV5

To construct a constitutively expressed T7 RNA polymerase, the plasmid vector pPRO005 was generated. The process is illustrated in Figure 15. The constitutive expression promoter *lacUV5* was positioned upstream of the ribosome binding site (RBS) sequence (SEQ ID No. 35) and the T7 RNA polymerase gene (SEQ ID No. 4). Furthermore, a chloramphenicol resistance gene (*cm*) (SEQ ID No. 37) flanked by *loxp* sequences on both sides was inserted upstream of *lacUV5*. This was done to facilitate subsequent homologous recombination into the *Salmonella* chromosome and for selection purposes.
1. Plasmid pSWT007 (synthesized by Beijing Liuhe Huada Gene Technology Co., Ltd.) was digested with SpeI and NotI restriction enzymes. An approximately 1100 bp fragment containing the chloramphenicol resistance gene (*cm*) flanked by *loxp* sequences was recovered.
2. Primers SWTO100 and SWTO101 were directly annealed to generate NotI and HindIII restriction sites, and an approximately 70 bp fragment comprising the lacUV5 promoter was recovered.
3. Plasmid pPRO004 (synthesized by Beijing Liuhe Huada Gene Technology Co., Ltd.) was digested with HindIII and XhoI restriction enzymes. An approximately 2690 bp fragment containing the T7 RNA polymerase gene was recovered.
4. Plasmid pSWT003 (plasmid backbone) was digested with SpeI and XhoI restriction enzymes. An approximately 3000 bp fragment was recovered.
5. The aforementioned five fragments were ligated using T4 DNA ligase. The ligation product was then transformed into DH10B competent cells (purchased from Shanghai Weidi Biotechnology Co., Ltd.). The transformed cells were plated onto LB agar plates containing both ampicillin and chloramphenicol for double antibiotic selection, and single colonies were screened and designated pPRO005.
6. Plasmid pPRO005 was extracted, and 100 ng of the extracted plasmid was mixed with NotI and XhoI restriction enzymes. The mixture was incubated at 37 °C for 1 hour and subsequently identified by agarose gel electrophoresis, as shown in Figure 16.

### (II) Recombination-Mediated Integration of the lacUV5-Controlled Constitutive T7 RNA Polymerase Expression Cassette into the Salmonella Chromosome

1. Single colonies of SWT1001, SWT1005, SWT2009, and SWT2013 were separately picked and inoculated into 5 mL LB liquid medium containing 100 µg/mL D-alanine, and cultured for 16 hours in a constant temperature shaker at 32°C and 220 rpm.
2. The culture was inoculated into fresh LB liquid medium at a ratio of 1:100, and cultivation was continued for 2-3 hours until the bacterial density reached an OD₆₀₀ of 0.3, at which point the culture flask was placed in a 42°C water bath and incubated with shaking for 15 minutes, then placed on ice to stand for 1 hour.
3. The bacterial cells were washed 3 times with sterilized pure water.
4. Prepare the PCR product of SWTO102 and SWTO103.

Primers SWTO102 and SWTO103 were mixed with the plasmid pPRO005 and a high-fidelity PCR amplification enzyme system, and the mixture was placed into a PCR amplification instrument.

The amplification program was: 95°C for 2 minutes; 30 cycles of 95°C for 30 seconds, 60°C for 30 seconds, and 72°C for 60 seconds; followed by 72°C for 10 minutes; and 4°C for 5 minutes.

The amplified PCR products were verified by agarose gel electrophoresis. The verification results of the PCR amplification products using SWTO102 and SWTO103 are shown in Figure 17A.

The amplified PCR product was purified and recovered using a DNA gel recovery system, and the concentration and purity of the recovered PCR product DNA were measured using a Nanodrop spectrophotometer.

5. The recovered bacterial cells were mixed with 100 ng of the PCR product of SWTO102 and SWTO103, and electroporation was performed at a voltage of 1.8 kV.

6. The electroporated bacterial cells were spread onto plates containing 25 µg/mL chloramphenicol, 100µg/ml D-alanine and 100 µg/mL DAP (diaminopimelic acid), and cultured overnight in a constant temperature incubator at 32°C until single colonies grew.

7. The single colonies were inoculated into 5 mL of LB medium containing 100µg/ml DAP and 100 µg/mL D-alanine and cultured in a constant temperature shaker at 37°C and 220 rpm for 16 hours to allow the CRE enzyme to function and excise the chloramphenicol resistance gene.

8. Positive clones were identified by colony PCR using primers SWT1O4, SWT1O6, and SWT1O5, SWT1O7.

The PCR amplification products of the target fragment of strains SWT1001-T7P, SWT1005-T7P, SWT2009-T7P,SWT2013-T7P were verified by agarose gel electrophoresis, and the verification results are shown in Figure 17B.

### Example 7: Construction of strains carrying the three-in-one expression vector pPRO010 (SWT5001, SWT5005, SWT6009, SWT6013)

In this example, an antibiotic resistance-free plasmid vector was constructed, as shown in Figure 18. This construction utilized the pUC replicon (SEQ ID No. 36) and the *Salmonella asd* gene (SEQ ID No. 11) as a balanced lethal control mechanism. Furthermore, a dual bacteriophage-derived T7 promoter system was employed to control the expression of the therapeutic protein diphtheria toxin A (DTA) fragment (SEQ ID No. 15) and membrane-disrupting protein gene LLO (SEQ ID No. 13), respectively. The constructed plasmid pPRO010 was then transformed into competent cells of SWT1001-T7P, SWT1005-T7P, SWT2009-T7P, and SWT2013-T7P, thereby generating strains SWT5001, SWT5005, SWT6009, and SWT6013, respectively.

The specific steps are as follows:
1. Plasmid pPRO006 (synthesized by Beijing Liuhe Huada Gene Technology Co., Ltd.) was digested with XhoI and NotI restriction enzymes. An approximately 820 bp fragment containing the pUC replicon was recovered.
2. Plasmid pPRO007 (synthesized by Beijing Liuhe Huada Gene Technology Co., Ltd.) was digested with PstI and XhoI restriction enzymes. An approximately 1270 bp fragment containing the *Salmonella asd* gene was recovered.
3. Plasmid pPRO008 (synthesized by Beijing Liuhe Huada Gene Technology Co., Ltd.) was digested with PstI restriction enzyme. An approximately 660 bp fragment containing the *Salmonella* codon-optimized DTA gene controlled by a T7 promoter was recovered.
4. Plasmid pPRO009 (synthesized by Beijing Liuhe Huada Gene Technology Co., Ltd.) was digested with NotI and PstI restriction enzymes. An approximately 1660 bp fragment containing the *Salmonella* codon-optimized LLO gene controlled by a T7 promoter was recovered.
5. The aforementioned four fragments were ligated using T4 DNA ligase. The ligation product was then transformed into the competent cells of SWT1001-T7P, SWT1005-T7P, SWT2009-T7P, and SWT2013-T7P. The transformed cells were plated onto LB agar plates containing 100 µg/mL D-alanine, and single clones were selected and designated SWT5001, SWT5005, SWT6009, and SWT6013.
6. Single clones were picked and verified by PCR amplification using the following primer pairs: SWTO108 and SWTO109; SWTO110 and SWTO111; SWTO112 and SWTO113; SWTO114 and SWTO115; SWTO116 and SWTO117; SWTO118 and SWTO119; SWTO120 and SWTO121; SWTO122 and SWTO123; SWTO124 and SWTO125. The PCR products were then identified by agarose gel electrophoresis, as shown in Figure 19.

### Example 8: Construction of control strains carrying the three-in-one expression vector pPRO012 (SWT7001, SWT7005, SWT7009, SWT7013)

As control strains, the present invention constructed an expression vector that replaces the T7 promoter. The promoter of the *sseA* gene on *Salmonella* pathogenicity island II (SEQ ID No. 40) was employed.

The *sseA* promoter is a strong promoter that can be induced to express after bacterial invasion into host cells. In this example, serving as a control for the T7 promoter, an antibiotic resistance-free plasmid vector was constructed, as shown in Figure 20. This construction utilized the pUC replicon (SEQ ID No. 36) and the *Salmonella asd* gene (SEQ ID No. 11) as a balanced lethal control mechanism. Furthermore, the *sseA* promoter was employed to separately control the expression of the therapeutic protein diphtheria toxin A (DTA) fragment (SEQ ID No. 15) and the membrane-disrupting protein gene LLO (SEQ ID No. 13). The constructed plasmid pPRO012 was then transformed into SWT1001-T7P, SWT1005-T7P, SWT2009-T7P, and SWT2013-T7P, thereby generating strains SWT7001, SWT7005, SWT7009, and SWT7013, respectively.

The specific steps are as follows:
1. Plasmid pPRO010 was digested with PstI and NotI restriction enzymes. An approximately 2050 bp fragment containing the pUC replicon and the *asd* gene was recovered.
2. Plasmid pPRO008 (synthesized by Beijing Liuhe Huada Gene Technology Co., Ltd.) was digested with NdeI and PstI restriction enzymes. An approximately 590 bp fragment containing the *Salmonella* codon-optimized DTA gene was recovered.
3. Plasmid pPRO009 (synthesized by Beijing Liuhe Huada Gene Technology Co., Ltd.) was digested with NdeI and PstI restriction enzyme. An approximately 1600 bp fragment containing the *Salmonella* codon-optimized LLO gene was recovered.
4. Plasmid pPRO011 (synthesized by Beijing Liuhe Huada Gene Technology Co., Ltd.) was digested with NotI and NdeI restriction enzymes. An approximately 425 bp fragment containing the *sseA* promoter and RBS site sequence was recovered. This fragment served as the promoter for LLO.
5. Plasmid pPRO011 (synthesized by Beijing Liuhe Huada Gene Technology Co., Ltd.) was amplified using primers SWTO126 and SWTO127. An approximately 425 bp fragment containing the *sseA* promoter and RBS site sequence was recovered and subsequently purified via PstI and NdeI digestion. This fragment served as the promoter for DTA.
6. The aforementioned five fragments were ligated using T4 DNA ligase. The ligation product was then transformed into the competent cells of SWT1001-T7P, SWT1005-T7P, SWT2009-T7P, and SWT2013-T7P. The transformed cells were plated onto LB agar plates containing 100 µg/mL D-alanine, and single clones were selected and designated SWT7001, SWT7005, SWT7009, and SWT7013.
7. Single clones were picked and verified by PCR amplification using primer pairs SWTO111 and SWTO128. The PCR products were then identified by agarose gel electrophoresis, as shown in Figure 21.

### Example 9: Verification of T7 RNA Polymerase, DTA, and LLO Protein Expression in Strains Carrying Expression Vectors and Control Vectors

This example was designed to demonstrate the expression stability of proteins regulated by the T7 promoter, both under culture conditions and in the tumor microenvironment.

### (I) Verification of Protein Expression in Expression Vector Strains SWT5001, SWT5005, SWT6009, SWT6013 and Control Vector Strains SWT7001, SWT7005, SWT7009, SWT7013

1. Strains SWT5001, SWT5005, SWT6009, SWT6013, SWT7001, SWT7005, SWT7009, and SWT7013 were separately streaked onto LB agar plates supplemented with 100 µg/mL D-alanine, and incubated statically overnight at 37 °C.
2.Single colonies of each of the strains SWT5001, SWT5005, SWT6009, SWT6013, SWT7001, SWT7005, SWT7009, and SWT7013 were separately inoculated into 5 mL LB liquid medium supplemented with 100 µg/mL D-alanine, and cultured in a thermostatic shaker at 37 °C and 220 rpm for 16 hours.
3. 1 mL of each bacterial culture was taken and centrifuged at 12,000 RPM. The supernatant was discarded, and the pellet was recovered and resuspended in lysis buffer (protein loading buffer, purchased from CST, catalog number 7722S). The samples were then heated at 95 °C for 10 minutes, followed by centrifugation again at 12,000 RPM. The supernatant was recovered and stored for subsequent use.
4. 5 µL of the recovered supernatant from the previous step was loaded onto an SDS-PAGE gel (SDS-PAGE gel preparation kit purchased from Sangon Biotech, catalog number C631100-0200) and subjected to electrophoresis at 120 V for 60 minutes.
5. After electrophoresis, the SDS-PAGE gel was transferred to a PVDF membrane (purchased from Roche, catalog number 03010040001) at a constant current of 250 mA for 30 minutes.
6. The PVDF membrane was then incubated in 5% non-fat dry milk solution on a horizontal shaker at 80 rpm for 1 hour at room temperature for blocking.
7. The blocked PVDF membranes were incubated separately with primary antibodies: Anti-T7 RNA Polymerase antibody (purchased from Creative Biomart, catalog number CABT-B8990), Anti-diphtheria toxin antibody (for DTA identification, purchased from Abcam, catalog number ab151222), and Anti-listeriolysin antibody (for LLO identification, purchased from Abcam, catalog number ab200538). Each primary antibody was diluted in 5% non-fat dry milk and incubated at 4 °C for 18 hours (each membrane was used with one specific antibody).
8. The secondary antibody was diluted in 5% non-fat dry milk. After primary antibody incubation, the PVDF membranes were transferred to the diluted secondary antibody solution and incubated on a horizontal shaker at 80 rpm for 1 hour at room temperature.
9. The PVDF membranes, after secondary antibody incubation, were washed three times with TBST Buffer (purchased from Sangon Biotech, catalog number C520009-0005), 5 minutes per wash. ECL developing reagent (SignalFire^{™} ECL Reagent, purchased from CST, catalog number 6883p3) was then added to the washed PVDF membranes, and incubated in the dark for 1 minute. After incubation, images were acquired using a chemiluminescence imager.

Under LB culture conditions, Western Blot analysis showed that strains SWT5001, SWT5005, SWT6009, SWT6013, SWT7001, SWT7005, SWT7009, and SWT7013 all expressed DTA at high intensity, as shown in Figure 22A. LLO expression is shown in Figure 22B, and T7 RNA polymerase expression is shown in Figure 22C.

### (II) Verification of protein expression by expression vector strains SWT5001, SWT5005, SWT6009, and SWT6013, and control vector strains SWT7001, SWT7005, SWT7009, and SWT7013 in a mouse CT26 tumor model

This example was designed to demonstrate that the T7 expression vector can stably express the protein of interest within the *in vivo* tumor environment.

Tumor tissues were isolated from tumor model mice injected via the tail vein with the corresponding expression vector and control vector strains. Subsequently, verification of target protein expression was performed to evaluate the expression of the target protein by different Salmonella strains in the tumor model.

The specific steps are as follows:

### 1.Establishment of the tumor model

BALB/c mice (purchased from Zhejiang Vital River Laboratory Animal Technology Co., Ltd., weighing approximately 18 g, and housed in an SPF environment) were subcutaneously inoculated with 1×10⁶ CT26 murine colon cancer cells to establish a subcutaneous murine colon cancer model. The experiment was initiated 10-12 days post-inoculation when the tumor volume reached approximately 100 mm³. The mice were divided into 8 groups (2 mice per group): SWT5001, SWT5005, SWT6009, SWT6013, SWT7001, SWT7005, SWT7009, and SWT7013. The tumor-bearing mice were injected via the tail vein with 1×10⁷ CFU of bacteria in a volume of 125 µL.

### 2.Preparation of bacteria

Taking strain SWT1005 as an example, the bacteria were streaked onto an LB plate supplemented with D-alanine and statically cultured overnight in a 37°C incubator. Single colonies of strains SWT5001, SWT5005, SWT6009, SWT6013, SWT7001, SWT7005, SWT7009, and SWT7013 were picked and inoculated into 5 mL of fresh LB liquid medium containing 100 µg/mL D-alanine, followed by cultivation in a constant temperature shaker at 37°C and 220 rpm for 16 hours. The absorbance (OD value) of the strains at 600 nm was measured. An appropriate volume of the culture corresponding to an OD value of 1 was calculated and harvested, washed 3 times with PBS, and resuspended in PBS to prepare a bacterial suspension, which was then administered via the tail vein of the mice.

### 3. Preparation of tumor samples

(1) On day 3 post-administration, the mice were sacrificed, and the tumor tissues were isolated. The tumor tissues were cut into 0.1 g pieces using surgical scissors, homogenized using a homogenizer, and finally resuspended by adding 0.9 mL of fresh PBS solution containing 100 µg/mL D-alanine. The samples were then resuspended in a lysis buffer (protein loading buffer, purchased from CST, Cat. No. 7722S), heated at 95°C for 10 minutes, and centrifuged again at 12,000 rpm. The supernatant was collected for subsequent use.
(2) A 5µL aliquot of the supernatant recovered in the previous step was aspirated and loaded onto an SDS-PAGE gel (SDS-PAGE gel preparation kit purchased from Sangon Biotech, Cat. No. C631100-0200), followed by electrophoresis at 120 V for 60 minutes.
(3) Following electrophoresis, the SDS-PAGE gel was transferred onto a PVDF membrane (purchased from Roche, Cat. No. 03010040001) under a constant current of 250 mA for 30 minutes.
(4) For blocking, the transferred PVDF membrane was placed in 5% non-fat dry milk and incubated at room temperature on a horizontal shaker at 80 rpm for 1 hour.
(5) The blocked PVDF membranes were respectively incubated with primary antibodies diluted in 5% non-fat dry milk at 4°C for 18 hours (each membrane was incubated with a single antibody separately). The primary antibodies used were: Anti-T7 RNA Polymerase antibody (purchased from Creative Biomart, Cat. No. CABT-B8990), Anti-diphtheria toxin antibody (identification antibody for DTA, purchased from Abcam, Cat. No. ab151222), and Anti-listeriolysin antibody (identification antibody for LLO, purchased from Abcam, Cat. No. ab200538).
(6) The secondary antibody was diluted in 5% non-fat dry milk. Following primary antibody incubation, the PVDF membranes were transferred into the secondary antibody dilution and incubated at room temperature on a horizontal shaker at 80 rpm for 1 hour.
(7) Following the secondary antibody incubation, the PVDF membranes were washed three times with TBST Buffer (purchased from Sangon Biotech, Cat. No. C520009-0005) for 5 minutes each. ECL developer (SignalFire^{™} ECL Reagent, purchased from CST, Cat. No. 6883p3) was added dropwise to the washed PVDF membranes, followed by incubation in the dark for 1 minute. After incubation, images of the PVDF membranes were acquired using a chemiluminescence imaging system.

In the isolated tumor tissues, Western Blot analysis demonstrated that strains SWT5001, SWT5005, SWT6009, and SWT6013 all expressed DTA at high intensity (see Figure 23A). For LLO expression, see Figure 23B; for T7 RNA polymerase expression, see Figure 23C. However, in the control strains SWT7001, SWT7005, SWT7009, and SWT7013, only the expression of T7 RNA polymerase was detected (see Figure 23C), whereas almost no stable expression of DTA and LLO was observed.

### Example 10: Immunohistochemical Analysis of the Tumor Region Following Injection of Strains Carrying Expression Vectors

To verify that the *Salmonella* carrying the expression vector accumulates in the hypoxic regions of the tumor and expresses the target protein, this example selected SWT5005 as a representative strain. The mouse tumor model, following tail vein injection of the *Salmonella*, was subjected to sectioning and staining to observe the distribution of the therapeutic protein within the tumor model.

The specific procedures were as follows:

### 1. Establishment of the Tumor Model

BALB/c mice (purchased from Zhejiang Vital River Laboratory Animal Technology Co., Ltd., body weight approximately 18 g, housed in an SPF environment) were subcutaneously inoculated with 1×10⁶ CT26 murine colon carcinoma cells to establish a subcutaneous murine colon carcinoma model. The experiment was initiated 10-12 days post-inoculation when the tumor volume reached approximately 100 mm³. The mice were divided into a PBS group and an SWT5005 group, with 5 mice per group. The tumor-bearing mice were inoculated via tail vein injection with 1×10⁷ CFU of bacteria in a volume of 125 µL.

### 2. Bacteria Preparation

Taking strain SWT5005 as an example, the bacteria were streaked onto an LB agar plate supplemented with 100 µg/mL D-alanine and incubated statically overnight in a 37°C constant-temperature incubator. A single colony of strain SWT5005 was picked and inoculated into 5 mL of fresh LB liquid medium containing 100 µg/mL D-alanine, followed by cultivation in a constant-temperature shaker at 37°C and 220 rpm for 16 hours. The absorbance (OD value) of the strain at 600 nm was measured. A culture volume corresponding to an OD value of 1 was calculated and aliquoted, washed three times with PBS, and resuspended in PBS to prepare a bacterial suspension for administration via the mouse tail vein.

### 3. Tumor Sample Sectioning Analysis

On day 5 post-administration, the mice were sacrificed. Tumor tissues were isolated and immersed in formalin fixative. The tissues were subjected to gradient dehydration, embedded in paraffin, and after complete cooling and solidification, sectioned and deparaffinized. The sections were immersed in a sodium citrate antigen retrieval solution and boiled for 10 minutes for antigen retrieval, followed by three washes with PBS. The antigen-retrieved sections were then immersed in a 3% hydrogen peroxide solution for 10 minutes, followed by three washes with PBS. After quenching endogenous peroxidase, the sections were immersed in a 2% BSA-PBS solution and blocked at room temperature for 1 hour, followed by three washes with PBS. Primary antibody incubation solutions, including anti-Salmonella (Salmonella identification marker, purchased from Abcam, Cat. No. ab35156), anti-HIF1α (hypoxic region identification marker, purchased from Abcam, Cat. No. ab51608), and anti-diphtheria toxin (DTA identification antibody, purchased from Abcam, Cat. No. ab151222), were added dropwise onto different blocked tissue sections, respectively. These were incubated overnight at 4°C, followed by three washes with PBS. A secondary antibody incubation solution was added dropwise onto the tissue sections, incubated for 25 minutes, and washed three times with PBS. DAB staining solution was added dropwise onto the tissue sections, followed by counterstaining with hematoxylin for 3 minutes. Finally, the tissue sections were sequentially subjected to gradient dehydration, mounted with a mounting medium, observed under a microscope, and photographed, as shown in Figure 24.

The results indicated that, based on antibody staining comparisons, the brown (dark) areas represented positive antibody staining. It was observed that the *Salmonella* harboring the hypoxia-specific gene expression cassette accumulated within the hypoxic regions of the tumor, as indicated by the arrows in Figures 24A and 24B. Furthermore, the expressed therapeutic protein DTA was found to be widely distributed throughout the tumor, as indicated by the arrows in Figure 24C.

### Example 11: Verification of the Cytotoxic Effect of Strains Carrying Expression Vectors and Control Vectors on Cancer Cells Under Hypoxic Conditions

By co-culturing with cancer cells under in vitro hypoxic conditions, the cytotoxic effects of strains SWT5001, SWT5005, SWT6009, SWT6013, SWT7001, SWT7005, SWT7009, and SWT7013 on various cancer cells were verified.

The specific procedures were as follows:
1. Strains SWT5001, SWT5005, SWT6009, SWT6013, SWT7001, SWT7005, SWT7009, and SWT7013 were streaked onto LB agar plates supplemented with 100 µg/mL D-alanine and incubated statically overnight in a 37°C constant-temperature incubator.
2. Single colonies of strains SWT5001, SWT5005, SWT6009, SWT6013, SWT7001, SWT7005, SWT7009, and SWT7013 were picked and inoculated into 5 mL of fresh LB liquid medium containing 100 µg/mL D-alanine, followed by cultivation in a constant-temperature shaker at 37°C and 220 rpm for 16 hours.
3. The absorbance of the strains at 600 nm was measured. A culture volume corresponding to an OD value of 1 was aliquoted, washed twice with PBS, and then washed once with the corresponding cell culture medium. Subsequently, the bacterial pellet was resuspended in the cell culture medium (at a multiplicity of infection (MOI) of 200:1).
4. The above bacteria were respectively co-cultured with breast cancer (EMT6 cell line), osteosarcoma (K7M2 cell line), liver cancer (Hepa1-6 cell line), lung cancer (A549 cell line), melanoma (B16F10 cell line), renal cancer (Renca cell line), gastric cancer (MFC cell line), pancreatic cancer (Pan02 cell line), prostate cancer (RM-1 cell line), colon cancer (CT26 cell line), ovarian cancer (ID8 cell line), neuroblastoma (Neuro-2a cell line), squamous cell carcinoma (SCC7 cell line), and bladder cancer (MB49 cell line) under a hypoxic condition of less than 0.8% oxygen concentration for 2 hours. Subsequently, the cells were washed three times with PBS containing gentamicin. After medium replacement, the cells were further cultured in an anaerobic jar under a hypoxic condition of less than 0.8% oxygen concentration for 24 hours. Finally, the cells were photographed and recorded under a bright-field microscope using a 20x objective lens, as shown in Figures 25-29.
5. The CCK-8 reagent was mixed with the above cell suspensions and incubated for 1 hour.
6. The absorbance was measured using a microplate reader (at a detection wavelength of 450 nm), and the percentage of cytotoxicity was calculated.

The cytotoxic effects of strains SWT5001, SWT5005, SWT6009, SWT6013, SWT7001, SWT7005, SWT7009, and SWT7013 on EMT6, K7M2, Hepa1-6, A549, B16F10, Renca, MFC, Pan02, RM-1, CT26, ID8, Neuro-2a, SCC7, and MB49 cells are shown in Figures 30 and 31.

The experimental results were analyzed using the Student's t-test, where p<0.05 (*), p<0.01 (**), and p≤0.001 (***) indicate significant differences.

The above results demonstrate that the Salmonella strains SWT5001, SWT5005, SWT6009, and SWT6013 carrying the expression vectors all exhibited significant cytotoxic effects on various cancer cells. Significant cell death was observed under a bright-field microscope, and analysis using the CCK-8 cytotoxicity assay revealed that their cell-killing effects were stronger than those of the control strains SWT7001, SWT7005, SWT7009, and SWT7013. This indicates that the Salmonella carrying the three-in-one drug expression vector with the T7 expression system possesses a universal cytotoxic effect against cancer cells.

### Example 12: Evaluation of Tumor Suppression by Strains Carrying the Expression Vector and the Control Vector in Mouse Tumor Models

To verify the inhibitory effect of the strains carrying the expression vector on tumor growth in mice, the present invention designed an inhibition assay using SWT5005 and SWT7005 as representatives in mouse EMT6, K7M2, Hepa1-6, A549, B16F10, Renca, MFC, Pan02, RM-1, CT26, ID8, Neuro-2a, SCC7, and MB49 tumor models.

### 1. Establishment of Various Mouse Tumor Models

### (1) Establishment of the EMT6 Mouse Tumor Model

BALB/c mice (purchased from Zhejiang Vital River Laboratory Animal Technology Co., Ltd., weighing approximately 18 g, housed in a Specific Pathogen-Free (SPF) environment) were subcutaneously inoculated with 1×10⁶ EMT6 cancer cells to establish a mouse subcutaneous breast tumor model. The experiment was initiated 14-18 days post-inoculation when the tumor volume reached approximately 100 mm³. The mice were divided into three groups: a PBS control group, an SWT5005 group, and an SWT7005 group, with 5 mice per group. The tumor-bearing mice were intravenously injected via the tail vein with
1× 10⁷CFU of bacteria in a volume of 125 µL.

### (2) Establishment of the K7M2 Mouse Tumor Model

BALB/c mice (purchased from Zhejiang Vital River Laboratory Animal Technology Co., Ltd., weighing approximately 18 g, housed in a Specific Pathogen-Free (SPF) environment) were subcutaneously inoculated with 1×10⁶ K7M2 cancer cells to establish a mouse subcutaneous tumor model. The experiment was initiated 14-18 days post-inoculation when the tumor volume reached approximately 100 mm³. The mice were divided into three groups: a PBS control group, an SWT5005 group, and an SWT7005 group, with 5 mice per group. The tumor-bearing mice were intravenously injected via the tail vein with 1×10⁷ CFU of bacteria in a volume of 125 µL.

### (3) Establishment of the Hepa1-6 Mouse Tumor Model

C57BL/6 mice (purchased from Zhejiang Vital River Laboratory Animal Technology Co., Ltd., weighing approximately 18 g, housed in a Specific Pathogen-Free (SPF) environment) were subcutaneously inoculated with 1×10⁶ Hepa1-6 cancer cells to establish a mouse subcutaneous tumor model. The experiment was initiated 14-18 days post-inoculation when the tumor volume reached approximately 100 mm³. The mice were divided into three groups: a PBS control group, an SWT5005 group, and an SWT7005 group, with 5 mice per group. The tumor-bearing mice were intravenously injected via the tail vein with 1×10⁷ CFU of bacteria in a volume of 125 µL.

### (4) Establishment of the A549 Mouse Tumor Model

Nu/Nu nude mice (purchased from Zhejiang Vital River Laboratory Animal Technology Co., Ltd., weighing approximately 18 g, housed in a Specific Pathogen-Free (SPF) environment) were subcutaneously inoculated with 1×10⁶ A549 cancer cells to establish a mouse subcutaneous tumor model. The experiment was initiated 14-18 days post-inoculation when the tumor volume reached approximately 100 mm³. The mice were divided into three groups: a PBS control group, an SWT5005 group, and an SWT7005 group, with 5 mice per group. The tumor-bearing mice were intravenously injected via the tail vein with 1×10⁷ CFU of bacteria in a volume of 125 µL.

### (5) Establishment of the B16F10 Mouse Tumor Model

C57BL/6 mice (purchased from Zhejiang Vital River Laboratory Animal Technology Co., Ltd., weighing approximately 18 g, housed in a Specific Pathogen-Free (SPF) environment) were subcutaneously inoculated with 1×10⁶ B16F10 cancer cells to establish a mouse subcutaneous tumor model. The experiment was initiated 14-18 days post-inoculation when the tumor volume reached approximately 100 mm³. The mice were divided into three groups: a PBS control group, an SWT5005 group, and an SWT7005 group, with 5 mice per group. The tumor-bearing mice were intravenously injected via the tail vein with 1×10⁷ CFU of bacteria in a volume of 125 µL.

### (6) Establishment of the Renca Mouse Tumor Model

BALB/c mice (purchased from Zhejiang Vital River Laboratory Animal Technology Co., Ltd., weighing approximately 18 g, housed in a Specific Pathogen-Free (SPF) environment) were subcutaneously inoculated with 1×10⁶ Renca cancer cells to establish a mouse subcutaneous tumor model. The experiment was initiated 14-18 days post-inoculation when the tumor volume reached approximately 100 mm³. The mice were divided into three groups: a PBS control group, an SWT5005 group, and an SWT7005 group, with 5 mice per group. The tumor-bearing mice were intravenously injected via the tail vein with 1×10⁷ CFU of bacteria in a volume of 125 µL.

### (7) Establishment of the MFC Mouse Tumor Model

BALB/c mice (purchased from Zhejiang Vital River Laboratory Animal Technology Co., Ltd., weighing approximately 18 g, housed in a Specific Pathogen-Free (SPF) environment) were subcutaneously inoculated with 1×10⁶ MFC cancer cells to establish a mouse subcutaneous tumor model. The experiment was initiated 14-18 days post-inoculation when the tumor volume reached approximately 100 mm³. The mice were divided into three groups: a PBS control group, an SWT5005 group, and an SWT7005 group, with 5 mice per group. The tumor-bearing mice were intravenously injected via the tail vein with
1×10⁷ CFU of bacteria in a volume of 125 µL.

### (8) Establishment of the Pan02 Mouse Tumor Model

C57BL/6 mice (purchased from Zhejiang Vital River Laboratory Animal Technology Co., Ltd., weighing approximately 18 g, housed in a Specific Pathogen-Free (SPF) environment) were subcutaneously inoculated with 1×10⁶ Pan02 cancer cells to establish a mouse subcutaneous tumor model. The experiment was initiated 14-18 days post-inoculation when the tumor volume reached approximately 100 mm³. The mice were divided into three groups: a PBS control group, an SWT5005 group, and an SWT7005 group, with 5 mice per group. The tumor-bearing mice were intravenously injected via the tail vein with 1×10⁷ CFU of bacteria in a volume of 125 µL.

### (9) Establishment of the RM-1 Mouse Tumor Model

C57BL/6 mice (purchased from Zhejiang Vital River Laboratory Animal Technology Co., Ltd., weighing approximately 18 g, housed in a Specific Pathogen-Free (SPF) environment) were subcutaneously inoculated with 1×10⁶ RM-1 cancer cells to establish a mouse subcutaneous tumor model. The experiment was initiated 14-18 days post-inoculation when the tumor volume reached approximately 100 mm³. The mice were divided into three groups: a PBS control group, an SWT5005 group, and an SWT7005 group, with 5 mice per group. The tumor-bearing mice were intravenously injected via the tail vein with 1×10⁷ CFU of bacteria in a volume of 125 µL.

### (10) Establishment of the CT26 Mouse Tumor Model

BALB/c mice (purchased from Zhejiang Vital River Laboratory Animal Technology Co., Ltd., weighing approximately 18 g, housed in a Specific Pathogen-Free (SPF) environment) were subcutaneously inoculated with 1×10⁶ CT26 cancer cells to establish a mouse subcutaneous tumor model. The experiment was initiated 14-18 days post-inoculation when the tumor volume reached approximately 100 mm³. The mice were divided into three groups: a PBS control group, an SWT5005 group, and an SWT7005 group, with 5 mice per group. The tumor-bearing mice were intravenously injected via the tail vein with 1×10⁷ CFU of bacteria in a volume of 125 µL.

### (11) Establishment of the ID8 Mouse Tumor Model

C57BL/6 mice (purchased from Zhejiang Vital River Laboratory Animal Technology Co., Ltd., weighing approximately 18 g, housed in a Specific Pathogen-Free (SPF) environment) were subcutaneously inoculated with 1×10⁶ ID8 cancer cells to establish a mouse subcutaneous tumor model. The experiment was initiated 14-18 days post-inoculation when the tumor volume reached approximately 100 mm³. The mice were divided into three groups: a PBS control group, an SWT5005 group, and an SWT7005 group, with 5 mice per group. The tumor-bearing mice were intravenously injected via the tail vein with 1×10⁷ CFU of bacteria in a volume of 125 µL.

### (12) Establishment of the Neuro-2a Mouse Tumor Model

A/J mice (purchased from Zhejiang Vital River Laboratory Animal Technology Co., Ltd., weighing approximately 18 g, housed in a Specific Pathogen-Free (SPF) environment) were subcutaneously inoculated with 1×10⁶ Neuro-2a cancer cells to establish a mouse subcutaneous tumor model. The experiment was initiated 14-18 days post-inoculation when the tumor volume reached approximately 100 mm³. The mice were divided into three groups: a PBS control group, an SWT5005 group, and an SWT7005 group, with 5 mice per group. The tumor-bearing mice were intravenously injected via the tail vein with 1×10⁷ CFU of bacteria in a volume of 125 µL.

### (13) Establishment of the SCC7 Mouse Tumor Model

Nu/Nu nude mice (purchased from Zhejiang Vital River Laboratory Animal Technology Co., Ltd., weighing approximately 18 g, housed in a Specific Pathogen-Free (SPF) environment) were subcutaneously inoculated with 1×10⁶ SCC7 cancer cells to establish a mouse subcutaneous tumor model. The experiment was initiated 14-18 days post-inoculation when the tumor volume reached approximately 100 mm³. The mice were divided into three groups: a PBS control group, an SWT5005 group, and an SWT7005 group, with 5 mice per group. The tumor-bearing mice were intravenously injected via the tail vein with 1×10⁷ CFU of bacteria in a volume of 125 µL.

### (14) Establishment of the MB49 Mouse Tumor Model

C57BL/6 mice (purchased from Zhejiang Vital River Laboratory Animal Technology Co., Ltd., weighing approximately 18 g, housed in a Specific Pathogen-Free (SPF) environment) were subcutaneously inoculated with 1×10⁶ MB49 cancer cells to establish a mouse subcutaneous tumor model. The experiment was initiated 14-18 days post-inoculation when the tumor volume reached approximately 100 mm³. The mice were divided into three groups: a PBS control group, an SWT5005 group, and an SWT7005 group, with 5 mice per group. The tumor-bearing mice were intravenously injected via the tail vein with 1×10⁷ CFU of bacteria in a volume of 125 µL.

### 2. Preparation of Bacteria and Treatment Protocol

Single colonies of the constructed Salmonella strains SWT5005 and SWT7005 were picked and inoculated into 5 mL of LB liquid medium supplemented with D-alanine. The cultures were incubated in a constant temperature shaker at 37°C and 220 rpm for 16 hours. The absorbance of the bacterial strains was measured at 600 nm (OD600). A volume of culture corresponding to an OD 600 of 1 was taken and washed three times with PBS. Model mice were administered 1×10⁷ CFU of bacteria in a volume of 125 µL via tail vein injection. The day of administration was designated as Day 0. The length and width of the tumors in the mice were measured on Days 1, 3, 5, 7, 9, and 11 post-administration, respectively, and the tumor volume was$Mouse Tumor Volume = \left(Length \times {Width}^{2}\right) \times 0.52$calculated according to the following formula:

Results of Tumor Inhibition in Mouse Models The tumor suppressive effects in the mouse tumor models comparing SWT5005 and SWT7005 are illustrated in Figures 32-35.

The experimental data were analyzed using the Student's t-test, where p<0.05 (*), p<0.01 (**), and p≤0.001 (***) indicate statistically significant differences.

The results demonstrate that the *Salmonella* strain SWT5005 carrying the expression vector exhibits a significant tumor growth inhibitory effect, which is markedly superior to both the control vector strain SWT7005 and the untreated control.

Information concerning the strains of the present invention is shown in Table 2.

**Table 2. Construction of the Strains of the Present Invention**

| Strain Name | Characteristic Description | Source |
|---|---|---|
| SWT001 | Wild-type *Salmonella* | Purchased from CICC |
| SWT002 | Wild-type *Salmonella* SWT001 transformed with temperature-inducible lambda-RED recombinase and loxP-Cre enzyme system (plasmid pSWT001) | Constructed |
| SWT003 | Derived from wild-type SWT001, *aroA* gene knockout | Constructed |
| SWT004 | Derived from wild-type SWT001, *aroA* and *alr* gene knockouts | Constructed |
| SWT007 | Derived from wild-type SWT001, *aroA* and *dadX* gene knockouts | Constructed |
| SWT1001 | Derived from wild-type SWT001, *aroA*, *alr*, and *dadX* gene knockouts; oxygen regulation system *yhbU-S-alr-cyoA-S* (from plasmid pOL1001) integrated into the *dadX* locus (*ΔdadX::yhbU-S-alr-cyoA-S*) | Constructed |
| SWT1005 | Derived from wild-type SWT001, *aroA*, *alr*, and *dadX* gene knockouts; oxygen regulation system *yhbU-S-alr-ydcI-S* (from plasmid pOL1005) integrated into the *dadX* locus (*ΔdadX::yhbU-S-alr-ydcI-S*) | Constructed |
| SWT2009 | Derived from wild-type SWT001, *aroA*, *alr*, and *dadX* gene knockouts; oxygen regulation system *ynfK-E-dadX-cyoA-S* (from plasmid pOL2009) integrated into the *alr* locus (*Δalr::yhbU-S-dadX-cyoA-S*) | Constructed |
| SWT2013 | Derived from wild-type SWT001, *aroA*, *alr*, and *dadX* ge*n*e knockouts; oxygen regulation system *ynfK-E-dadX-ydcI-S* (from plasmid pOL2013) integrated into the *alr* locus (*Δalr::ynfK-E-dadX-ydcI-S*) | Constructed |
| Strain Name | Characteristic Description | Source |
| SWT1001-T7P | Derived from SWT1001, *asd* gene kno*c*kout, T7 RNA polymerase integrated into the *asd* locus (*Δasd::lacUV5-T7P*) | Constructed |
| SWT1005-T7P | Derived from SWT1005, asd gene knockout, T7 RNA polymerase integrated into the asd locus (Δ*asd*::*lacUV5-T7P*) | Constructed |
| SWT2009-T7P | Derived from SWT2009, *asd* gene kno*c*kout, T7 RNA polymerase integrated into the *asd* locus (Δ*asd:*:*lacUV5-T7P*) | Constructed |
| SWT2013-T7P | Derived from SWT2013, *asd* gene knockout, T7 RNA polymerase integrated into the asd locus (Δ*asd*::*lacUV5-T7P*) | Constructed |
| SWT5001 | Derived from SWT1001-T7P, *asd* gene knockout, transformed with expression vector plasmid pPRO010 | Constructed |
| SWT5005 | Derived from SWT1005-T7P, *asd* gene knockout, transformed with expression vector plasmid pPRO010 | Constructed |
| SWT6009 | Derived from SWT2009-T7P, *asd* gene knockout, transformed with expression vector plasmid pPRO010 | Constructed |
| SWT6013 | Derived from SWT2013-T7P, *asd* gene knockout, transformed with expression vector plasmid pPRO010 | Constructed |
| SWT7001 | Derived from SWT1001-T7P, *asd* gene knockout, transformed with control expression vector plasmid pPRO012 | Constructed |
| SWT7005 | Derived from SWT1005-T7P, *asd* gene knockout, transformed with control expression vector plasmid pPRO012 | Constructed |
| SWT7009 | Derived from SWT2009-T7P, *asd* gene knockout, transformed with control expression vector plasmid pPRO012 | Constructed |
| SWT7013 | Derived from SWT2013-T7P, *asd* gene knockout, transformed with control expression vector plasmid pPRO012 | Constructed |

Information concerning the tool plasmids in the present invention is shown in Table 3.

**Table 3: Information of the tool plasmids adopted in the present invention**

| Name | Description | Source |
|---|---|---|
| pSWT001 | Temperature-inducible recombinase and Cre recombinase system | Constructed |
| pSWT002 | A vector comprising bilateral co-directional *loxP* sequences and a chloramphenicol resistance gene in the middle, flanked by NotI | Constructed |
| | restriction sites on both sides, used for gene knockout following amplification | |
| pSWT003 | pBluescript - cloning backbone vector | Purchased from Biofeng |
| pSWT007 | A vector comprising bilateral co-directional *loxP* sequences and a chloramphenicol resistance gene in the middle, flanked by SpeI and NotI restriction sites on both sides, used for constructing a hypoxia-specific gene expression cassette library | Constructed |
| pPRO004 | A vector comprising a codon-optimized T7 RNA polymerase gene | Constructed |
| pPRO005 | A vector comprising a T7 RNA polymerase controlled by a *lacUV5* promoter | Constructed |
| pPRO006 | A vector comprising a pUC replicon | Constructed |
| pPRO007 | A vector comprising an *asd* balanced-lethal control gene | Constructed |
| pPRO008 | A vector comprising DTA | Constructed |
| pPRO009 | A vector comprising codon-optimized LLO | Constructed |
| pPRO010 | An expression vector comprising a pUC replicon, an asd balanced-lethal control gene, and a T7 promoter controlling DTA and LLO | Constructed |
| pPRO011 | A vector comprising an *sseA* promoter and an RBS | Constructed |
| pPRO012 | An expression vector comprising a pUC replicon, an *asd* balanced-lethal control gene, and a *sseA* promoter controlling DTA and LLO | Constructed |
| pOL1001 | A plasmid comprising a *cm* resistance gene sequence with *loxP* sequences and a *yhbU-S-alr-cyoA-S* regulatory element | Constructed |
| pOL1005 | A plasmid comprising a *cm* resistance gene sequence with *loxP* sequences and a *yhbU-S-alr-ydcI-S* regulatory element | Constructed |
| pOL2009 | A plasmid comprising a *cm* resist*anc*e gene sequence with *loxP* sequences and a *ynfK-E-dadX-cyoA-S* regulatory element | Constructed |
| pOL2013 | A plasmid comprising a *cm* resistance gene sequence with *loxP* sequences and a *ynfK-E-dadX-ydcI-S* regulatory element | Constructed |

The sequences of the promoters, gene coding regions, proteins, and the like adopted in the present invention are shown in Table 4.

**Table 4: Specific sequences of promoters, gene coding regions, proteins, and the like adopted in the present invention**

| **SEQ ID NO:** | **Description** | **Sequence** |
|---|---|---|
| SEQ ID No.1 | T7 promoter | TAATACGACTCACTATAG |
| SEQ ID No.2 | T3 promoter | AATTAACCCTCACTAAAG |
| SEQ ID No.3 | SP6 promoter | ATTTAGGTGACACTATAG |
| SEQ ID No.4 | T7 RNA polymerase gene coding region | ATGAACACCATCAACATTGCCAAAAACGATTTTAGCGATATTGAACTGGCAG CGATTCCGTTTAATACCCTGGCAGATCATTATGGTGAACGTCTGGCACGTGA ACAGCTGGCACTGGAACATGAAAGCTATGAAATGGGTGAAGCACGTTTTCG CAAAATGTTTGAACGTCAGCTGAAAGCCGGTGAAGTTGCAGATAATGCAGC AGCAAAACCGCTGATTACCACACTGCTGCCGAAAATGATTGCACGTATTAAC GATTGGTTCGAAGAGGTTAAAGCCAAACGTGGTAAACGTCCGACCGCATTTC AGTTTCTGCAAGAAATTAAACCGGAAGCCGTTGCCTATATTACCATTAAAAC CACACTGGCATGTCTGACCAGCGCAGATAATACCACCGTTCAGGCAGTTGCA AGCGCAATTGGTCGTGCAATTGAAGATGAAGCCCGTTTTGGTCGTATTCGTG ATCTGGAAGCAAAACACTTTAAGAAAAACGTGGAAGAACAGCTGAATAAAC GTGTTGGTCACGTTTATAAGAAAGCCTTTATGCAGGTTGTTGAAGCAGATAT GCTGAGCAAAGGTCTGTTAGGTGGTGAAGCATGGTCAAGCTGGCATAAAGA AGATAGCATTCACGTTGGTGTTCGCTGTATTGAAATGCTGATTGAAAGCACC GGTATGGTTAGCCTGCATCGTCAGAATGCCGGTGTTGTTGGTCAGGATAGCG AAACCATCGAACTGGCACCGGAATATGCCGAAGCAATTGCAACCCGTGCCG GTGCACTGGCAGGTATTAGCCCGATGTTTCAGCCGTGTGTTGTTCCGCCTAA ACCGTGGACCGGCATTACCGGTGGTGGTTATTGGGCAAATGGTCGTCGTCCG CTGGCGCTGGTTCGTACCCATAGCAAAAAGGCACTGATGCGTTATGAAGATG TTTATATGCCGGAAGTGTACAAAGCCATTAACATTGCACAGAATACCGCCTG GAAAATCAATAAAAAGGTTCTGGCCGTTGCCAACGTTATCACCAAATGGAA ACATTGTCCGGTTGAAGATATTCCGGCAATTGAACGTGAAGAACTGCCGATG AAGCCGGAAGATATTGATATGAATCCGGAAGCACTGACCGCATGGAAACGT GCAGCAGCCGCAGTTTATCGTAAAGATAAAGCACGTAAAAGCCGTCGTATTA GCCTGGAATTTATGCTGGAACAGGCAAACAAATTTGCCAACCATAAAGCAAT CTGGTTCCCGTATAATATGGATTGGCGTGGTCGTGTTTATGCCGTTAGCATGT TTAATCCGCAGGGTAATGATATGACCAAAGGCCTGCTGACCCTGGCAAAAG GTAAACCGATTGGTAAAGAAGGTTACTATTGGCTGAAAATTCATGGTGCAAA TTGTGCCGGTGTGGATAAAGTTCCGTTTCCGGAACGCATTAAATTCATCGAA GAAAACCACGAAAACATTATGGCCTGTGCAAAAAGTCCGCTGGAAAATACC TGGTGGGCAGAACAGGATAGTCCGTTTTGTTTTCTGGCCTTTTGTTTTGAATA TGCGGGTGTTCAGCATCATGGTCTGAGCTATAATTGTAGCCTGCCGCTGGCA TTTGATGGTAGCTGTAGCGGTATTCAGCATTTTAGCGCAATGCTGCGTGATG AAGTTGGTGGTCGTGCCGTTAATCTGCTGCCGTCAGAAACCGTGCAGGATAT TTATGGTATTGTTGCCAAGAAAGTGAACGAAATTCTGCAAGCAGATGCAATT AATGGCACCGATAATGAAGTTGTTACCGTGACCGATGAAAACACCGGTGAA ATTAGCGAAAAAGTTAAACTGGGCACCAAAGCGCTGGCAGGTCAGTGGCTG GCCTATGGTGTTACCCGTAGCGTTACCAAACGTAGCGTGATGACACTGGCGT ATGGTAGCAAAGAATTTGGTTTTCGTCAGCAGGTTCTGGAAGATACAATTCA GCCTGCAATTGATAGCGGTAAAGGTCTGATGTTTACCCAGCCGAATCAGGCA GCAGGTTATATGGCAAAACTGATTTGGGAAAGCGTTAGCGTGACCGTTGTTG CAGCAGTTGAAGCAATGAACTGGCTGAAATCAGCAGCCAAACTGCTGGCAG CAGAAGTTAAAGATAAGAAAACGGGTGAAATCCTGCGTAAACGTTGTGCCG TTCATTGGGTTACACCGGATGGTTTTCCGGTTTGGCAAGAATATAAGAAACC GATTCAGACACGCCTGAACCTGATGTTTCTGGGTCAGTTTCGTCTGCAACCG ACCATTAATACCAATAAAGATTCCGAAATCGACGCCCACAAACAAGAAAGC GGTATTGCACCGAATTTTGTTCATAGCCAGGATGGTAGCCATCTGCGTAAAA CCGTTGTTTGGGCACATGAAAAATATGGCATTGAATCCTTTGCGCTGATCCA TGATAGCTTTGGCACCATTCCGGCAGATGCAGCAAACCTGTTTAAAGCAGTT CGTGAAACAATGGTGGATACCTATGAAAGCTGTGATGTTCTGGCGGATTTTT ATGATCAGTTTGCAGATCAGCTGCATGAAAGCCAGCTGGATAAAATGCCTGC ACTGCCTGCCAAAGGTAATCTGAATCTGCGTGATATTCTGGAAAGCGATTTT GCCTTTGCCTAA |
| SEQ ID No.5 | T7 RNA polymerase protein sequence | MNTINIAKNDFSDIELAAIPFNTLADHYGERLAREQLALEHESYEMGEARFRKMF ERQLKAGEVADNAAAKPLITTLLPKMIARINDWFEEVKAKRGKRPTAFQFLQEI KPEAVAYITIKTTLACLTSADNTTVQAVASAIGRAIEDEARFGRIRDLEAKHFKK NVEEQLNKRVGHVYKKAFMQVVEADMLSKGLLGGEAWSSWHKEDSIHVGVR CIEMLIESTGMVSLHRQNAGVVGQDSETIELAPEYAEAIATRAGALAGISPMFQP CVVPPKPWTGITGGGYWANGRRPLALVRTHSKKALMRYEDVYMPEVYKAINIA QNTAWKINKKVLAVANVITKWKHCPVEDIPAIEREELPMKPEDIDMNPEALTA WKRAAAAVYRKDKARKSRRISLEFMLEQANKFANHKAIWFPYNMDWRGRVY AVSMFNPQGNDMTKGLLTLAKGKPIGKEGYYWLKIHGANCAGVDKVPFPERIK FIEENHENIMACAKSPLENTWWAEQDSPFCFLAFCFEYAGVQHHGLSYNCSLPL AFDGSCSGIQHFSAMLRDEVGGRAVNLLPSETVQDIYGIVAKKVNEILQADAING TDNEVVTVTDENTGEISEKVKLGTKALAGQWLAYGVTRSVTKRSVMTLAYGSK EFGFRQQVLEDTIQPAIDSGKGLMFTQPNQAAGYMAKLIWESVSVTVVAAVEA MNWLKSAAKLLAAEVKDKKTGEILRKRCAVHWVTPDGFPVWQEYKKPIQTRL NLMFLGQFRLQPTINTNKDSEIDAHKQESGIAPNFVHSQDGSHLRKTVVWAHEK YGIESFALIHDSFGTIPADAANLFKAVRETMVDTYESCDVLADFYDQFADQLHES QLDKMPALPAKGNLNLRDILESDFAFA |
| SEQ ID No.6 | T3 RNA polymerase gene coding region | ATGAACATCATCGAAAACATCGAAAAGAATGACTTCTCAGAAATCGAACTG GCTGCTATCCCGTTCAACACACTGGCTGACCACTACGGAAGCGCCTTGGCTA AAGAGCAGTTGGCTTTAGAACATGAGTCTTATGAGCTAGGCGAGCGCCGCTT CCTCAAGATGCTTGAGCGTCAAGCGAAAGCTGGTGAGATTGCAGACAACGC AGCCGCTAAGCCGTTACTCGCTACGCTTCTCCCTAAGTTAACCACACGTATC GTCGAGTGGCTCGAAGAGTACGCATCGAAGAAAGGCCGCAAGCCTAGCGCA TACGCACCGCTCCAGTTACTCAAGCCGGAGGCCTCCGCGTTTATCACCCTGA AAGTTATCCTTGCGTCACTAACCAGTACGAACATGACAACCATTCAGGCCGC TGCTGGTATGCTGGGGAAAGCCATTGAGGACGAGGCACGATTTGGGCGCAT CCGTGACCTAGAAGCGAAGCACTTCAAGAAGCACGTTGAGGAACAGCTTAA CAAGCGCCACGGGCAAGTCTACAAGAAAGCATTTATGCAGGTGGTCGAGGC CGATATGATTGGTCGAGGTCTGCTTGGTGGCGAGGCGTGGTCTAGCTGGGAT AAAGAAACCACGATGCACGTAGGGATTCGCCTGATTGAAATGCTGATTGAAT CCACGGGTCTGGTGGAATTACAGCGCCACAACGCAGGTAACGCAGGCTCTG ACCATGAGGCACTGCAACTGGCCCAAGAGTACGTGGACGTATTAGCGAAGC GTGCAGGCGCTCTGGCGGGTATCTCTCCGATGTTCCAGCCGTGTGTCGTACC GCCGAAACCTTGGGTAGCAATCACAGGGGGCGGCTATTGGGCTAACGGTCG CAGACCTTTGGCACTCGTTCGCACTCACTCTAAGAAGGGCTTGATGCGCTAC GAAGACGTTTACATGCCAGAAGTCTACAAGGCTGTGAACCTCGCGCAAAAC ACCGCATGGAAAATCAACAAGAAAGTTCTTGCTGTTGTCAATGAGATTGTTA ACTGGAAGAATTGCCCGGTAGCAGACATTCCATCGCTGGAGCGCCAAGAGTT ACCGCCTAAGCCTGACGACATTGACACCAACGAGGCAGCGCTCAAGGAGTG GAAGAAAGCCGCTGCTGGTATCTATCGCTTGGACAAGGCACGAGTGTCTCGC CGTATCAGCTTAGAGTTCATGCTGGAGCAGGCCAACAAGTTCGCAAGTAAGA AAGCAATCTGGTTCCCTTACAACATGGACTGGCGCGGTCGTGTGTACGCTGT GCCGATGTTCAACCCGCAAGGCAACGACATGACGAAAGGTCTGCTGACCCTT GCTAAAGGCAAGCCAATCGGTGAGGAAGGTTTCTACTGGCTGAAAATCCAC GGTGCGAACTGTGCGGGTGTTGATAAGGTTCCATTCCCGGAGCGCATCGCGT TCATTGAGAAGCACGTAGACGACATTCTGGCTTGCGCTAAAGACCCAATCAA TAACACTTGGTGGGCTGAGCAGGATTCACCGTTCTGTTTCCTCGCGTTTTGCT TCGAGTATGCAGGCGTTACGCACCACGGTCTGAGCTACAATTGCTCTCTGCC GCTGGCGTTCGACGGGTCTTGCTCTGGTATCCAGCACTTCTCCGCGATGCTCC GCGATGAGGTAGGCGGTCGTGCGGTTAACCTGCTGCCAAGCGAAACCGTGC AGGACATTTACGGCATCGTTGCACAGAAAGTAAACGAGATTCTCAAACAGG ATGCAATCAACGGCACGCCTAACGAGATGATTACCGTGACCGACAAGGACA CCGGGGAAATCTCAGAGAAGCTCAAACTTGGAACCTCAACGCTGGCGCAAC AGTGGCTGGCATATGGTGTAACCCGTAGCGTAACTAAACGTTCGGTCATGAC GCTGGCTTACGGTTCCAAGGAGTTCGGCTTTCGTCAACAGGTATTGGATGAC ACCATTCAGCCTGCAATTGACAGCGGTAAGGGCTTGATGTTCACCCAACCGA ACCAAGCGGCTGGCTATATGGCTAAGCTGATTTGGGATGCGGTAAGCGTGAC CGTAGTTGCAGCGGTTGAGGCGATGAACTGGCTCAAATCTGCCGCTAAGCTG CTGGCTGCTGAGGTCAAGGACAAGAAGACCAAGGAGATTCTGCGCCACCGT TGCGCGGTTCACTGGACTACGCCGGACGGCTTCCCGGTCTGGCAGGAATACC GCAAGCCACTCCAGAAGCGTCTCGATATGATTTTCTTAGGGCAATTCCGTCT GCAACCGACGATTAATACCCTCAAGGATTCAGGCATTGACGCACACAAGCA GGAGTCTGGCATCGCTCCTAACTTTGTTCACTCACAGGACGGTAGCCACCTC CGCATGACAGTCGTTTATGCTCACGAGAAGTATGGCATTGAGTCCTTTGCGC TCATCCATGACAGCTTTGGGACTATCCCGGCAGACGCTGGTAAGCTCTTTAA GGCTGTGCGTGAAACGATGGTTATCACCTATGAGAACAACGATGTGCTGGCA GACTTCTACTCTCAGTTTGCCGACCAGCTACACGAGACCCAACTGGACAAGA TGCCTCCGCTTCCGAAGAAAGGAAACCTGAACCTGCAAGACATTCTCAAGTC TGACTTTGCCTTTGCATAA |
| SEQ ID No.7 | T3 RNA polymerase protein sequence | MNIIENIEKNDFSEIELAAIPFNTLADHYGSALAKEQLALEHESYELGERRFLKML ERQAKAGEIADNAAAKPLLATLLPKLTTRIVEWLEEYASKKGRKPSAYAPLQLL KPEASAFITLKVILASLTSTNMTTIQAAAGMLGKAIEDEARFGRIRDLEAKHFKK HVEEQLNKRHGQVYKKAFMQVVEADMIGRGLLGGEAWSSWDKETTMHVGIR LIEMLIESTGLVELQRHNAGNAGSDHEALQLAQEYVDVLAKRAGALAGISPMFQ PCVVPPKPWVAITGGGYWANGRRPLALVRTHSKKGLMRYEDVYMPEVYKAVN LAQNTAWKINKKVLAVVNEIVNWKNCPVADIPSLERQELPPKPDDIDTNEAALK EWKKAAAGIYRLDKARVSRRISLEFMLEQANKFASKKAIWFPYNMDWRGRVY AVPMFNPQGNDMTKGLLTLAKGKPIGEEGFYWLKIHGANCAGVDKVPFPERIA FIEKHVDDILACAKDPINNTWWAEQDSPFCFLAFCFEYAGVTHHGLSYNCSLPL AFDGSCSGIQHFSAMLRDEVGGRAVNLLPSETVQDIYGIVAQKVNEILKQDAING TPNEMITVTDKDTGEISEKLKLGTSTLAQQWLAYGVTRSVTKRSVMTLAYGSKE FGFRQQVLDDTIQPAIDSGKGLMFTQPNQAAGYMAKLIWDAVSVTVVAAVEA MNWLKSAAKLLAAEVKDKKTKEILRHRCAVHWTTPDGFPVWQEYRKPLQKRL DMIFLGQFRLQPTINTLKDSGIDAHKQESGIAPNFVHSQDGSHLRMTVVYAHEK YGIESFALIHDSFGTIPADAGKLFKAVRETMVITYENNDVLADFYSQFADQLHET QLDKMPPLPKKGNLNLQDILKSDFAFA |
| SEQ ID No.8 | SP6 RNA polymerase gene coding region | ATGCAAGATTTACACGCTATCCAGCTTCAATTAGAAGAAGAGATGTTTAATG GTGGCATTCGTCGCTTCGAAGCAGATCAACAACGCCAGATTGCAGCAGGTAG CGAGAGCGACACAGCATGGAACCGCCGCCTGTTGTCAGAACTTATTGCACCT ATGGCTGAAGGCATTCAGGCTTATAAAGAAGAGTACGAAGGTAAGAAAGGT CGTGCACCTCGCGCATTGGCTTTCTTACAATGTGTAGAAAATGAAGTTGCAG CATACATCACTATGAAAGTTGTTATGGATATGCTGAATACGGATGCTACCCT TCAGGCTATTGCAATGAGTGTAGCAGAACGCATTGAAGACCAAGTGCGCTTT TCTAAGCTAGAAGGTCACGCCGCTAAATACTTTGAGAAGGTTAAGAAGTCAC TCAAGGCTAGCCGTACTAAGTCATATCGTCACGCTCATAACGTAGCTGTAGT TGCTGAAAAATCAGTTGCAGAAAAGGACGCGGACTTTGACCGTTGGGAGGC GTGGCCAAAAGAAACTCAATTGCAGATTGGTACTACCTTGCTTGAAATCTTA GAAGGTAGCGTTTTCTATAATGGTGAACCTGTATTTATGCGTGCTATGCGCA CTTATGGCGGAAAGACTATTTACTACTTACAAACTTCTGAAAGTGTAGGCCA GTGGATTAGCGCATTCAAAGAGCACGTAGCGCAATTAAGCCCAGCTTATGCC CCTTGCGTAATCCCTCCTCGTCCTTGGAGAACTCCATTTAATGGAGGGTTCCA TACTGAGAAGGTAGCTAGCCGTATCCGTCTTGTAAAAGGTAACCGTGAGCAT GTACGCAAGTTGACTCAAAAGCAAATGCCAAAGGTTTATAAGGCTATCAAC GCATTACAAAATACACAATGGCAAATCAACAAGGATGTATTAGCAGTTATTG AAGAAGTAATCCGCTTAGACCTTGGTTATGGTGTACCTTCCTTCAAGCCACT GATTGACAAGGAGAACAAGCCAGCTAACCCGGTACCTGTTGAATTCCAACA CCTGCGCGGTCGTGAACTGAAAGAGATGCTATCACCTGAGCAGTGGCAACA ATTCATTAACTGGAAAGGCGAATGCGCGCGCCTATATACCGCAGAAACTAA GCGCGGTTCAAAGTCCGCCGCCGTTGTTCGCATGGTAGGACAGGCCCGTAAA TATAGCGCCTTTGAATCCATTTACTTCGTGTACGCAATGGATAGCCGCAGCC GTGTCTATGTGCAATCTAGCACGCTCTCTCCGCAGTCTAACGACTTAGGTAA GGCATTACTCCGCTTTACCGAGGGACGCCCTGTGAATGGCGTAGAAGCGCTT AAATGGTTCTGCATCAATGGTGCTAACCTTTGGGGATGGGACAAGAAAACTT TTGATGTGCGCGTGTCTAACGTATTAGATGAGGAATTCCAAGATATGTGTCG AGACATCGCCGCAGACCCTCTCACATTCACCCAATGGGCTAAAGCTGATGCA CCTTATGAATTCCTCGCTTGGTGCTTTGAGTATGCTCAATACCTTGATTTGGT GGATGAAGGAAGGGCCGACGAATTCCGCACTCACCTACCAGTACATCAGGA CGGGTCTTGTTCAGGCATTCAGCACTATAGTGCTATGCTTCGCGACGAAGTA GGGGCCAAAGCTGTTAACCTGAAACCCTCCGATGCACCGCAGGATATCTATG GGGCGGTGGCGCAAGTGGTTATCAAGAAGAATGCGCTATATATGGATGCGG ACGATGCAACCACGTTTACTTCTGGTAGCGTCACGCTGTCCGGTACAGAACT GCGAGCAATGGCTAGCGCATGGGATAGTATTGGTATTACCCGTAGCTTAACC AAAAAGCCCGTGATGACCTTGCCATATGGTTCTACTCGCTTAACTTGCCGTG AATCTGTGATTGATTACATCGTAGACTTAGAGGAAAAAGAGGCGCAGAAGG CAGTAGCAGAAGGGCGGACGGCAAACAAGGTACATCCTTTTGAAGACGATC GTCAAGATTACTTGACTCCGGGCGCAGCTTACAACTACATGACGGCACTAAT CTGGCCTTCTATTTCTGAAGTAGTTAAGGCACCGATAGTAGCTATGAAGATG ATACGCCAGCTTGCACGCTTTGCAGCGAAACGTAATGAAGGCCTGATGTACA CCCTGCCTACTGGCTTCATCTTAGAACAGAAGATCATGGCAACCGAGATGCT ACGCGTGCGTACCTGTCTGATGGGTGATATCAAGATGTCCCTTCAGGTTGAA ACGGATATCGTAGATGAAGCCGCTATGATGGGAGCAGCAGCACCTAATTTCG TACACGGTCATGACGCAAGTCACCTTATCCTTACCGTATGTGAATTGGTAGA CAAGGGCGTAACTAGTATCGCTGTAATCCACGACTCTTTTGGTACTCATGCA GACAACACCCTCACTCTTAGAGTGGCACTTAAAGGGCAGATGGTTGCAATGT ATATTGATGGTAATGCGCTTCAGAAACTACTGGAGGAGCATGAAGAGCGCT GGATGGTTGATACAGGTATCGAAGTACCTGAGCAAGGGGAGTTCGACCTTA ACGAAATCATGGATTCTGAATACGTATTTGCCTAA |
| SEQ ID No.9 | SP6 RNA polymerase protein sequence | MQDLHAIQLQLEEEMFNGGIRRFEADQQRQIAAGSESDTAWNRRLLSELIAPMA EGIQAYKEEYEGKKGRAPRALAFLQCVENEVAAYITMKVVMDMLNTDATLQAI AMSVAERIEDQVRFSKLEGHAAKYFEKVKKSLKASRTKSYRHAHNVAVVAEKS VAEKDADFDRWEAWPKETQLQIGTTLLEILEGSVFYNGEPVFMRAMRTYGGKT IYYLQTSESVGQWISAFKEHVAQLSPAYAPCVIPPRPWRTPFNGGFHTEKVASRI RLVKGNREHVRKLTQKQMPKVYKAINALQNTQWQINKDVLAVIEEVIRLDLGY GVPSFKPLIDKENKPANPVPVEFQHLRGRELKEMLSPEQWQQFINWKGECARLY TAETKRGSKSAAVVRMVGQARKYSAFESIYFVYAMDSRSRVYVQSSTLSPQSN DLGKALLRFTEGRPVNGVEALKWFCINGANLWGWDKKTFDVRVSNVLDEEFQ DMCRDIAADPLTFTQWAKADAPYEFLAWCFEYAQYLDLVDEGRADEFRTHLPV HQDGSCSGIQHYSAMLRDEVGAKAVNLKPSDAPQDIYGAVAQVVIKKNALYM DADDATTFTSGSVTLSGTELRAMASAWDSIGITRSLTKKPVMTLPYGSTRLTCRE SVIDYIVDLEEKEAQKAVAEGRTANKVHPFEDDRQDYLTPGAAYNYMTALIWP SISEVVKAPIVAMKMIRQLARFAAKRNEGLMYTLPTGFILEQKIMATEMLRVRT CLMGDIKMSLQVETDIVDEAAMMGAAAPNFVHGHDASHLILTVCELVDKGVTS IAVIHDSFGTHADNTLTLRVALKGQMVAMYIDGNALQKLLEEHEERWMVDTGI EVPEQGEFDLNEIMDSEYVFA |
| SEQ ID No.10 | *lacUV5* promoter | TCACTCATTAGGCACCCCAGGCTTTACACTTTATGCTTCCGGCTCGTATAATG TGTGGAA |
| SEQ ID No.11 | *asd* gene | TTGCAGGAAAAAAACGCTATGAAAAATGTTGGTTTTATCGGCTGGCGCGGAA TGGTCGGCTCTGTTCTCATGCAACGCATGGTAGAGGAGCGCGATTTCGACGC TATTCGCCCTGTTTTCTTTTCTACCTCCCAGTTTGGACAGGCGGCGCCCACCT TCGGCGACACCTCCACCGGCACGCTACAGGACGCTTTTGATCTGGATGCGCT AAAAGCGCTCGATATCATCGTGACCTGCCAGGGCGGCGATTATACCAACGA AATTTATCCAAAGCTGCGCGAAAGCGGATGGCAGGGTTACTGGATTGATGCG GCTTCTACGCTGCGCATGAAAGATGATGCCATTATTATTCTCGACCCGGTCA ACCAGGACGTGATTACCGACGGCCTGAACAATGGCGTGAAGACCTTTGTGG GCGGTAACTGTACCGTTAGCCTGATGTTGATGTCGCTGGGCGGTCTCTTTGCC CATAATCTCGTTGACTGGGTATCCGTCGCGACCTATCAGGCCGCCTCCGGCG GCGGCGCGCGCCATATGCGCGAGCTGTTAACCCAGATGGGTCAGTTGTATGG CCATGTCGCCGATGAACTGGCGACGCCGTCTTCCGCAATTCTTGATATTGAA CGCAAAGTTACGGCATTGACCCGCAGCGGCGAGCTGCCGGTTGATAACTTTG GCGTACCGCTGGCGGGAAGCCTGATCCCCTGGATCGACAAACAGCTCGATA ACGGCCAGAGCCGCGAAGAGTGGAAAGGCCAGGCGGAAACCAACAAGATT CTCAATACTGCCTCTGTGATTCCGGTTGATGGTTTGTGTGTGCGCGTCGGCGC GCTGCGCTGTCACAGCCAGGCGTTCACCATCAAGCTGAAAAAAGAGGTATCC ATTCCGACGGTGGAAGAACTGCTGGCGGCACATAATCCGTGGGCGAAAGTG GTGCCGAACGATCGTGATATCACTATGCGCGAATTAACCCCGGCGGCGGTGA CCGGCACGTTGACTACGCCGGTTGGTCGTCTGCGTAAGCTGAACATGGGGCC AGAGTTCTTGTCGGCGTTTACCGTAGGCGACCAGTTGTTATGGGGCGCCGCC GAGCCGCTGCGTCGAATGCTGCGCCAGTTGGCGTAG |
| SEQ ID No.12 | Asd protein sequence | LQEKNAMKNVGFIGWRGMVGSVLMQRMVEERDFDAIRPVFFSTSQFGQAAPTF GDTSTGTLQDAFDLDALKALDIIVTCQGGDYTNEIYPKLRESGWQGYWIDAAST LRMKDDAIIILDPVNQDVITDGLNNGVKTFVGGNCTVSLMLMSLGGLFAHNLV DWVSVATYQAASGGGARHMRELLTQMGQLYGHVADELATPSSAILDIERKVTA LTRSGELPVDNFGVPLAGSLIPWIDKQLDNGQSREEWKGQAETNKILNTASVIPV DGLCVRVGALRCHSQAFTIKLKKEVSIPTVEELLAAHNPWAKVVPNDRDITMRE LTPAAVTGTLTTPVGRLRKLNMGPEFLSAFTVGDQLLWGAAEPLRRMLRQLA |
| SEQ ID No.13 | Listeriolysin O gene coding region | ATGAAAAAAATCATGCTGGTTTTCATCACCCTCATACTGGTAAGCCTGCCGA TCGCGCAGCAGACCGAAGCGAAAGACGCGTCTGCGTTCAACAAAGAAAACT CTATCTCTTCTATGGCGCCGCCGGCGTCTCCGCCGGCGTCTCCGAAAACCCC GATCGAAAAAAAACACGCGGACGAAATCGACAAATACATCCAGGGCCTGGA CTACAACAAAAACAACGTTCTGGTTTACCACGGCGACGCGGTTACCAACGTT CCGCCGCGTAAAGGCTACAAAGACGGCAACGAATACATCGTTGTTGAAAAA AAAAAAAAATCTATCAACCAGAACAACGCGGACATCCAGGTTGTTAACGCG ATCTCTTCTCTGACCTACCCGGGCGCGCTGGTTAAAGCGAACTCTGAACTGG TTGAAAACCAGCCGGACGTTCTGCCGGTTAAACGTGACTCTCTGACCCTGTC TATCGACCTGCCGGGCATGACCAACCAGGACAACAAAATCGTTGTTAAAAA CGCGACCAAATCTAACGTTAACAACGCGGTTAACACCCTGGTTGAACGTTGG AACGAAAAATACGCGCAGGCGTACCCGAACGTTTCTGCGAAAATCGACTAC GACGACGAAATGGCGTACTCTGAATCTCAGCTGATCGCGAAATTCGGCACCG CGTTCAAAGCGGTTAACAACTCTCTGAACGTTAACTTCGGCGCGATCTCTGA AGGCAAAATGCAGGAAGAAGTTATCTCTTTCAAACAGATCTACTACAACGTT AACGTTAACGAACCGACCCGTCCGTCTCGTTTCTTCGGCAAAGCGGTTACCA AAGAACAGCTTCAGGCGCTGGGCGTTAACGCGGAAAACCCGCCGGCGTACA TCTCTTCTGTTGCGTACGGCCGTCAGGTTTACCTGAAACTGTCTACCAACTCT CACTCTACCAAAGTTAAAGCGGCTTTCGACGCGGCGGTAAGCGGCAAATCTG TTTCTGGCGACGTTGAACTGACCAACATCATCAAAAACTCTTCTTTCAAAGC GGTTATCTACGGCGGCTCTGCGAAAGACGAAGTTCAGATCATCGACGGCAAC CTGGGCGACCTGCGTGACATCCTGAAAAAAGGCGCGACCTTCAACCGTGAA ACCCCGGGCGTTCCGATCGCGTACACCACCAACTTCCTGAAAGACAACGAAC TGGCGGTTATCAAAAACAACTCTGAATACATCGAAACCACCTCTAAAGCGTA CACCGACGGCAAAATCAACATCGACCACTCTGGCGGCTACGTTGCGCAGTTC AACATCTCTTGGGACGAAGTTAACTACGACCCGGAAGGCAACGAAATCGTTC AGCACAAAAACTGGTCTGAAAACAACAAATCTAAACTGGCGCACTTCACCTC TTCTATCTACCTGCCGGGCAACGCGCGTAACATCAACGTTTACGCGAAAGAA TGCACCGGCCTGGCGTGGGAATGGTGGCGTACCGTTATCGACGACCGTAACC TGCCGCTGGTTAAAAACCGTAACATCTCTATCTGGGGCACCACCCTGTACCC GAAATACTCTAACAAAGTTGACAACCCGATCGAATAATGA |
| SEQ ID No.14 | Listeriolysin O protein sequence | MKKIMLVFITLILVSLPIAQQTEAKDASAFNKENSISSMAPPASPPASPKTPIEKKH ADEIDKYIQGLDYNKNNVLVYHGDAVTNVPPRKGYKDGNEYIVVEKKKKSINQ NNADIQVVNAISSLTYPGALVKANSELVENQPDVLPVKRDSLTLSIDLPGMTNQ DNKIVVKNATKSNVNNAVNTLVERWNEKYAQAYPNVSAKIDYDDEMAYSESQ LIAKFGTAFKAVNNSLNVNFGAISEGKMQEEVISFKQIYYNVNVNEPTRPSRFFG KAVTKEQLQALGVNAENPPAYISSVAYGRQVYLKLSTNSHSTKVKAAFDAAVS GKSVSGDVELTNIIKNSSFKAVIYGGSAKDEVQIIDGNLGDLRDILKKGATFNRET PGVPIAYTTNFLKDNELAVIKNNSEYIETTSKAYTDGKINIDHSGGYVAQFNISW DEVNYDPEGNEIVQHKNWSENNKSKLAHFTSSIYLPGNARNINVYAKECTGLA WEWWRTVIDDRNLPLVKNRNISIWGTTLYPKYSNKVDNPIE |
| SEQ ID No.15 | *Salmonella* codon-optimized DTA gene | ATGGGCGCGGACGACGTTGTTGACTCTTCTAAATCTTTCGTTATGGAAAACTT CTCTTCTTACCACGGCACCAAACCGGGCTACGTTGACTCTATCCAGAAAGGC ATCCAGAAACCGAAATCTGGCACCCAGGGCAACTACGACGACGACTGGAAA GGCTTCTACTCTACCGACAACAAATACGACGCGGCGGGCTACTCTGTTGACA ACGAAAACCCGCTGTCTGGCAAAGCGGGCGGCGTTGTTAAAGTTACCTACCC GGGCCTGACCAAAGTTCTGGCGCTGAAAGTTGACAACGCGGAAACCATCAA AAAAGAACTGGGCCTGTCTCTGACCGAACCGCTGATGGAACAGGTTGGCAC CGAAGAATTTATCAAACGTTTCGGCGACGGCGCGTCTCGTGTTGTTCTGTCTC TGCCGTTCGCGGAAGGCTCTTCTTCTGTTGAATACATCAACAACTGGGAACA GGCGAAAGCGCTGTCTGTTGAACTGGAAATCAACTTCGAAACCCGTGGCAA ACGTGGCCAGGACGCGATGTACGAATACATGGCGCAGGCGTGCGCGGGCAA CCGTGTTCGTCGTTCTTAA |
| SEQ ID No.16 | DTA protein sequence | MGADDVVDSSKSFVMENFSSYHGTKPGYVDSIQKGIQKPKSGTQGNYDDDWK GFYSTDNKYDAAGYSVDNENPLSGKAGGVVKVTYPGLTKVLALKVDNAETIK KELGLSLTEPLMEQVGTEEFIKRFGDGASRVVLSLPFAEGSSSVEYINNWEQAKA LSVELEINFETRGKRGQDAMYEYMAQACAGNRVRRS |
| SEQ ID No.17 | *Salmonella* codon-optimized Cholera toxin subunit A gene | ATGGTGAAAATCATTTTCGTATTTTTTATTTTTCTGAGCAGCTTCAGCTATGC CAACGATGATAAACTGTACCGCGCGGATAGCCGCCCGCCGGATGAAATTAA ACAGAGCGGTGGTCTGATGCCGCGCGGCCAGTCGGAATACTTTGATCGCGGC ACCCAGATGAACATTAACCTGTACGACCATGCCCGCGGCACCCAGACCGGCT TTGTGCGCCATGATGATGGCTACGTGAGCACCAGTATTAGCCTGCGCAGCGC GCATCTGGTGGGCCAGACCATTCTGAGCGGCCATTCGACCTATTATATTTAT GTGATTGCGACCGCGCCGAACATGTTTAATGTGAATGATGTGCTGGGCGCCT ATAGCCCGCACCCGGATGAACAGGAAGTGAGCGCGCTGGGCGGCATTCCGT ATAGCCAGATTTACGGCTGGTATCGCGTGCACTTTGGCGTCCTGGATGAACA GCTGCACCGCAACCGCGGCTACCGTGATCGCTATTATAGCAACCTGGATATT GCGCCGGCCGCGGATGGCTATGGCCTGGCGGGCTTTCCTCCGGAACATCGCG CGTGGCGCGAGGAACCGTGGATTCACCACGCCCCGCCGGGCTGCGGCAACG CCCCGCGCAGCAGCATGAGCAACACCTGCGATGAAAAAACCCAGAGCCTGG GCGTGAAATTTCTGGATGAATACCAGTCAAAAGTGAAACGCCAGATCTTTAG CGGCTATCAGAGCGATATTGATACCCATAACCGCATTAAAGATGAACTGTGA |
| SEQ ID No.18 | Cholera toxin subunit A protein sequence | MVKIIFVFFIFLSSFSYANDDKLYRADSRPPDEIKQSGGLMPRGQSEYFDRGTQM NINLYDHARGTQTGFVRHDDGYVSTSISLRSAHLVGQTILSGHSTYYIYVIATAP NMFNVNDVLGAYSPHPDEQEVSALGGIPYSQIYGWYRVHFGVLDEQLHRNRGY RDRYYSNLDIAPAADGYGLAGFPPEHRAWREEPWIHHAPPGCGNAPRSSMSNT CDEKTQSLGVKFLDEYQSKVKRQIFSGYQSDIDTHNRIKDEL |
| SEQ ID No.19 | Shiga toxin 1 subunit A gene | ATGAAAATAATTATTTTTAGAGTGCTAACTTTTTTCTTTGTTATCTTTTCAGTT AATGTGGTTGCGAAGGAATTTACCTTAGACTTCTCGACTGCAAAGACGTATG TAGATTCGCTGAATGTCATTCGCTCTGCAATAGGTACTCCATTACAGACTATT TCATCAGGAGGTACGTCTTTACTGATGATTGATAGTGGCACAGGGGATAATT TGTTTGCAGTTGATGTCAGAGGGATAGATCCAGAGGAAGGGCGGTTTAATAA TCTACGGCTTATTGTTGAACGAAATAATTTATATGTGACAGGATTTGTTAACA GGACAAATAATGTTTTTTATCGCTTTGCTGATTTTTCACATGTTACCTTTCCA GGTACAACAGCGGTTACATTGTCTGGTGACAGTAGCTATACCACGTTACAGC GTGTTGCAGGGATCAGTCGTACGGGGATGCAGATAAATCGCCATTCGTTGAC TACTTCTTATCTGGATTTAATGTCGCATAGTGGAACCTCACTGACGCAGTCTG TGGCAAGAGCGATGTTACGGTTTGTTACTGTGACAGCTGAAGCTTTACGTTTT CGGCAAATACAGAGGGGATTTCGTACAACACTGGATGATCTCAGTGGGCGTT CTTATGTAATGACTGCTGAAGATGTTGATCTTACATTGAACTGGGGAAGGTT GAGTAGTGTCCTGCCTGATTATCATGGACAAGACTCTGTTCGTGTAGGAAGA ATTTCTTTTGGAAGCATTAATGCAATTCTGGGAAGCGTGGCATTAATACTGA ATTGTCATCATCATGCATCGCGAGTTGCCAGAATGGCATCTGATGAGTTTCCT TCTATGTGTCCGGCAGATGGAAGAGTCCGTGGGATTACGCACAATAAAATAT TGTGGGATTCATCCACTCTGGGGGCAATTCTGATGCGCAGAACTATTAGCAG TTGA |
| SEQ ID No.20 | Shiga toxin 1 subunit A protein sequence | |
| SEQ ID No.21 | Shiga toxin 2 subunit A gene | ATGAAGTGTATATTATTTAAATGGGTACTGTGCCTGTTACTGGGTTTTTCTTC GGTATCCTATTCCCGGGAGTTTACGATAGACTTTTCGACCCAACAAAGTTAT GTCTCTTCGTTAAATAGTATACGGACAGAGATATCGACCCCTCTTGAACATA TATCTCAGGGGACCACATCGGTGTCTGTTATTAACCACACCCCACCGGGCAG TTATTTTGCTGTGGATATACGAGGGCTTGATGTCTATCAGGCGCGTTTTGACC ATCTTCGTCTGATTATTGAGCAAAATAATTTATATGTGGCCGGGTTCGTTAAT ACGGCAACAAATACTTTCTACCGTTTTTCAGATTTTACACATATATCAGTGCC CGGTGTGACAACGGTTTCCATGACAACGGACAGCAGTTATACCACTCTGCAA CGTGTCGCAGCGCTGGAACGTTCCGGAATGCAAATCAGTCGTCACTCACTGG |
| | | TTTCATCATATCTGGCGTTAATGGAGTTCAGTGGTAATACAATGACCAGAGA TGCATCCAGAGCAGTTCTGCGTTTTGTCACTGTCACAGCAGAAGCCTTACGC TTCAGGCAGATACAGAGAGAATTTCGTCAGGCACTGTCTGAAACTGCTCCTG TGTATACGATGACGCCGGGAGACGTGGACCTCACTCTGAACTGGGGGCGAA TCAGCAATGTGCTTCCGGAGTATCGGGGAGAGGATGGTGTCAGAGTGGGGA GAATATCCTTTAATAATATATCAGCGATACTGGGGACTGTGGCCGTTATACT GAATTGCCATCATCAGGGGGCGCGTTCTGTTCGCGCCGTGAATGAAGAGAGT CAACCAGAATGTCAGATAACTGGCGACAGGCCTGTTATAAAAATAAACAAT ACATTATGGGAAAGTAATACAGCTGCAGCGTTTCTGAACAGAAAGTCACAGT TTTTATATACAACGGGTAAATAA |
| SEQ ID No.22 | Shiga toxin 2 subunit A protein sequence | MKCILFKWVLCLLLGFSSVSYSREFTIDFSTQQSYVSSLNSIRTEISTPLEHISQGTT SVSVINHTPPGSYFAVDIRGLDVYQARFDHLRLIIEQNNLYVAGFVNTATNTFYR FSDFTHISVPGVTTVSMTTDSSYTTLQRVAALERSGMQISRHSLVSSYLALMEFS GNTMTRDASRAVLRFVTVTAEALRFRQIQREFRQALSETAPVYTMTPGDVDLTL NWGRISNVLPEYRGEDGVRVGRISFNNISAILGTVAVILNCHHQGARSVRAVNEE SQPECQITGDRPVIKINNTLWESNTAAAFLNRKSQFLYTTGK |
| SEQ ID No.23 | *Salmonella* codon-optimized Pertussis toxin subunit A gene | ATGGATGATCCGCCGGCCACCGTGTATCGCTACGATTCGCGCCCGCCGGAAG ATGTGTTTCAGAACGGCTTTACCGCCTGGGGCAATAACGATAATGTTCTGGA TCACCTGACCGGTCGTTCATGCCAGGTGGGCAGCAGCAACAGCGCCTTCGTG AGCACCAGCAGCAGCCGCCGCTATACCGAAGTGTACCTGGAACATCGCATG CAGGAAGCCGTGGAAGCGGAACGCGCAGGCCGTGGCACCGGCCACTTTATT GGCTATATTTATGAGGTGCGCGCCGATAACAACTTTTACGGCGCCGCATCAA GCTACTTTGAATACGTGGATACCTATGGCGATAACGCGGGCCGCATTCTGGC GGGCGCGCTGGCGACCTATCAGAGCGAATATCTGGCCCACCGCCGTATTCCG CCGGAAAATATTCGCCGCGTTACCCGCGTGTATCATAATGGCATTACCGGCG AAACCACCACCACCGAATATAGCAACGCGCGCTATGTGAGCCAGCAGACCC GCGCGAACCCGAACCCGTATACCTCCCGTCGTAGCGTGGCTAGCATTGTGGG TACCCTGGTGCGCATGGCGCCGGTGATTGGCGCGTGCATGGCGCGTCAGGCC GAAAGCAGCGAAGCGATGGCGGCCTGGAGCGAACGCGCGGGCGAAGCGAT GGTGCTGGTGTACTACGAATCGATTGCCTATAGCTTTTAA |
| SEQ ID No.24 | Pertussis toxin subunit A protein sequence | MDDPPATVYRYDSRPPEDVFQNGFTAWGNNDNVLDHLTGRSCQVGSSNSAFVS TSSSRRYTEVYLEHRMQEAVEAERAGRGTGHFIGYIYEVRADNNFYGAASSYFE YVDTYGDNAGRILAGALATYQSEYLAHRRIPPENIRRVTRVYHNGITGETTTTEY SNARYVSQQTRANPNPYTSRRSVASIVGTLVRMAPVIGACMARQAESSEAMAA WSERAGEAMVLVYYESIAYSF |
| SEQ ID No.25 | *Salmonella* codon-optimized Pseudomonas exotoxin A subunit gene | ATGCACCTGACCCCACATTGGATTCCGCTGGTGGCAAGCCTGGGCCTGCTTG CGGGCGGCAGCTTTGCGAGCGCGGCCGAAGAAGCATTTGATTTGTGGAACG AATGTGCCAAAGCGTGTGTGCTGGATCTTAAAGATGGCGTTCGTAGTAGCCG CATGTCAGTTGATCCGGCAATCGCGGATACCAATGGTCAGGGCGTTCTGCAT TATTCAATGGTGCTGGAAGGCGGCAATGATGCGCTGAAACTGGCCATTGACA ATGCACTGAGCATTACCAGCGATGGCCTGACCATTCGTCTGGAAGGCGGCGT GGAACCGAATAAACCGGTGCGCTATAGCTACACCCGCCAGGCCCGTGGCAG CTGGAGCCTGAACTGGCTGGTGCCGATTGGCCATGAAAAACCGAGCAATATT AAAGTGTTTATTCACGAATTAAACGCCGGTAACCAGCTGAGCCACATGAGTC CGATTTATACTATTGAAATGGGCGATGAACTGCTGGCGAAACTGGCCCGTGA TGCCACCTTCTTCGTTCGCGCGCATGAAAGCAATGAAATGCAGCCGACCCTG GCGATTTCACACGCCGGCGTATCAGTTGTGATGGCCCAGGCGCAGCCTCGCC GCGAAAAACGCTGGAGCGAATGGGCCAGCGGTAAAGTGCTGTGCCTGCTGG ATCCACTGGATGGCGTGTATAATTATCTGGCGCAGCAGCGTTGCAATCTGGA TGATACCTGGGAAGGTAAAATTTATCGTGTGCTGGCCGGCAATCCGGCGAAA CATGATCTGGATATTAAACCGACCGTGATTAGCCATCGCCTGCATTTTCCGG AAGGCGGTAGCCTGGCGGCCCTGACCGCGCATCAGGCGTGCCATCTGCCGTT GGAAACCTTTACCCGCCATCGTCAGCCGCGTGGTTGGGAACAGCTGGAACAG TGTGGCTATCCGGTGCAGCGCCTGGTGGCACTGTACCTGGCGGCCCGTCTGA GCTGGAACCAGGTTGATCAGGTTATTCGCAATGCCCTGGCCAGCCCGGGCAG CGGCGGCGATTTAGGCGAAGCTATTCGCGAACAGCCGGAACAGGCCCGCCT GGCCCTGACCCTGGCGGCGGCCGAAAGCGAACGCTTTGTGCGCCAGGGCAC CGGCAATGATGAAGCGGGCGCGGCGAGCGCAGATGTTGTGAGCCTGACCTG CCCGGTGGCGGCCGGCGAATGTGCGGGCCCGGCGGATAGCGGTGATGCCCT GCTGGAACGTAATTATCCGACCGGTGCCGAATTTCTGGGCGATGGCGGCGAT ATCTCGTTCAGCACCCGTGGCACGCAGAACTGGACGGTCGAACGTCTGCTGC AAGCCCATCGCCAGCTGGAAGAACGCGGCTATGTATTCGTGGGCTATCATGG CACCTTTCTGGAAGCGGCCCAGAGCATTGTTTTTGGCGGCGTGCGTGCCCGT TCTCAGGATCTGGATGCGATTTGGCGCGGCTTTTATATTGCCGGTGATCCCGC GCTGGCTTATGGCTACGCACAGGATCAGGAACCGGACGCGCGCGGCCGTATT CGCAACGGCGCGCTGCTGCGTGTGTATGTGCCGCGCAGCAGCCTGCCGGGCT TTTATCGTACGGGCCTGACCCTGGCCGCCCCGGAAGCCGCCGGCGAAGTAGA ACGTCTGATTGGCCATCCGCTGCCGCTTCGCCTGGATGCGATTACCGGCCCG GAAGAGGAAGGCGGTCGTCTGGAAACGATTCTGGGCTGGCCGTTGGCGGAA CGCACCGTGGTGATTCCGAGCGCGATTCCGACCGATCCGCGCAACGTTGGCG |
| | | GCGACCTGGACCCGTCATCGATTCCAGATAAAGAACAGGCGATCAGCGCGC TGCCGGATTATGCGTCGCAGCCGGGCAAACCGCCGCGCGAAGATCTGAAAT AA |
| SEQ ID No.26 | Pseudomonas exotoxin A subunit protein sequence | MHLTPHWIPLVASLGLLAGGSFASAAEEAFDLWNECAKACVLDLKDGVRSSRM SVDPAIADTNGQGVLHYSMVLEGGNDALKLAIDNALSITSDGLTIRLEGGVEPN KPVRYSYTRQARGSWSLNWLVPIGHEKPSNIKVFIHELNAGNQLSHMSPIYTIEM GDELLAKLARDATFFVRAHESNEMQPTLAISHAGVSVVMAQAQPRREKRWSE WASGKVLCLLDPLDGVYNYLAQQRCNLDDTWEGKIYRVLAGNPAKHDLDIKP TVISHRLHFPEGGSLAALTAHQACHLPLETFTRHRQPRGWEQLEQCGYPVQRLV ALYLAARLSWNQVDQVIRNALASPGSGGDLGEAIREQPEQARLALTLAAAESER FVRQGTGNDEAGAASADVVSLTCPVAAGECAGPADSGDALLERNYPTGAEFLG DGGDISFSTRGTQNWTVERLLQAHRQLEERGYVFVGYHGTFLEAAQSIVFGGVR ARSQDLDAIWRGFYIAGDPALAYGYAQDQEPDARGRIRNGALLRVYVPRSSLPG FYRTGLTLAAPEAAGEVERLIGHPLPLRLDAITGPEEEGGRLETILGWPLAERTV VIPSAIPTDPRNVGGDLDPSSIPDKEQAISALPDYASQPGKPPREDLK |
| SEQ ID No.27 | *Salmonella alr* gene | 5'- ATGCAAGCGGCAACAGTCGTCATTAACCGCCGCGCTCTGCGACACAACCTGC AACGTCTGCGTGAACTGGCGCCTGCCAGTAAGCTGGTTGCGGTGGTGAAAGC GAACGCTTATGGACACGGTCTTCTGGAGACCGCGCGAACGCTCCCTGATGCT GACGCTTTTGGCGTGGCGCGTCTTGAAGAGGCTCTACGTCTGCGAGCGGGCG GGATCACGCAGCCAATCCTGCTGCTGGAGGGTTTTTTCGACGCCGCCGATTT GCCGACCATTTCCGCGCAATGTCTGCATACCGCCGTACATAATCAAGAGCAG CTTGCCGCCCTGGAGGCGGTGGAGCTGGCGGAGCCGGTAACGGTCTGGATG AAGCTGGATACCGGTATGCATCGTCTCGGCGTGCGTCCCGAAGAGGCGGAG GCGTTCTACCAGCGTCTGACGCACTGTAAAAATGTACGCCAGCCGGTGAATA TCGTCAGCCATTTTGCCCGTGCGGATGAGCCGGAATGCGGCGCTACCGAACA TCAGCTCGACATTTTTAATGCCTTCTGTCAGGGTAAACCCGGTCAGCGCTCTA TTGCCGCGTCTGGCGGTATCCTGCTGTGGCCGCAGTCTCACTTTGACTGGGCG CGTCCGGGCATCATTTTGTATGGCGTATCGCCGCTGGAGCACAAACCCTGGG GGCCGGATTTTGGTTTTCAGCCGGTGATGTCCTTAACCTCCAGTTTGATCGCG GTGCGTGACCACAAAGCGGGCGAACCGGTGGGCTACGGCGGGACATGGGTG AGTGAGCGCGACACGCGCCTGGGCGTGGTGGCGATGGGTTATGGCGATGGC TACCCACGAGCGGCGCCTTCCGGTACGCCAGTACTGGTCAATGGTCGTGAAG TTCCGATTGTCGGGCGGGTGGCGATGGATATGATTTGCGTAGATTTGGGGCC AAACGCGCAGGATAACGCGGGCGATCCGGTGGTCTTATGGGGTGAAGGTCT GCCGGTTGAACGTATCGCTGAAATGACAAAAGTAAGTGCTTACGAACTTATC ACGCGCCTGACCTCAAGGGTGGCGATGAAGTATATTGATTAA-3' |
| SEQ ID No.28 | *Salmonella* Alr protein | MQAATVVINRRALRHNLQRLRELAPASKLVAVVKANAYGHGLLETARTLPDA DAFGVARLEEALRLRAGGITQPILLLEGFFDAADLPTISAQCLHTAVHNQEQLAA LEAVELAEPVTVWMKLDTGMHRLGVRPEEAEAFYQRLTHCKNVRQPVNIVSHF ARADEPECGATEHQLDIFNAFCQGKPGQRSIAASGGILLWPQSHFDWARPGIILY GVSPLEHKPWGPDFGFQPVMSLTSSLIAVRDHKAGEPVGYGGTWVSERDTRLG VVAMGYGDGYPRAAPSGTPVLVNGREVPIVGRVAMDMICVDLGPNAQDNAGD PVVLWGEGLPVERIAEMTKVSAYELITRLTSRVAMKYID |
| SEQ ID No.29 | *Salmonella dadX* gene | 5’- ATGACCCGCCCTATACAGGCCAGCCTTGATTTACAGGTAATGAAACAAAATT TGGCTATTGTGCGCCGGGCGGCCCCAGAGGCGCGCGTCTGGTCGGTAGTGAA AGCCAACGCCTACGGCCACGGTATTGAACGCGTCTGGAGCGCGCTGGGCGC CACAGATGGTTTCGCTATGCTCAACCTTGAAGAGGCGATTACCCTGCGTGAG CGCGGGTGGAAAGGGCCGATATTGATGCTGGAGGGGTTTTTCCATGCGCAAG ACCTGGAGGCGTATGACACTTATCGGCTGACCACCTGCATCCACAGTAACTG GCAGTTGAAAGCGCTGCAAAATGCGCGGCTTAACGCGCCGCTGGATATCTAT GTCAAAGTCAACAGCGGCATGAACCGACTCGGTTTTCAGCCTGAGCGGGCGC AGACCGTCTGGCAGCAATTGCGGGCAATGCGCAATGTCGGTGAAATGACCCT GATGTCACATTTTGCTCAGGCCGATCATCCGGAAGGTATCGGGGAGGCGATG AGGCGTATTGCGCTGGCGACGGAAGGTCTTCAGTGTGCATACTCGTTATCAA ATTCGGCAGCGACGCTGTGGCATCCCCAGGCGCATTATGACTGGGTCAGGCC GGGCATTATTTTGTACGGCGCGTCGCCGTCAGGACAGTGGCGGGATATTGCC GATACCGGACTAAAACCTGTGATGACGCTGAGTAGCGAAATTATTGGCGTGC AGACGCTGAGCGCGGGTGAAAGGGTAGGCTATGGCGGAGGGTATTCTGTGA CTCAGGAACAACGCATTGGCATCGTCGCGGCGGGTTATGCCGATGGCTATCC ACGCCATGCGCCGACCGGGACGCCTGTGCTGGTGGACGGTATCCGTACCAGA ACAGTAGGCACCGTTTCAATGGATATGCTGGCGGTGGATTTGACGCCGTGTC CGCAGGCGGGAATCGGCACGCCGGTTGAATTATGGGGCAAAGAAATTAAGG TCGATGATGTCGCTTCTGCGGCAGGCACGCTGGGCTATGAGCTACTGTGCGC CGTAGCGCCGCGTGTGCCGTTTGTGACAACGTAA-3’ |
| SEQ ID No.30 | *Salmonella* DadX protein sequence | MTRPIQASLDLQVMKQNLAIVRRAAPEARVWSVVKANAYGHGIERVWSALGA TDGFAMLNLEEAITLRERGWKGPILMLEGFFHAQDLEAYDTYRLTTCIHSNWQL KALQNARLNAPLDIYVKVNSGMNRLGFQPERAQTVWQQLRAMRNVGEMTLM SHFAQADHPEGIGEAMRRIALATEGLQCAYSLSNSAATLWHPQAHYDWVRPGII LYGASPSGQWRDIADTGLKPVMTLSSEIIGVQTLSAGERVGYGGGYSVTQEQRI GIVAAGYADGYPRHAPTGTPVLVDGIRTRTVGTVSMDMLAVDLTPCPQAGIGTP VELWGKEIKVDDVASAAGTLGYELLCAVAPRVPFVTT |
| SEQ ID No.31 | *Salmonella yhbU* promoter | 5'- GCGTGAACGCAGTTTATCTAACACGAGCTTTACTCCCTGTTTCAATAGTCCTG TTATTTTGCCATATCCAATAAATCACATGGCGGCGTAAATCAACAATTGGGT CTGATTTTCTACCCCATTATTGGTGTTACCAATACGCCTTTAACTGCCTTAAA TCAAAAATTGTCGCAGCAAGGTTAACTAAAATCCCTGTTCGTTAACAATTTT GCGTCTCAGACGTAACCCTCAGGATAA-3' |
| SEQ ID No.32 | *E. coli ynfK* promoter | 5'- CGCTGCACTGGTAAAAGACGCGATGTATAACGGTTCTTTGTTGATTCGTCTGT TGCAGGGTTAACATTTTTTAACTGTTCTACCAAAATTTGCGCTATCTCAATTT GGGCCAGGAAAGCATAACTTAGACTTTCAAGGTTAATTATTTTCCTGGTTTAT ATTTGTGAAGCATAACGGTGGAGTTA-3' |
| SEQ ID No.33 | *Salmonella cyoA* promoter | 5’- AACATTATTTGCCGTTGTAATAACGACCCGGAATTATAACGGCTAAATAACC ATCAATTGATAAAAAATATATTTGTCTTTTTGTGCGCAACAGAATTTTGGAA ATTATTGGTAATTTCTACGCTACCTATTTTCTTTACACGATTCAGCCGATTAA ATTTAAATTTCTGCCGTTGATTGTCCTTTTATTGATAATAAATTGTTAAGTAA TTGTTTTATTTTTACATTGGTTATACCAATTATCCCTCAACATCCCACTTACAG ACCTCATCCGTTATAACGCAAAGTTTCAACAGTGTCTTAAAGTTATTTCGACA TACTTGGCAACATATGTGACATGCGCGGCAGAACTTGGTAACACAGAACCA AT-3’ |
| SEQ ID No.34 | *Salmonella ydcI* promoter | 5’- AATGTCATGCCTCCAGTGAATGTTACCTGGAGTGTAGAAAACCTCATCCATT CCGGGGAGAAGAATTTATAAAGTGTGATCCGACCTGAATTCATTATGCAGAA CGGATTGACATGAGCTGGCATATCCCCGACCAGATCGCGGCTTAACACGCTT AACGGCAGTTGAATCATCAGTAAAATAAAACAGTTGTAACTTGAAATGGTA AAATGTTAATAATATTTTGCTATCAGGTTATCAAAAACAAACAACTTTACTT GTCACCCGCACGGCTCTGTTTTTAACATCGCTT-3’ |
| SEQ ID No.35 | Ribosome binding site (RBS) | TAGTAAGAAGGAGATATACAT |
| SEQ ID No.36 | pUC replicon | CTCGAGCATGTGAGCAAAAGGCCAGCAAAAGGCCAGGAACCGTAAAAAGGC CGCGTTGCTGGCGTTTTTCCATAGGCTCCGCCCCCCTGACGAGCATCACAAA AATCGACGCTCAAGTCAGAGGTGGCGAAACCCGACAGGACTATAAAGATAC CAGGCGTTTCCCCCTGGAAGCTCCCTCGTGCGCTCTCCTGTTCCGACCCTGCC GCTTACCGGATACCTGTCCGCCTTTCTCCCTTCGGGAAGCGTGGCGCTTTCTC ATAGCTCACGCTGTAGGTATCTCAGTTCGGTGTAGGTCGTTCGCTCCAAGCT GGGCTGTGTGCACGAACCCCCCGTTCAGCCCGACCGCTGCGCCTTATCCGGT AACTATCGTCTTGAGTCCAACCCGGTAAGACACGACTTATCGCCACTGGCAG CAGCCACTGGTAACAGGATTAGCAGAGCGAGGTATGTAGGCGGTGCTACAG AGTTCTTGAAGTGGTGGCCTAACTACGGCTACACTAGAAGGACAGTATTTGG TATCTGCGCTCTGCTGAAGCCAGTTACCTTCGGAAAAAGAGTTGGTAGCTCT TGATCCGGCAAACAAACCACCGCTGGTAGCGGTGGTTTTTTTGTTTGCAAGC AGCAGATTACGCGCAGAAAAAAAGGATCTCAAGAAGATCCTTTGATCTTTTC TACGGGGTCTGACGCTCAGTGGAACGAAAACTCACGTTAAGGGATTTTGGTC ATGAGATTATCAAAAAGGATCTTCACCTAGATCCTTTTAAATTAAAAATGAA GTTTTAAATCAATCTAAAGTATATATGAGTAAGCGGCCGC |
| SEQ ID No.37 | SpeI-loxP-chlorampheni col resistance gene-loxP-NotI cassette in pSWT007 | ACTAGTGAACCTCTTCGAGGGACCTAATAACTTCGTATAGCATACATTATAC GAAGTTATATTAAGGGTTCCGGATCCTAATTGATATCTAAGGGCACCAATAA CTGCCTTAAAAAAATTATGCACCACCTTGCCATTCATCACAATACTGTTGCA GTTCATTCAGCATACGACCAACATGAAAACCATCACAAACTGCATGATGAAC CTGAATTGCCAGCGGCATCAGAACTTTATCACCCTGGGTGTAATATTTGCCC ATCGTAAAAACCGGTGCGAAAAAGTTATCCATATTGGCCACATTCAGATCAA ATGAGGTAAAGCTAACCCACGGATTTGCGCTAACAAAGAACATGTTTTCGAT AAAGCCTTTCGGAAAATATGCCAGGTTTTCACCATAACATGCAACATCCTGG CTATAGATGTGCAGGAACTGACGAAAATCATCATGATATTCTGACCACAGGC TGCTAAAGGTTTCGGTCTGTTCATGAAAAACGGTATAACACGGATGAACGCT ATCCCAAATAACCAGTTCACCATCTTTCATTGCCATACGAAATTCCGGATGG GCATTCATCAGACGTGCCAGAATATGAATAAAGGCAGGGTAGAACTTGTGTT TGTTCTTTTTCACGGTTTTCAGAAATGCGGTTATATCCAGCTGAACGGTCTGA TTATAGGTACACTGTGCAACGCTCTGAAATGCTTCAAAGTGTTCTTTACGATG CCACTGGCTAATATCAACGGTGGTATAACCGGTGATTTTCTTTTCCATTTTAG CTTCCTTAGCTCCTGAAAATCTCGATAACTCAAAAAATACGCCCGGTAGTGA |
| | | TCTTATTTCATTATGGTGAAAGTTGGAACCTCTTACGTGCCGATCAACGTCTC ATTTTCGCCAAAAGTTGGCCCAGGGCTTCCCGGTATCAACAGGGACACCAGG ATTTATTTATTCTGCGAAGTGATCTTCCGTCACAGGTATTTATTCGGCGCAAA GTGCGTCGGGTGATGCTGCCGATATCGGACCCGGGATCCTAATTGGCTGGAC GTAAACTCCTCTTCAGACCTAATAACTTCGTATAGCATACATTATACGAAGTT ATATTAAGGGTTATTGAATATGATCGGGCGGCCGC |
| SEQ ID No.38 | The pSWT001 plasmid harboring the recombinase and Cre recombinase genes | GAATTCTCAGCCAAACGTCTCTTCAGGCCACTGACTAGCGATAACTTTCCCC ACAACGGAACAACTCTCATTGCATGGGATCATTGGGTACTGTGGGTTTAGTG GTTGTAAAAACACCTGACCGCTATCCCTGATCAGTTTCTTGAAGGTAAACTC ATCACCCCCAAGTCTGGCTATGCAGAAATCACCTGGCTCAACAGCCTGCTCA GGGTCAACGAGAATTAACATTCCGTCAGGAAAGCTTGGCTTGGAGCCTGTTG GTGCGGTCATGGAATTACCTTCAACCTCAAGCCAGAATGCAGAATCACTGGC TTTTTTGGTTGTGCTTACCCATCTCTCCGCATCACCTTTGGTAAAGGTTCTAA GCTTAGGTGAGAACATCCCTGCCTGAACATGAGAAAAAACAGGGTACTCAT ACTCACTTCTAAGTGACGGCTGCATACTAACCGCTTCATACATCTCGTAGATT TCTCTGGCGATTGAAGGGCTAAATTCTTCAACGCTAACTTTGAGAATTTTTGT AAGCAATGCGGCGTTATAAGCATTTAATGCATTGATGCCATTAAATAAAGCA CCAACGCCTGACTGCCCCATCCCCATCTTGTCTGCGACAGATTCCTGGGATA AGCCAAGTTCATTTTTCTTTTTTTCATAAATTGCTTTAAGGCGACGTGCGTCC TCAAGCTGCTCTTGTGTTAATGGTTTCTTTTTTGTGCTCATACGTTAAATCTAT CACCGCAAGGGATAAATATCTAACACCGTGCGTGTTGACTATTTTACCTCTG GCGGTGATAATGGTTGCATGTACTAAGGAGGTTGCGCATGCAGGTACCAATG TCCAATTTACTGACCGTACACCAAAATTTGCCTGCATTACCGGTCGATGCAA CGAGTGATGAGGTTCGCAAGAACCTGATGGACATGTTCAGGGATCGCCAGG CGTTTTCTGAGCATACCTGGAAAATGCTTCTGTCCGTTTGCCGGTCGTGGGCG GCATGGTGCAAGTTGAATAACCGGAAATGGTTTCCCGCAGAACCTGAAGAT GTTCGCGATTATCTTCTATATCTTCAGGCGCGCGGTCTGGCAGTAAAAACTAT CCAGCAACATTTGGGCCAGCTAAACATGCTTCATCGTCGGTCCGGGCTGCCA CGACCAAGTGACAGCAATGCTGTTTCACTGGTTATGCGGCGGATCCGAAAAG AAAACGTTGATGCCGGTGAACGTGCAAAACAGGCTCTAGCGTTCGAACGCA CTGATTTCGACCAGGTTCGTTCACTCATGGAAAATAGCGATCGCTGCCAGGA TATACGTAATCTGGCATTTCTGGGGATTGCTTATAACACCCTGTTACGTATAG CCGAAATTGCCAGGATCAGGGTTAAAGATATCTCACGTACTGACGGTGGGA GAATGTTAATCCATATTGGCAGAACGAAAACGCTGGTTAGCACCGCAGGTGT AGAGAAGGCACTTAGCCTGGGGGTAACTAAACTGGTCGAGCGATGGATTTC CGTCTCTGGTGTAGCTGATGATCCGAATAACTACCTGTTTTGCCGGGTCAGA AAAAATGGTGTTGCCGCGCCATCTGCCACCAGCCAGCTATCAACTCGCGCCC TGGAAGGGATTTTTGAAGCAACTCATCGATTGATTTACGGCGCTAAGGATGA CTCTGGTCAGAGATACCTGGCCTGGTCTGGACACAGTGCCCGTGTCGGAGCC GCGCGAGATATGGCCCGCGCTGGAGTTTCAATACCGGAGATCATGCAAGCTG GTGGCTGGACCAATGTAAATATTGTCATGAACTATATCCGTAACCTGGATAG TGAAACAGGGGCAATGGTGCGCCTGCTGGAAGATGGCGATTAGGGGTACCG AGCTCATGAACGCTTATTACATTCAGGATCGTCTTGAGGCTCAGAGCTGGGC GCGTCACTACCAGCAGCTCGCCCGTGAAGAGAAAGAGGCAGAACTGGCAGA CGACATGGAAAAAGGCCTGCCCCAGCACCTGTTTGAATCGCTATGCATCGAT CATTTGCAACGCCACGGGGCCAGCAAAAAATCCATTACCCGTGCGTTTGATG ACGATGTTGAGTTTCAGGAGCGCATGGCAGAACACATCCGGTACATGGTTGA AACCATTGCTCACCACCAGGTTGATATTGATTCAGAGGTATAAAACGAATGA GTACTGCACTCGCAACGCTGGCTGGGAAGCTGGCTGAACGTGTCGGCATGGA TTCTGTCGACCCACAGGAACTGATCACCACTCTTCGCCAGACGGCATTTAAA GGTGATGCCAGCGATGCGCAGTTCATCGCATTACTGATCGTTGCCAACCAGT ACGGCCTTAATCCGTGGACGAAAGAAATTTACGCCTTTCCTGATAAGCAGAA TGGCATCGTTCCGGTGGTGGGCGTTGATGGCTGGTCCCGCATCATCAATGAA AACCAGCAGTTTGATGGCATGGACTTTGAGCAGGACAATGAATCCTGTACAT GCCGGATTTACCGCAAGGACCGTAATCATCCGATCTGCGTTACCGAATGGAT GGATGAATGCCGCCGCGAACCATTCAAAACTCGCGAAGGCAGAGAAATCAC GGGGCCGTGGCAGTCGCATCCCAAACGGATGTTACGTCATAAAGCCATGATT CAGTGTGCCCGTCTGGCCTTCGGATTTGCTGGTATCTATGACAAGGATGAAG CCGAGCGCATTGTCGAAAATACTGCATACACTGCAGAACGTCAGCCGGAAC GCGACATCACTCCGGTTAACGATGAAACCATGCAGGAGATTAACACTCTGCT GATCGCCCTGGATAAAACATGGGATGACGACTTATTGCCGCTCTGTTCCCAG ATATTTCGCCGCGACATTCGTGCATCGTCAGAACTGACACAGGCCGAAGCAG TAAAAGCTCTTGGATTCCTGAAACAGAAAGCCGCAGAGCAGAAGGTGGCAG CATGACACCGGACATTATCCTGCAGCGTACCGGGATCGATGTGAGAGCTGTC GAACAGGGGGATGATGCGTGGCACAAATTACGGCTCGGCGTCATCACCGCTT CAGAAGTTCACAACGTGATAGCAAAACCCCGCTCCGGAAAGAAGTGGCCTG ACATGAAAATGTCCTACTTCCACACCCTGCTTGCTGAGGTTTGCACCGGTGT GGCTCCGGAAGTTAACGCTAAAGCACTGGCCTGGGGAAAACAGTACGAGAA CGACGCCAGAACCCTGTTTGAGTTCACTTCCGGCGTGAATGTTACTGAATCC CCGATCATCTATCGCGACGAAAGTATGCGTACCGCCTGCTCTCCCGATGGTT |
| | | TATGCAGTGACGGCAACGGCCTTGAACTGAAATGCCCGTTTACCTCCCGGGA TTTCATGAAGTTCCGGCTCGGTGGTTTCGAGGCCATAAAGTCAGCTTACATG GCCCAGGTGCAGTACAGCATGTGGGTGACGCGAAAAAATGCCTGGTACTTTG CCAACTATGACCCGCGTATGAAGCGTGAAGGCCTGCATTATGTCGTGATTGA GCGGGATGAAAAGTACATGGCGAGTTTTGACGAGATCGTGCCGGAGTTCATC GAAAAAATGGACGAGGCACTGGCTGAAATTGGTTTTGTATTTGGGGAGCAAT GGCGATGACTCGAG |
| SEQ ID No.39 | NotI-loxP-chlorampheni col resistance gene-loxP-NotI cassette in pSWT002 | GCGGCCGCGACTAGTGAACCTCTTCGAGGGACCTAATAACTTCGTATAGCAT ACATTATACGAAGTTATATTAAGGGTTCCGGATCCTAAGCTTGATATCTAAG GGCACCAATAACTGCCTTAAAAAAATTACGCCCCGCCCTGCCACTCATCGCA GTACTGTTGTAATTCATTAAGCATTCTGCCGACATGGAAGCCATCACAGACG GCATGATGAACCTGAATCGCCAGCGGCATCAGCACCTTGTCGCCTTGCGTAT AATATTTGCCCATGGTGAAAACGGGGGCGAAGAAGTTGTCCATATTGGCCAC GTTTAAATCAAAACTGGTGAAACTCACCCAGGGATTGGCTGAGACGAAAAA CATATTCTCAATAAACCCTTTAGGGAAATAGGCCAGGTTTTCACCGTAACAC GCCACATCTTGCGAATATATGTGTAGAAACTGCCGGAAATCGTCGTGGTATT CACTCCAGAGCGATGAAAACGTTTCAGTTTGCTCATGGAAAACGGTGTAACA AGGGTGAACACTATCCCATATCACCAGCTCACCGTCTTTCATTGCCATACGG AATTCCGGATGAGCATTCATCAGGCGGGCAAGAATGTGAATAAAGGCCGGA TAAAACTTGTGCTTATTTTTCTTTACGGTCTTTAAAAAGGCCGTAATATCCAG CTGAACGGTCTGGTTATAGGTACATTGAGCAACTGACTGAAATGCCTCAAAA TGTTCTTTACGATGCCATTGGGATATATCAACGGTGGTATATCCAGTGATTTT TTTCTCCATTTTAGCTTCCTTAGCTCCTGAAAATCTCGATAACTCAAAAAATA CGCCCGGTAGTGATCTTATTTCATTATGGTGAAAGTTGGAACCTCTTACGTGC CGATCAACGTCTCATTTTCGCCAAAAGTTGGCCCAGGGCTTCCCGGTATCAA CAGGGACACCAGGATTTATTTATTCTGCGAAGTGATCTTCCGTCACAGGTAT TTATTCGGCGCAAAGTGCGTCGGGTGATGCTGCCGATATCGGTACCCGGGAT CCTAAGCTTGGCTGGACGTAAACTCCTCTTCAGACCTAATAACTTCGTATAG CATACATTATACGAAGTTATATTAAGGGTTATTGAATATGATCGGGCGGCCG C |
| SEQ ID No.40 | sThe *sseA* promoter-RBS regulatory module | GCGGCCGCAAGAGAACAACGGCAAGTTACAGGATCCGCAGCAATATCAGCA AAACACCTTATTGCTTGAAGCGATCGAGCAGGCCGAAAATATCATCAACATT ATTTATTATCGTTACCATAACAGCGCACTTGTAGTGAGTGAGCAAGAGTAAA GTAAAAATATCTTAGAGCCTATCCCACCAGGCGTTAATTGGCGCAGCCAGTT TGGACACGGATAGCGCGCAAAAACCGCAGCGTACACGTAGTACGTGAGGTT TGACTCGCTACGCTCGCCCTTCGGGCCGCCGCTAGCGGCGTTCAAAACGCTA ACGCGTTTTGGCGAGCACTGCCCAGGTTCAAAATGGCAAGTAAAATAGCCCT AATGGGATAGGCTCTTAGTTAGCACGTTAATTATCTATCGAAGCTTAAGAAG GAGATATACATATG |

Sequences of all primers used in the present invention are shown in Table 5.

**Table 5: Sequences of all primers used in the present invention**

| SEQ ID | No. | Sequence | Purpose |
|---|---|---|---|
| SEQ ID No.41 | SWTO1 | ATGGAATCCCTGACGTTACAACCCATCGCGCGGG TCGATGGCGCCATTAACCGATCATATTCAATAACC CT | *aroA* gene knockout |
| SEQ ID No.42 | SWTO2 | GAAGACTTAGGCAGGCGTACTCATTCGCGCCAGT TGTTCGAAATAATCAGGACTAGTGAACCTCTTCGA GGG | |
| SEQ ID No.43 | SWTO3 | CGGACAATACGCTGGACAAG | Forward primer for *aroA* identification |
| SEQ ID No.44 | SWTO4 | GCGGCTTTGTAGGTTGACAT | Reverse primer for *aroA* identification |
| SEQ ID No.45 | SWTO5 | TCCTTAGCTCCTGAAAATCTCG | Reverse primer for detecting chloramphenicol resistance gene |
| SEQ ID No.46 | SWTO6 | TGTGGCGTGTTACGGTGAAA | Forward primer for detecting a chloramphenicol resistance gene |
| SEQ ID No.47 | SWTO19 | ATTTGCGGCCGCGCGTGAACGCAGTTTATCTAACA CG | *NotI-yhbU-S* promoter forward primer |
| SEQ ID No.48 | SWTO20 | CCCAAGCTTTTATCCTGAGGGTTACGTCTGAGACG | *HindIII-yhbU-S* promoter reverse primer |
| SEQ ID No.49 | SWTO21 | ATTTGCGGCCGCCGCTGCACTGGTAAAAGACG | *NotI-ynfK-E* promoter forward primer |
| SEQ ID No.50 | SWTO22 | CCCAAGCTTAACTCCACCGTTATGCTTCAC | *HindIII-ynfK-E* promoter reverse primer |
| SEQ ID No.51 | SWTO35 | CCCAAGCTTTGGAGCGAAACCGATGCAAGCGGCA ACAGTCGTC | *HindIII-Alr-S* gene forward primer |
| SEQ ID No.52 | SWTO36 | CCGCTCGAGTTAATCAATATACTTCATCGCCACCC TTG | *XhoI-Alr-S* gene reverse primer |
| SEQ ID No.53 | SWTO39 | CCCAAGCTTTGGAGCGAAACCGATGACCCGCCCT ATACAGGC | *HindIII-dadX-S* gene forward primer |
| SEQ ID No.54 | SWTO40 | CCCAAGCTTTTACGTTGTCACAAACGGCAC | *XhoI-dadX-S* gene reverse primer |
| SEQ ID No.55 | SWTO43 | CCGCTCGAGATTGGTTCTGTGTTACCAAGTTCTGC | *XhoI-cyoA-S* promoter forward primer |
| SEQ ID No.56 | SWTO44 | AAAACTGCAGAACATTATTTGCCGTTGTAATAACG | *PstI-cyoA-S* promoter reverse prime |
| SEQ ID No.57 | SWTO51 | CCGCTCGAGAAGCGATGTTAAAAACAGAGCCG | *XhoI-ydcI-S* promoter forward primer |
| SEQ ID No.58 | SWTO52 | AAAACTGCAGAATGTCATGCCTCCAGTGAATGTT AC | *PstI-ydcI-S* promoter reverse primer |
| SEQ ID No.59 | SWTO59 | GGTACGCAAGATTGGGGTAC | dadX knockout detection reverse primer 1 |
| SEQ ID No.60 | SWTO61 | TGTCAGCTCTTTACCCAGCG | *dadX* identification primer 1 for strain verification |
| SEQ ID No.61 | SWTO70 | GCAATACGATGATGAGTAACTCTCCGTCATTCTTT TAACAAGGAATTCAACCGATCATATTCAATAACC CT | *alr* gene knockout forward primer |
| SEQ ID No.62 | SWTO71 | TGCGCGATACAAGCCGGATAAGCGCAAGCGCCAC CCGGCCCGCCGCGTATGACTAGTGAACCTCTTCGA GGG | *alr* gene knockout strain verification reverse primer |
| SEQ ID No.63 | SWTO72 | CAGGTCTTACGCCGACAGAA | *alr* gene knockout strain verification forward primer |
| SEQ ID No.64 | SWTO73 | GTTGCTGAAGAACCGGGTGA | *alr* gene knockout strain verification reverse primer |
| SEQ ID No.65 | SWTO75 | CGACGGCGATGCTTAAAGGC | *dadX* identification primer 2 for strain verification |
| SEQ ID No.66 | SWTO78 | CACCCCCCCGCAACGTTTGCATAGCGCGCATAACT GATAAAGGAAGTGAACAGGAAACAGCTATGAC | *dadX* gene knockout universal forward primer |
| SEQ ID No.67 | SWTO79 | ACGTCGACCCGCCCCCAAGTGGACCGGTCGACGC CTTAGCCTGAATTAGGGTAAAACGACGGCCAGT | *dadX* gene knockout universal reverse primer |
| SEQ ID No.68 | SWTO85 | GCAATACGATGATGAGTAACTCTCCGTCATTCTTT TAACAAGGAATTCAAGAACAGGAAACAGCTATGA C | *alr* gene knockout universal forward primer |
| SEQ ID No.69 | SWTO86 | TGCGCGATACAAGCCGGATAAGCGCAAGCGCCAC CCGGCCCGCCGCGTATGTAAAACGACGGCCAGT | *alr* gene knockout universal reverse primer |
| SEQ ID No.70 | SWTO87 | GCACCTTAGGCTGGACGATG | *dadX* identification primer 3 for strain verification |
| SEQ ID No.71 | SWTO98 | TAACCGCTCCCTGGAACAAC | *alr* identification primer 1 for strain verification |
| SEQ ID No.72 | SWTO99 | GGTAAGCAACGTAACGGTCC | *alr* identification primer 2 for strain verification |
| SEQ ID No.73 | SWTO100 | GGCCGCTCACTCATTAGGCACCCCAGGCTTTACAC TTTATGCTTCCGGCTCGTATAATGTGTGGAAA | *NotI-lacUV5-HindIII* forward primer |
| SEQ ID No.74 | SWTO101 | AGCTTTTCCACACATTATACGAGCCGGAAGCATA AAGTGTAAAGCCTGGGGTGCCTAATGAGTGAGC | *NotI-lacUV5-HindIII* reverse primer |
| SEQ ID No.75 | SWTO102 | CGCATACACAGCACATCTCTTTGCAGGAAAAAAA CGCTGAACAGGAAACAGCTATGAC | *asd* gene knockout universal forward primer for T7 polymerase amplification |
| SEQ ID No.76 | SWTO103 | GAACCACACGCAGGCCCGATAAGCGCTGCAATAG CCAGTAAAACGACGGCCAGT | *asd* gene knockout universal reverse primer for T7 polymerase amplification |
| SEQ ID No.77 | SWTO104 | GACTGCCGATTGCCATTACC | *Salmonella asd* mutation identification forward primer |
| SEQ ID No.78 | SWTO105 | TGAGGTTCTGCTGTCCGTGC | *Salmonella asd* mutation identification reverse primer |
| SEQ ID No.79 | SWTO106 | CATATCTGCTTCAACAACCTGC | T7 RNA polymerase identification reverse primer |
| SEQ ID No.80 | SWTO107 | CGTGAAGAACTGCCGATGAA | T7 RNA polymerase identification forward primer |
| SEQ ID No.81 | SWTO108 | ATCGTCTTGAGTCCAACCCG | pPRO010 detection forward primer 1 |
| SEQ ID No.82 | SWTO109 | TTCGTTGCCGTCTTTGTAGC | pPRO010 detection forward primer 1 |
| SEQ ID No.83 | SWTO110 | AATACGACTCACTATAGGGAGAGCT | pPRO010 detection forward primer 2 |
| SEQ ID No.84 | SWTO111 | TACGCCATTTCGTCGTCGTA | pPRO010 detection forward primer 2 |
| SEQ ID No.85 | SWTO112 | ACCCTGTCTATCGACCTGCC | pPRO010 detection forward primer 3 |
| SEQ ID No.86 | SWTO113 | AACAGATTTGCCGCTTACCG | pPRO010 detection forward primer 3 |
| SEQ ID No.87 | SWTO114 | ACCCGTCCGTCTCGTTTCTT | pPRO010 detection forward primer 4 |
| SEQ ID No.88 | SWTO115 | CGATCGGGTTGTCAACTTTG | pPRO010 detection forward primer 4 |
| SEQ ID No.89 | SWTO116 | GAATGGTGGCGTACCGTTAT | pPRO010 detection forward primer 5 |
| SEQ ID No.90 | SWTO117 | ACCGACAACAAATACGACGC | pPRO010 detection forward primer 5 |
| SEQ ID No.91 | SWTO118 | TACGGTAAACGCCGACAAGA | pPRO010 detection forward primer 6 |
| SEQ ID No.92 | SWTO119 | AAAACAGGGCGAATAGCGTC | pPRO010 detection forward primer 6 |
| SEQ ID No.93 | SWTO120 | ACTTGCGACTTTGGCTGCTT | pPRO010 detection forward primer 7 |
| SEQ ID No.94 | SWTO121 | CGGGTCAATGCCGTAACTTT | pPRO010 detection forward primer 7 |
| SEQ ID No.95 | SWTO122 | GCCTGATGTTGATGTCGCTG | pPRO010 detection forward primer 8 |
| SEQ ID No.96 | SWTO123 | TACGGTAAACGCCGACAAGA | pPRO010 detection forward primer 8 |
| SEQ ID No.97 | SWTO124 | CATAATCCGTGGGCGAAAGT | pPRO010 detection forward primer 9 |
| SEQ ID No.98 | SWTO125 | TACCAGCGGTGGTTTGTTTG | pPRO010 detection forward primer 9 |
| SEQ ID No.99 | SWTO126 | AAACTGCAGAAGAGAACAACGGCAAGTTACA | *sseA* promoter amplification forward primer |
| SEQ ID No.100 | SWTO127 | GGGAATTCCATATGTATATCTCCTTCTTAAGCTTC G | *sseA* promoter amplification reverse primer |
| SEQ ID No.101 | SWTO128 | TGACGCTCAGTGGAACGAAA | pPRO012 detection reverse primer 1 |

Although the present application has been described above with reference to preferred embodiments, these embodiments are merely exemplary and illustrative. On this basis, various substitutions and modifications can be made to the present application, all of which fall within the scope of protection of the present application.

## Claims

1. A constitutive plasmid expression vector, comprising dual bacteriophage-derived promoters respectively controlling the expression of a therapeutic protein gene and a membrane-disrupting protein gene, wherein the bacteriophage-derived promoters bind to an RNA polymerase encoded by a specific bacteriophage RNA polymerase gene on a host bacterial chromosome.

2. The expression vector according to claim 1, wherein the dual bacteriophage-derived promoters are selected from the group consisting of T7, T3, and SP6 promoters.

3. The expression vector according to claim 2, wherein the dual bacteriophage-derived promoters are T7 promoters.

4. The expression vector according to any one of claims 1 to 3, wherein the dual bacteriophage-derived promoters are oriented convergently, constituting a convergent promoter expression vector.

5. The expression vector according to any one of the preceding claims, comprising the essential gene *asd* of *Salmonella*, wherein the *asd* gene on the host bacterial chromosome is deleted.

6. The expression vector according to any one of the preceding claims, comprising a replicon essential for plasmid replication.

7. The expression vector according to claim 6, wherein the replicon is selected from the group consisting of pUC, p15A, ColE1, and R6K replicons.

8. The expression vector according to claim 7, wherein the replicon is a pUC replicon.

9. The expression vector according to any one of the preceding claims, wherein the therapeutic protein gene is a bacteria-derived AB-type exotoxin A fragment gene.

10. The expression vector according to claim 9, wherein the therapeutic protein gene is selected from the group consisting of a diphtheria toxin A fragment gene, a cholera toxin A fragment gene, a Shiga toxin A fragment gene, a pertussis toxin A fragment gene, and a Pseudomonas exotoxin A fragment gene.

11. The expression vector according to claim 10, wherein the therapeutic protein gene is a diphtheria toxin A fragment gene.

12. The expression vector according to any one of the preceding claims, (wherein the membrane-disrupting protein gene is selected from the group consisting of a *Listeria* hemolysin *hlyA* gene.

13. The expression vector according to any one of the preceding claims, wherein the host bacterium is a Gram-negative bacterium.

14. A method for constructing the expression vector according to any one of the preceding claims, comprising the following steps:
(1) sequentially ligating the bacteriophage-derived promoter, a ribosome binding site, and the membrane-disrupting protein gene according to any one of the preceding claims to construct a membrane-disrupting protein gene expression unit;
(2) sequentially ligating the bacteriophage-derived promoter, a ribosome binding site, and the therapeutic protein gene according to any one of the preceding claims to construct a therapeutic protein gene expression unit;
(3) ligating a plasmid replicon, an *asd* gene, and the above two gene expression units together to form a complete plasmid expression vector.

15. A modified Gram-negative bacterium, comprising the expression vector according to any one of the preceding claims.

16. The Gram-negative bacterium according to claim 15, wherein the specific bacteriophage RNA polymerase gene is a gene corresponding to the dual bacteriophage-derived promoters.

17. The Gram-negative bacterium according to claim 16, wherein the specific bacteriophage RNA polymerase gene is selected from the group consisting of a T7 RNA polymerase gene, a T3 RNA polymerase gene, and an SP6 RNA polymerase gene.

18. The Gram-negative bacterium according to claim 17, wherein the specific bacteriophage RNA polymerase gene is a T7 RNA polymerase gene.

19. The Gram-negative bacterium according to any one of claims 15 to 18, wherein the expression of the specific bacteriophage RNA polymerase gene is regulated by a constitutive promoter.

20. The Gram-negative bacterium according to claim 19, wherein the constitutive expression promoter is a *lacUV5* promoter.

21. The Gram-negative bacterium according to any one of claims 15 to 20, further comprising a hypoxia-specific gene expression cassette, wherein the hypoxia-specific gene expression cassette comprises:
a) a forward hypoxia promoter, which is a promoter comprising an FNR binding site, wherein the forward hypoxia promoter is capable of being induced for expression under hypoxia;
b) a survival-essential gene; and
c) a reverse hyperoxia promoter, which is a promoter comprising FNR and ArcA binding sites, wherein the reverse hyperoxia promoter is capable of functioning under oxygen concentration condition of normal organs;
wherein the survival-essential gene is a gene encoding alanine racemase.

22. The Gram-negative bacterium according to claim 21, wherein:
the FNR binding site of the forward hypoxia promoter or the reverse hyperoxia promoter conforms to the pattern of *TTGATNNNNATCAA*, wherein N is any base of A, T, C, and G, and wherein any base in the *TTGAT* and *ATCAA* sequences of the conserved binding site can be substituted, provided that the total number of substitutions does not exceed 3, and 3 consecutive adjacent bases cannot be substituted;
the ArcA binding site of the reverse hyperoxia promoter conforms to the pattern of *GTTAATTA*, wherein any base can be substituted, provided that the total number of substitutions does not exceed 2.

23. The Gram-negative bacterium according to claim 21 or 22, wherein the forward hypoxia promoter and/or the reverse hyperoxia promoter of the hypoxia-specific gene expression cassette are promoters derived from a Gram-negative bacterium.

24. The Gram-negative bacterium according to any one of claims 21 to 23, wherein:
the forward hypoxia promoter is selected from the promoter region sequences of *yhbU* or *ynfK*;
the survival-essential gene is selected from *alr* or *dadX*;
the reverse hyperoxia promoter is selected from the promoter region sequences of *cyoA* or *ydcI.*

25. The Gram-negative bacterium according to claim 24, (wherein the forward hypoxia prom*o*ter is *yhbU*, which is *s*elected from the group consisting of *Salmonella*, *Escherichia coli*, *Shigella*, *Yersinia*, *Enterobacter cloacae*, *Cronobacter*, *Klebsiella*, *Pantoea*, *Serratia*, and *Shimwellia*.

26. The Gram-negative bacterium according to claim 24, wherein the forward hypoxia promoter is *ynfK*, which is selected from the group consisting of *Salmonella*, *Escherichia coli*, *Serratia*, *Shigella*, and *Lactobacillus reuteri*.

27. The Gram-negative bacterium according to claim 24, wherein the *alr* gene and the *dadX* gene are selected from the group consisting of *Salmonella*, *Escherichia coli*, *Shigella*, *Klebsiella*, *Yersinia*, *Haemophilus*, and *Pseudomonas*.

28. The Gram-negative bacterium according to claim 24, wherein the hypoxia-specific gene expression cassette consists of the forward hypoxia promoter *yhbU*, the survival-essential gene *alr*, and the reverse hyperoxia promoter *cyoA*.

29. The Gram-negative bacterium according to claim 24, wherein the hypoxia-specific gene expression cassette consists of the forward hypoxia promoter *yhbU*, the survival-essential gene *alr*, and the reverse hyperoxia promoter *ydcI.*

30. The Gram-negative bacterium according to claim 24, wherein the hypoxia-specific gene expression cassette consists of the forward hypoxia promoter *ynfK*, the survival-essential gene *dadX*, and the reverse hyperoxia promoter *cyoA*.

31. The Gram-negative bacterium according to claim 24, wherein the hypoxia-specific gene expression cassette consists of the forward hypoxia promoter *ynfK*, the survival-essential gene *dadX*, and the reverse hyperoxia promoter *ydcI*.

32. The Gram-negative bacterium according to any one of claims 21-31, wherein the hypoxia-specific gene expression cassette is regulated by oxygen concentration.

33. The Gram-negative bacterium according to claim 32, wherein the forward hypoxia promoter functions when the oxygen concentration is lower than 1%, and does not function when the oxygen concentration is higher than 1%; and/or the reverse hyperoxia promoter functions when the oxygen concentration is higher than 1%, and does not function when the oxygen concentration is lower than 1%.

34. The Gram-negative bacterium according to claim 33, wherein the forward hypoxia promoter functions when the oxygen concentration is lower than 0.8%, and does not function when the oxygen concentration is higher than 0.8%; and/or the reverse hyperoxia promoter functions when the oxygen concentration is higher than 0.8%, and does not function when the oxygen concentration is lower than 0.8%.

35. A method for controlling expression of a therapeutic protein in a prokaryotic cell using the expression vector according to any one of claims 1 to 13, comprising the following steps:
(1) preparing the expression vector according to any one of claims 1 to 13;
(2) integrating the constitutively expressed bacteriophage RNA polymerase gene expression cassette into the chromosome of a host bacterium;
(3) knocking out the *asd* gene on the chromosome of the host bacterium;
(4) transforming the expression vector according to any one of the preceding claims into the host bacterium.

36. The method according to claim 35, further comprising integrating the hypoxia-specific gene expression cassette according to any one of claims 21 to 34 into the chromosome of the host bacterium.

37. The method according to claim 35 or 36, wherein the host bacterium is a Gram-negative bacterium.

38. The Gram-negative bacterium according to any one of the preceding claims, which is selected from the group consisting of *Salmonella*, *Escherichia coli*, *Shigella*, *Yersinia*, *Enterobacter cloacae*, *Cronobacter*, *Klebsiella*, *Pantoea*, *Serratia*, *Shimwellia*, *Enterobacter ludwigii*, *Haemophilus*, *Vibrio*, *Pseudomonas*, *Pasteurella*, *Bordetella*, *Bordetella pertussis*, *Acinetobacter baumannii*, *Burkholderia*, *Vibrio vulnificus*, *Bacteroides fragilis*, *Pseudomonas syringae*, *Pseudomonas putida*, *Legionella*, *Klebsiella pneumoniae*, *Vibrio parahaemolyticus*, *Vibrio cholerae*, *Yersinia pestis*, *Catarrhalis coccus*, *Moraxella catarrhalis*, *Campylobacter jejuni*, *Shigella dysenteriae*, *Neisseria gonorrhoeae*, *Haemophilus influenzae*, *Moraxella*, *Neisseria meningitidis*, *Proteus vulgaris*, *Proteus mirabilis*, *Pasteurella haemolytica*, *Legionella pneumophila*, *Yersinia pestis*, *Shigella sonnei*, *Pseudomonas aeruginosa*, *Yersinia enterocolitica*, *Cryptococcus neoformans*, *Burkholderia cenocepacia*, and *Helicobacter pylori*.

39. The Gram-negative bacterium according to claim 38, wherein the bacterium is *Salmonella*.

40. The Gram-negative bacterium according to claim 38 or 39, wherein the bacterium is an attenuated Gram-negative bacterium.

41. The Gram-negative bacterium according to claim 40, wherein the attenuated Gram-negative bacterium is *Salmonella.*

42. A pharmaceutical composition, comprising the expression vector according to any one of the preceding claims, or the Gram-negative bacterium according to any one of the preceding claims.

43. The pharmaceutical composition according to claim 42, further comprising a pharmaceutically acceptable carrier.

44. The pharmaceutical composition according to claim 43, wherein the pharmaceutically acceptable carrier is selected from the group consisting of disintegrants, binders, fillers, buffers, tonicity agents, stabilizers, antioxidants, surfactants, and lubricants.

45. The pharmaceutical composition according to any one of claims 42-44, which is used for treating solid tumors.

46. Use of the expression vector according to any one of the preceding claims, the Gram-negative bacterium according to any one of the preceding claims, or the pharmaceutical composition according to any one of the preceding claims in the preparation of a medicament for treating a tumor.

47. The use according to claim 46, wherein the tumor is a solid tumor.

48. The pharmaceutical composition according to claim 45 or the use according to claim 47, wherein the solid tumor is selected from the group consisting of tumors/cancers of the breast, bone, liver, lung, skin, kidney, stomach, pancreas, prostate, lymph (non-Hodgkin's lymphoma, Hodgkin's lymphoma), intestine (colon cancer, rectal cancer), pelvis (cervical cancer, ovarian malignancy, endometrial cancer, ovarian cancer), nervous system, head and neck, and bladder, etc.

49. The pharmaceutical composition or the use according to claim 48, wherein the solid tumor is selected from the group consisting of breast cancer, osteosarcoma, liver cancer, lung cancer, melanoma, kidney cancer, gastric cancer, pancreatic cancer, prostate cancer, colon cancer, ovarian cancer, neuroblastoma, squamous cell carcinoma, and bladder cancer.

50. A method for treating a tumor, comprising administering to a subject the expression vector according to any one of claims 1 to 13, the Gram-negative bacterium according to any one of claims 15 to 34 and 38 to 41, or the pharmaceutical composition according to any one of claims 42 to 45.

51. The method according to claim 50, wherein the tumor is a solid tumor.

52. The method according to claim 51, wherein the solid tumor is selected from the group consisting of tumors/cancers of the breast, bone, liver, lung, skin, kidney, stomach, pancreas, prostate, lymph (non-Hodgkin's lymphoma, Hodgkin's lymphoma), intestine (colon colon cancer, rectal cancer), pelvis (cervical cancer, ovarian malignancy, endometrial cancer, ovarian cancer), nervous system, head and neck, and bladder, etc.

53. The method according to claim 52, wherein the solid tumor is selected from the group consisting of breast cancer, osteosarcoma, liver cancer, lung cancer, melanoma, kidney cancer, gastric cancer, pancreatic cancer, prostate cancer, colon cancer, ovarian cancer, neuroblastoma, squamous cell carcinoma, and bladder cancer.
